# EUROPEAN PATENT APPLICATION

(11) **EP 3 178 905 A1**
(43) Date of publication of application: **14.06.2017**
(21) Application number: 15829339.9
(22) Date of filing: 22.06.2015
(51) Int. Cl.: C09K 15/06, C07C 69/96, C08K 5/109, C08L 101/00, C09K 15/12, C09K 15/20, C09K 15/28, C09K 15/32

(54) **ANTIOXIDANT FOR THERMOPLASTIC RESIN AND THERMOPLASTIC RESIN COMPOSITION INCLUDING SAME**

(30) Priority: 05.08.2014 JP 2014159894
(71) Applicant: Adeka Corporation, Tokyo 116-8554 (JP)
(72) Inventor: OKADA, Mitsuhiro, Tokyo 116-8554 (JP); USUI, Takashi, Saitama-shi Saitama 336-0022 (JP); MATSUI, Izumi, Tokyo 116-8554 (JP)
(74) Representative: Forstmeyer, Dietmar
(86) International application number: PCT/JP2015/067890
(87) International publication number: WO 2016/021315

(57) **Abstract**

The present invention provides an antioxidant for a thermoplastic resin that provides an excellent oxidation-preventing effect to a thermoplastic resin and that has an excellent effect of preventing a change in color caused by nitrogen oxides. Specifically, a compound represented by any of General Formulae (2) to (4), particularly a compound in which R² in the formulae is an alkyl group having 1 to 8 carbon atoms whose terminal on an oxygen atom side is linked with -CO-O-, is used as the antioxidant for a thermoplastic resin. Details of General Formulae (2) to (4) are as described in the description.

## Description

### Technical Field

The present invention relates to an antioxidant for stabilizing a thermoplastic resin that is easily oxidatively deteriorated at room temperature. More specifically, the present invention relates to providing a thermoplastic resin composition containing an antioxidant that has a structure in which a phenolic hydroxyl group is protected with a specific substituent, particularly with an alkyl carbonate group, and that has an excellent effect of preventing oxidation, particularly of a polyolefin-based resin, and suppresses a change in color of the polyolefin-based resin due to contact with a nitrogen oxide.

It is known that compounds having a 3,5-di-tert-butyl-4-hydroxyphenyl group provide an excellent stabilizing effect to various thermoplastic resins such as a polyvinyl chloride resin, a polyethylene resin, a polypropylene resin, a polybutene resin, a polybutylene terephthalate resin, a polycarbonate resin, an ABS resin, nylon 6, nylon 66, an ethylene-vinyl acetate copolymer, a petroleum resin, and a coumarone resin.

In particular, as disclosed in Patent Literatures 1 to 23, phenol-based antioxidants are widely used as antioxidants that improve the thermal resistance of polyolefin-based resins such as polyethylene and polypropylene. In addition to the phenol-based antioxidants, examples of an additive for preventing the oxidation and deterioration of the thermoplastic resins include phosphorus-based antioxidants, sulfur-based antioxidants, hydroxyamine compounds, hindered amine compounds, ultraviolet absorbers, and acid scavengers.

Patent Literature 1: JP S53-29343B
Patent Literature 2: JP S53-34153B
Patent Literature 3: U.S. Patent No. 3531483
Patent Literature 4: JP S51-41899B
Patent Literature 5: JP S38-17164B
Patent Literature 6: JP S41-565B
Patent Literature 7: JP S42-9651B
Patent Literature 8: JP S52-41779B
Patent Literature 9: U.S. Patent No. 4405807
Patent Literature 10: JP S47-2211A
Patent Literature 11: JP S59-25826A
Patent Literature 12: JP S62-141066A
Patent Literature 13: JP S62-181352A
Patent Literature 14: JP S62-220544A
Patent Literature 15: JP S39-9863B
Patent Literature 16: U.S. Patent No. 4857572
Patent Literature 17: JP H3-68897B
Patent Literature 18: U.S. Patent No. 5128398
Patent Literature 19: U.S. Patent No. 4774274
Patent Literature 20: U.S. Patent No. 4778842
Patent Literature 21: U.S. Patent No. 5889095
Patent Literature 22: Japanese Patent No. 3272658
Patent Literature 23: U.S. Patent No. 4719257

### Summary of Invention

### Technical Problem

With regard to molded articles, particularly vehicle members, made of a resin compound obtained by blending a known phenol-based antioxidant into a polyolefin-based resin, the number of the molded articles whose color changes to yellow or brown has been increasing.

As a result of research on the causes for such a change in color, the inventors of the present invention found that nitrogen oxides generated from automobile exhaust gas caused the change in color. In recent years, the content of nitrogen oxides in the atmosphere has been increasing every year due to combustion of petroleum fuel and the like. Therefore, there is a concern that such nitrogen oxides in the atmosphere cause coloring of not only molded articles made of a polyolefin-based resin but also those made of another thermoplastic resin.

However, a conventionally used formulation of a stabilizer has difficulty in preventing the change in color caused by nitrogen oxides as mentioned above. For example, Patent Literature 23 states that yellowing caused by nitrogen oxide gas can be prevented by blending 8,9-bis[2-(8-(3-tert-butyl-4-hydroxy-5-methylphenyl)propionyloxy)-1,1-dimethylethyl]-2, 4,8-tetraoxaspiro[5.5]undecane into a polyolefin resin, but the prevention of yellowing is not sufficient yet.

Therefore, an object of the present invention is to provide an antioxidant for a thermoplastic resin that provides an excellent oxidation-preventing effect to a thermoplastic resin and has an excellent effect of preventing a change in color caused by nitrogen oxides.

### Solution to Problem

As a result of research to solve the foregoing problems, the inventors of the present invention found that antioxidants for a thermoplastic resin that have a specific structure can be used to solve the foregoing problems, and the present invention was achieved.

That is, the present invention provides an antioxidant for a thermoplastic resin represented by any of General Formulae (2) to (4) below: 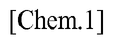 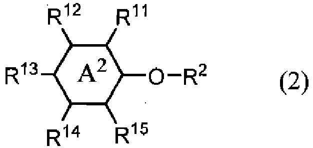
wherein a ring A² is a six-membered hydrocarbon ring, aromatic ring, or heterocycle,
R² represents an unsubstituted or substituted alkyl group having 1 to 20 carbon atoms, an unsubstituted or substituted alkenyl group having 2 to 20 carbon atoms, an unsubstituted or substituted aryl group having 6 to 30 carbon atoms, an unsubstituted or substituted arylalkyl group having 7 to 30 carbon atoms, an unsubstituted or substituted heterocycle-containing group having 3 to 35 carbon atoms, or a trialkylsilyl group,
a methylene group in the alkyl group, alkoxy group or arylalkyl group represented by R² is optionally replaced with one or more groups selected from a carbon-carbon double bond, -O-, -S-, -CO-, -O-CO-, -CO-O-, -O-CO-O-, -S-CO-, -CO-S-, -S-CO-O-, -O-CO-S-, -CO-NH-, -NH-CO-, -NH-CO-O-, -NR'-, -S-S-, and -SO₂-(provided that oxygen atoms are not adjacent to each other when the methylene group is replaced with two or more groups),
R' represents a hydrogen atom or an alkyl group having 1 to 8 carbon atoms,
R¹¹, R¹², R¹³, R¹⁴, and R¹⁵ represent a hydrogen atom, a halogen atom, a cyano group, a hydroxyl group, a nitro group, carboxyl group, an unsubstituted or substituted alkyl group having 1 to 40 carbon atoms, an unsubstituted or substituted alkoxy group having 1 to 8 carbon atoms, an unsubstituted or substituted aryl group having 6 to 30 carbon atoms, an unsubstituted or substituted arylalkyl group having 7 to 30 carbon atoms, an unsubstituted or substituted heterocycle-containing group having 3 to 20 carbon atoms, or -O-R²,
a methylene group in the alkyl group or arylalkyl group represented by R¹¹, R¹², R¹³, R¹⁴, and R¹⁵ is optionally replaced with one or more groups selected from -O-, -CO-, -OC-, -O-CO-, and -CO-O- (provided that oxygen atoms are not adjacent to each other when the methylene group is replaced with two or more groups), and
at least one of R¹¹, R¹², R¹³, R¹⁴, and R¹⁵ is not a hydrogen atom;

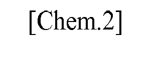

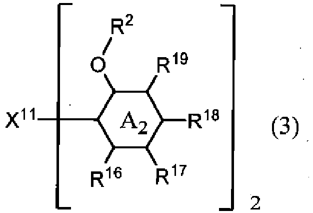

wherein X¹¹ represents a group represented by General Formula (3-1) below,
R¹⁶, R¹⁷, R¹⁸, and R¹⁹ represent a hydrogen atom, a halogen atom, a cyano group, a hydroxyl group, a nitro group, a carboxyl group, an unsubstituted or substituted alkyl group having 1 to 40 carbon atoms, an unsubstituted or substituted alkoxy group having 1 to 8 carbon atoms, an unsubstituted or substituted aryl group having 6 to 30 carbon atoms, an unsubstituted or substituted arylalkyl group having 7 to 30 carbon atoms, or an unsubstituted or substituted heterocycle-containing group having 3 to 20 carbon atoms,
at least one of R¹⁶, R¹⁷, R¹⁸, and R¹⁹ is not a hydrogen atom, and
R² and a ring A² are the same as R² and the ring A² in General Formula (2),
[Chem.3]

*-Z¹-X¹-Z²-* (3-1)

wherein X¹ represents -CR³⁰R³¹-, -NR³²-, an unsubstituted or substituted divalent aliphatic hydrocarbon group having 1 to 120 carbon atoms, an unsubstituted or substituted divalent aromatic ring-containing hydrocarbon group having 6 to 35 carbon atoms, an unsubstituted or substituted divalent heterocycle-containing group having 3 to 35 carbon atoms, or a substituent represented by one of formulae (3-2) to (3-5) below,
the aliphatic hydrocarbon group is optionally interrupted by -O-, -S-, -CO-, -COO-, -OCO-, or -NH-, or a linking group in which these groups are used in combination in a state in which oxygen atoms are not adjacent to each other,
R³⁰ and R³¹ represent a hydrogen atom, an unsubstituted or substituted alkyl group having 1 to 10 carbon atoms, an unsubstituted or substituted alkoxy group having 1 to 8 carbon atoms, an unsubstituted or substituted aryl group having 6 to 30 carbon atoms, or an unsubstituted or substituted arylalkyl group having 7 to 30 carbon atoms,
Z¹ and Z² independently represent a direct bond, -O-, -S-, >CO, -CO-O-, -O-CO-, -SO₂-, -SS-, -SO-, >NR³³, an unsubstituted or substituted aliphatic hydrocarbon group having 1 to 35 carbon atoms, an unsubstituted or substituted aromatic ring-containing hydrocarbon group having 6 to 35 carbon atoms, or an unsubstituted or substituted heterocycle-containing group having 3 to 35 carbon atoms, and
R³² and R³³ represent a hydrogen atom, an unsubstituted or substituted aliphatic hydrocarbon group having 1 to 35 carbon atoms, an unsubstituted or substituted aromatic ring-containing hydrocarbon group having 6 to 35 carbon atoms, or an unsubstituted or substituted heterocycle-containing group having 3 to 35 carbon atoms,

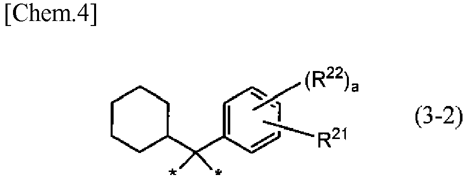

wherein R²¹ represents a hydrogen atom, an unsubstituted or substituted phenyl group, or an unsubstituted or substituted cycloalkyl group having 3 to 10 carbon atoms,
R²² represents an unsubstituted or substituted alkyl group having 1 to 10 carbon atoms, an unsubstituted or substituted alkoxy group having 1 to 8 carbon atoms, an unsubstituted or substituted alkenyl group having 2 to 10 carbon atoms, or a halogen atom, and
a is an integer from 0 to 5,

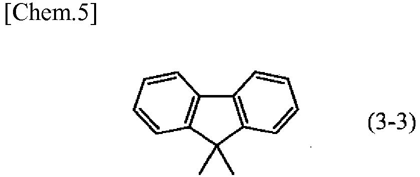

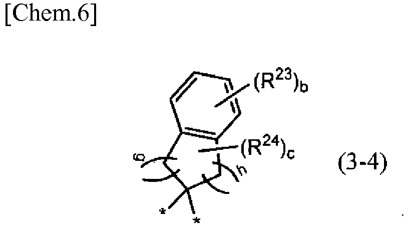

wherein R²³ and R²⁴ independently represent an unsubstituted or substituted alkyl group having 1 to 10 carbon atoms, an unsubstituted or substituted alkoxy group having 1 to 8 carbon atoms, an unsubstituted or substituted aryl group having 6 to 30 carbon atoms, an unsubstituted or substituted aryloxy group having 6 to 30 carbon atoms, an unsubstituted or substituted arylthio group having 6 to 30 carbon atoms, an unsubstituted or substituted arylalkenyl group having 6 to 30 carbon atoms, an unsubstituted or substituted arylalkyl group having 7 to 30 carbon atoms, an unsubstituted or substituted heterocyclic group having 3 to 20 carbon atoms, or a halogen atom,
a methylene group in the alkyl group, the alkoxy group, or the arylalkyl group is optionally replaced with an unsaturated bond, -O-, or -S-,
R²³ optionally forms a ring together with adjacent R²³,
b represents a number from 0 to 4,
c represents a number from 0 to 8,
g represents a number from 0 to 4,
h represents a number from 0 to 4, and
a total number of g and h is 2 to 4, and 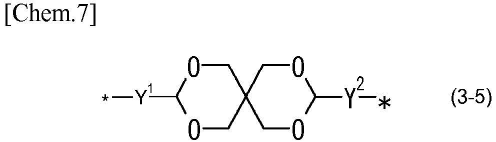
wherein Y¹ and Y² independently represent an alkylene group having 1 to 8 carbon atoms, and a methylene group in the alkylene group is optionally replaced with one or more groups selected from -O-, -CO-, -OC-, -O-CO-, and -CO-O-; and 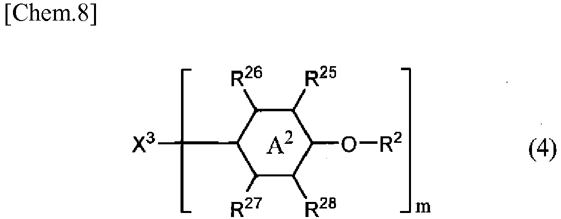
wherein m=2 to 6,
X³ is a group represented by General Formula (3-1) when m=2, X³ is a group represented by General Formula (4-1) when m=3, X³ is a group represented by General Formula (4-2) when m=4, X³ is a group represented by General Formula (4-3) when m=5, and X³ is a group represented by General Formula (4-4) when m=6,
R²⁵, R²⁶, R²⁷, and R²⁸ represent a hydrogen atom, a halogen atom, a cyano group, a hydroxyl group, a nitro group, a carboxyl group, an unsubstituted or substituted alkyl group having 1 to 40 carbon atoms, an unsubstituted or substituted alkoxy group having 1 to 8 carbon atoms, an unsubstituted or substituted aryl group having 6 to 30 carbon atoms, an unsubstituted or substituted arylalkyl group having 7 to 30 carbon atoms, or an unsubstituted or substituted heterocycle-containing group having 3 to 20 carbon atoms,
at least one of R²⁵, R²⁶, R²⁷, and R²⁸ is not a hydrogen atom, and
R² and a ring A² are the same as R² and the ring A² in General Formula (2),

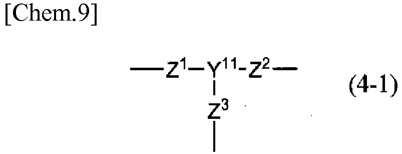

wherein Y¹¹ represents an unsubstituted or substituted trivalent aliphatic hydrocarbon group having 1 to 35 carbon atoms, an unsubstituted or substituted trivalent aromatic ring-containing hydrocarbon group having 6 to 35 carbon atoms, or an unsubstituted or substituted trivalent heterocycle-containing group having 3 to 35 carbon atoms,
Z¹, Z², and Z³ independently represent a direct bond, -O-, -S-, >CO, -CO-O-, -O-CO-, -SO₂-, -SS-, -SO-, >NR³⁴, >PR³⁴, an unsubstituted or substituted aliphatic hydrocarbon group having 1 to 35 carbon atoms, an unsubstituted or substituted aromatic ring-containing hydrocarbon group having 6 to 35 carbon atoms, or an unsubstituted or substituted heterocycle-containing group having 3 to 35 carbon atoms,
R³⁴ represents a hydrogen atom, an unsubstituted or substituted aliphatic hydrocarbon group having 1 to 35 carbon atoms, an unsubstituted or substituted aromatic ring-containing hydrocarbon group having 6 to 35 carbon atoms, or an unsubstituted or substituted heterocycle-containing group having 3 to 35 carbon atoms, and
the aliphatic hydrocarbon group is optionally interrupted by a carbon-carbon double bond, -O-, -CO-, -O-CO-, -CO-O-, or -SO₂-,

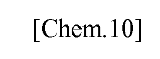

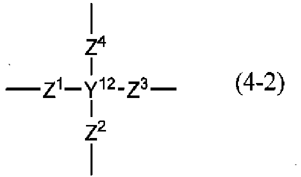

wherein Y¹² represents a carbon atom, an unsubstituted or substituted tetravalent aliphatic hydrocarbon group having 1 to 35 carbon atoms, an unsubstituted or substituted tetravalent aromatic ring-containing hydrocarbon group having 6 to 35 carbon atoms, or an unsubstituted or substituted tetravalent heterocycle-containing group having 3 to 35 carbon atoms,
the aliphatic hydrocarbon group is optionally interrupted by -COO-, -O-, -OCO-, -NHCO-, -NH-, or -CONH-, and
Z¹ to Z⁴ independently represent the same group as the group represented by Z¹ to Z³ in General Formula (4-1),

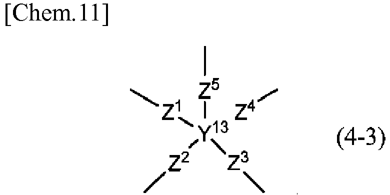

wherein Y¹³ represents an unsubstituted or substituted pentavalent aliphatic hydrocarbon group having 2 to 35 carbon atoms, an unsubstituted or substituted pentavalent aromatic ring-containing hydrocarbon group having 6 to 35 carbon atoms, or an unsubstituted or substituted pentavalent heterocycle-containing group having 3 to 35 carbon atoms,
the aliphatic hydrocarbon group is optionally interrupted by -COO-, -O-, -OCO-, -NHCO-, -NH-, or -CONH-, and
Z¹ to Z⁵ independently represent the same group as the group represented by Z¹ to Z³ in General Formula (4-1),

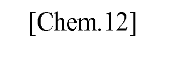

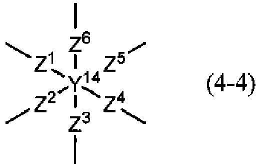

wherein Y¹⁴ represents an unsubstituted or substituted hexavalent aliphatic hydrocarbon group having 2 to 35 carbon atoms, an unsubstituted or substituted hexavalent aromatic ring-containing hydrocarbon group having 6 to 35 carbon atoms, or an unsubstituted or substituted hexavalent heterocycle-containing group having 3 to 35 carbon atoms,
the aliphatic hydrocarbon group is optionally interrupted by -COO-, -O-, -OCO-, -NHCO-, -NH-, or -CONH-, and
Z¹ to Z⁶ independently represent the same group as the group represented by Z¹ to Z³ in General Formula (4-1).

### Advantageous Effects of the Invention

With the present invention, it is possible to provide an antioxidant for a thermoplastic resin that provides an excellent oxidation-preventing effect to a thermoplastic resin and can prevent a change in color caused by nitrogen oxides. Moreover, a thermoplastic resin composition into which the antioxidant for a thermoplastic resin has been blended can be preferably used in a vehicle member.

### Description of Embodiments

Hereinafter, an antioxidant for a thermoplastic resin according to the present invention will be described based on a preferred embodiment.

The antioxidant for a thermoplastic resin according to the present invention is a compound represented by any of General Formulae (2) to (4) above, and is characterized in that the antioxidant is inactive at room temperature or in a prebaking step at 150°C or lower due to a protecting group being introduced at a phenol site and is activated by being heated at 100 to 250°C or being heated at 80 to 200°C in the presence of an acid/base catalyst to eliminate the protecting group.

Examples of the six-membered hydrocarbon ring represented by the ring A² in General Formula (2) include aliphatic hydrocarbon rings such as cyclohexane, cyclohexene, and cyclohexadiene. Examples of the six-membered aromatic ring include aromatic hydrocarbon rings such as a benzene ring.

Examples of the six-membered heterocycle include piperidine, piperazine, morpholine, thiomorpholine, pyridine, pyrazine, pyrimidine, pyridazine, and triazine.

These rings are optionally condensed with another ring or substituted, and examples thereof include quinoline, isoquinoline, indole, julolidine, benzoxazole, benzotriazole, and azulene.

Examples of the halogen atom represented by R¹¹, R¹², R¹³, R¹⁴, and R¹⁵ include fluorine, chlorine, bromine, and iodine.

Examples of the alkyl group having 1 to 40 carbon atoms represented by R¹¹, R¹², R¹³, R¹⁴, and R¹⁵ include a methyl, an ethyl, a propyl, an iso-propyl, a butyl, a sec-butyl, a tert-butyl, an iso-butyl, an amyl, an iso-amyl, a tert-amyl, a cyclopentyl, a hexyl, a 2-hexyl, a 3-hexyl, a cyclohexyl, 4-methylcyclohexyl, a heptyl, a 2-heptyl, a 3-heptyl, an iso-heptyl, a tert-heptyl, a 1-octyl, an iso-octyl, a tert-octyl, a nonyl, an isononyl, a decyl, an undecyl, a dodecyl, a tridecyl, an isotridecyl, a tetradecyl, a pentadecyl, a hexadecyl, a heptadecyl, an octadecyl, an adamantyl, a 1-adamantyl, a 2-adamantyl, a 2-methyl-1-adamantyl, a 2-methyl-2-adamantyl, a 2-ethyl-1-adamantyl, a 2-ethyl-2-adamantyl, a 2-norbornyl, and a 2-norbornylmethyl.

Examples of the alkoxy group having 1 to 8 carbon atoms represented by R¹¹, R¹², R¹³, R¹⁴, and R¹⁵ include a methyloxy, an ethyloxy, an iso-propyloxy, a butyloxy, a sec-butyloxy, a tert-butyloxy, an iso-butyloxy, an amyloxy, an iso-amyloxy, a tert-amyloxy, a hexyloxy, a 2-hexyloxy, a 3-hexyloxy, a cyclohexyloxy, a 4-methylcyclohexyloxy, a heptyloxy, a 2-heptyloxy, a 3-heptyloxy, an iso-heptyloxy, a tert-heptyloxy, a 1-octyloxy, an iso-octyloxy, and a tert-octyloxy.

Examples of the aryl group having 6 to 30 carbon atoms represented by R¹¹, R¹², R¹³, R¹⁴, and R¹⁵ include a phenyl, a naphthyl, an anthracenyl, a phenanthryl, a fluorenyl, an indenyl, a 2-methylphenyl, a 3-methylphenyl, a 4-methylphenyl, a 4-vinylphenyl, a 3-iso-propylphenyl, a 4-iso-propylphenyl, a 4-butylphenyl, a 4-iso-butylphenyl, a 4-tert-butylphenyl, a 4-hexylphenyl, a 4-cyclohexylphenyl, a 4-octylphenyl, a 4-(2-ethylhexyl)phenyl, a 4-stearylphenyl, a 2,3-dimethylphenyl, a 2,4-dimethylphenyl, a 2,5-dimethylphenyl, a 2,6-dimethylphenyl, a 3,4-dimethylphenyl, a 3,5-dimethylphenyl, a 2,4-di-tert-butylphenyl, a 2,5-di-tert-butylphenyl, a 2,6-di-tert-butylphenyl, a 2,4-di-tert-pentylphenyl, a 2,5-di-tert-amylphenyl, a 2,5-di-tert-octylphenyl, a 2,4-dicumylphenyl, a 4-cyclohexylphenyl, a (1,1'-biphenyl)-4-yl, a 2,4,5-trimethylphenyl, and a ferrocenyl.

Examples of the arylalkyl group having 7 to 30 carbon atoms represented by R¹¹, R¹², R¹³, R¹⁴, and R¹⁵ include a benzyl, a 1-methyl-1-phenylethyl, a 1-naphthylmethyl, a 9-anthracenylmethyl, a fluorenyl, an indenyl, a 9-fluorenylmethyl, a 2-phenylpropan-2-yl, a diphenylmethyl, a triphenylmethyl, a phenethyl, a styryl, and a cinnamyl.

Examples of the heterocycle-containing group having 3 to 20 carbon atoms represented by R¹¹, R¹², R¹³, R¹⁴, and R¹⁵ include a pyridyl, a pyrimidyl, a pyridazyl, a piperidyl, a pyranyl, a pyrazolyl, a triazile, a pyrrolyl, a quinolyl, an isoquinolyl, an imidazolyl, a benzimidazolyl, a triazolyl, a furyl, a furanyl, a benzofuranyl, a thienyl, a thiophenyl, a benzothiophenyl, a thiadiazolyl, a thiazolyl, a benzothiazolyl, an oxazolyl, a benzoxazolyl, an isothiazolyl, an isooxazolyl, an indolyl, a 2-pyrrolidinon-1-yl, a 2-piperidon-1-yl, a 2,4-dioxyimidazolidin-3-yl, and a 2,4-dioxyoxazolidin-3-yl.

Examples of the alkyl group having 1 to 20 carbon atoms represented by R² include a methyl, an ethyl, a propyl, an isopropyl, a butyl, a sec-butyl, a tert-butyl, an isobutyl, a pentyl, an isopentyl, a tert-pentyl, a hexyl, a 2-hexyl, a 3-hexyl, a heptyl, a 2-heptyl, a 3-heptyl, an isoheptyl, a tert-heptyl, a n-octyl, an isooctyl, a tert-octyl, a nonyl, an isononyl, a decyl, an undecyl, and a dodecyl.

Examples of the alkenyl group having 2 to 20 carbon atoms represented by R² include a vinyl, a 1-propenyl, an isopropenyl, a 2-methyl-1-propenyl, a 1-butenyl, a 2-butenyl, a 3-butenyl, a 2-ethyl-1-butenyl, a 1-pentenyl, a 2-pentenyl, a 3-pentenyl, a 4-pentenyl, a 4-methyl-3-pentenyl, a 1-hexenyl, a 2-hexenyl, a 3-hexenyl, a 4-hexenyl, and a 5-hexenyl.

Examples of the aryl group having 6 to 30 carbon atoms represented by R² include those shown as the examples of the aryl group having 6 to 30 carbon atoms represented by R¹¹ and the like.

Examples of the arylalkyl group having 7 to 30 carbon atoms represented by R² include those shown as the examples of the arylalkyl group having 7 to 30 carbon atoms represented by R¹¹ and the like.

Examples of the heterocycle-containing group having 3 to 35 carbon atoms represented by R² include a pyridyl, a pyrimidyl, a pyridazyl, a piperidyl, a pyranyl, a pyrazolyl, a triazile, a pyrrolyl, a quinolyl, an isoquinolyl, an imidazolyl, a benzimidazolyl, a triazolyl, a furyl, a furanyl, a benzofuranyl, a thienyl, a thiophenyl, a benzothiophenyl, a thiadiazolyl, a thiazolyl, a benzothiazolyl, an oxazolyl, a benzoxazolyl, an isothiazolyl, an isooxazolyl, an indolyl, a 2-pyrrolidinon-1-yl, a 2-piperidon-1-yl, a 2,4-dioxyimidazolidin-3-yl, and a 2,4-dioxyoxazolidin-3-yl.

Examples of the trialkylsilyl group represented by R² include silyl groups such as trimethylsilyl, triethylsilyl, and ethyldimethylsilyl substituted with alkyl groups having 1 to 6 carbon atoms (three alkyl groups may be the same or different).

Examples of the alkyl group having 1 to 8 carbon atoms represented by R' include alkyl groups having a predetermined number of carbon atoms out of the above-mentioned alkyl groups represented by R¹¹ and the like.

In the present invention, alkyl groups, alkoxy groups, and arylalkyl groups have a linear methylene group site or a branched methylene group site.

In the present invention, examples of the substituent with which the methylene group of the alkyl group, alkoxy group, and arylalkyl group mentioned above is substituted include ethylenically unsaturated groups such as a vinyl, an allyl, an acryl, and a methacryl; halogen atoms such as fluorine, chlorine, bromine, and iodine; acyl groups such as an acetyl, a 2-chloroacetyl, a propionyl, an octanoyl, an acryloyl, a methacryloyl, a phenylcarbonyl (benzoyl), a phthaloyl, a 4-trifluoromethylbenzoyl, a pivaloyl, a salicyloyl, an oxaloyl, a stearoyl, a methoxycarbonyl, an ethoxycarbonyl, a tert-butoxycarbonyl, a n-octadecyloxycarbonyl, and a carbamoyl; acyloxy groups such as an acetyloxy and a benzoyloxy; substituted amino groups such as an amino, an ethylamino, a dimethylamino, a diethylamino, a butylamino, a cyclopentylamino, a 2-ethylhexylamino, a dodecylamino, an anilino, a chlorophenylamino, a toluidino, an anisidino, an N-methyl-anilino, a diphenylamino, a naphthylamino, a 2-pyridylamino, a methoxycarbonylamino, a phenoxycarbonylamino, an acetylamino, a benzoylamino, a formylamino, a pivaloylamino, a lauroylamino, a carbamoylamino, an N,N-dimethylaminocarbonylamino, an N,N-diethylaminocarbonylamino, a morpholinocarbonylamino, a methoxycarbonylamino, an ethoxycarbonylamino, a t-butoxycarbonylamino, a n-octadecyloxycarbonylamino, a N-methyl-methoxycarbonylamino, a phenoxycarbonylamino, a sulfamoylamino, an N,N-dimethylaminosulfonylamino, a methylsulfonylamino, a butylsulfonylamino, and a phenylsulfonylamino; a sulfonamide group, a sulfonyl group, a carboxyl group, a cyano group, a sulfo group, a hydroxyl group, a nitro group, a mercapto group, an imido group, a carbamoyl group, a sulfonamide group, a phosphonate group, a phosphate group, and salts of a carboxyl group, a sulfo group, a phosphonate group, and a phosphate group.

In the present invention, the substituents may be further substituted with the above-mentioned substituents, unless otherwise stated. Moreover, the above-mentioned substituents are also taken as examples of substituents of the aryl group, heterocycle-containing group, and alkenyl group in General Formula (2).

When the above-mentioned substituents include carbon atoms, the number of carbon atoms in the above-mentioned groups includes the number of carbon atoms in these substituents.

Examples of the halogen atom, the alkyl group having 1 to 40 carbon atoms, the alkoxy group having 1 to 8 carbon atoms, the aryl group having 6 to 30 carbon atoms, the arylalkyl group having 7 to 30 carbon atoms, and the heterocycle-containing group having 3 to 20 carbon atoms represented by R¹⁶, R¹⁷, R¹⁸, and R¹⁹ in General Formula (3) above include those shown as the examples in the description of R¹¹ and the like in General Formula (2) above.

Examples of the aliphatic hydrocarbon group having 1 to 35 carbon atoms represented by R³² and R³³ in General Formula (3-1) above include alkyl groups such as a methyl, an ethyl, a propyl, an isopropyl, a cyclopropyl, a butyl, a sec-butyl, a tert-butyl, an isobutyl, an amyl, an isoamyl, a tert-amyl, a cyclopentyl, a hexyl, a 2-hexyl, a 3-hexyl, a cyclohexyl, a bicyclohexyl, a 1-methylcyclohexyl, a heptyl, a 2-heptyl, a 3-heptyl, an isoheptyl, a tert-heptyl, a n-octyl, an isooctyl, a tert-octyl, a 2-ethylhexyl, a nonyl, an isononyl, and a decyl; alkoxy groups such as a methyloxy, an ethyloxy, a propyloxy, an isopropyloxy, a butyloxy, a sec-butyloxy, a tert-butyloxy, an isobutyloxy, an amyloxy, an isoamyloxy, a tert-amyloxy, a hexyloxy, a cyclohexyloxy, a heptyloxy, an isoheptyloxy, a tert-heptyloxy, a n-octyloxy, an isooctyloxy, a tert-octyloxy, a 2-ethylhexyloxy, a nonyloxy, and a decyloxy; alkylthio groups such as a methylthio, an ethylthio, a propylthio, an isopropylthio, a butylthio, a sec-butylthio, a tert-butylthio, an isobutylthio, an amylthio, an isoamylthio, a tert-amylthio, a hexylthio, a cyclohexylthio, a heptylthio, an isoheptylthio, a tert-heptylthio, a n-octylthio, an isooctylthio, a tert-octylthio, and a 2-ethylhexylthio; alkenyl groups such as a vinyl, a 1-methylethenyl, a 2-methylethenyl, a 2-propenyl, a 1-methyl-3-propenyl, a 3-butenyl, a 1-methyl-3-butenyl, an isobutenyl, a 3-pentenyl, a 4-hexenyl, a cyclohexenyl, a bicyclohexenyl, a heptenyl, an octenyl, a decenyl, a pentadecenyl, an eicosenyl, and a tricosenyl; and the like, and groups obtained by substituting these groups with later-described substituents.

Examples of the divalent aliphatic hydrocarbon group having 1 to 120 carbon atoms represented by X¹ include divalent groups obtained by substituting, with Z¹ and Z², the aforementioned groups; alkylenes such as a methylene, an ethylene, a propylene, a butylene, and a butyldiyl; groups obtained by replacing the methylene chain in the above-mentioned alkylenes with -O-, -S-, -CO-O-, or -O-CO-; residues of diols such as ethanediol, propanediol, butanediol, pentanediol, and hexanediol; residues of dithiols such as ethanedithiol, propanedithiol, butanedithiol, pentanedithiol, and hexanedithiol; and the like, and groups obtained by substituting these groups with later-described substituents.

It should be noted that groups having a predetermined number of carbon atoms out of the above-mentioned divalent aliphatic hydrocarbon groups are also taken as examples of the aliphatic hydrocarbon having 1 to 35 carbon atoms represented by Z¹ to Z⁶.

Examples of the aromatic ring-containing hydrocarbon group having 6 to 35 carbon atoms represented by R³² and R³³ include arylalkyl groups such as a benzyl, a phenethyl, a diphenylmethyl, a triphenylmethyl, a styryl, and a cinnamyl; aryl groups such as a phenyl and a naphthyl; aryloxy groups such as a phenoxy and a naphthyloxy; arylthio groups such as a phenylthio and a naphthylthio; and the like, and groups obtained by substituting these groups with later-described substituents, and examples of the divalent aromatic ring-containing hydrocarbon groups having 6 to 35 carbon atoms represented by X¹ include divalent groups obtained by substituting, with Z¹ and Z², the aforementioned groups; arylene groups such as a phenylene and a naphthylene; residues of bifunctional phenols such as catechol and bisphenol; 2,4,8,10-tetraoxaspiro[5,5]undecane; and the like, and groups obtained by substituting these groups with later-described substituents.

It should be noted that the above-mentioned divalent aromatic ring-containing hydrocarbon groups are also taken as examples of the aromatic ring-containing hydrocarbon group having 6 to 35 carbon atoms represented by Z¹ to Z⁶.

Examples of the heterocycle-containing group having 3 to 35 carbon atoms represented by R³² and R³³ include a pyridyl, a pyrimidyl, a pyridazyl, a piperidyl, a pyranyl, a pyrazolyl, a triazile, a pyrrolyl, a quinolyl, an isoquinolyl, an imidazolyl, a benzimidazolyl, a triazolyl, a furyl, a furanyl, a benzofuranyl, a thienyl, a thiophenyl, a benzothiophenyl, a thiadiazolyl, a thiazolyl, a benzothiazolyl, an oxazolyl, a benzoxazolyl, an isothiazolyl, an isooxazolyl, an indolyl, a 2-pyrrolidinon-1-yl, a 2-piperidon-1-yl, a 2,4-dioxyimidazolidin-3-yl, a 2,4-dioxyoxazolidin-3-yl, a benzotriazoyl, and the like, and groups obtained by substituting these groups with later-described substituents, and examples of the divalent heterocycle-containing groups having 3 to 35 carbon atoms represented by X¹ include divalent groups obtained by substituting the aforementioned groups with Z¹ and Z².

It should be noted that the above-mentioned divalent heterocycle-containing groups are also taken as examples of the heterocycle-containing group having 3 to 35 carbon atoms represented by Z¹ to Z⁶.

Examples of the alkyl group having 1 to 10 carbon atoms represented by R³⁰ and R³¹ include groups having a predetermined number of carbon atoms out of the examples of the alkyl groups having 1 to 40 carbon atoms represented by R¹¹ and the like.

Examples of the alkoxy group having 1 to 8 carbon atoms, the aryl group having 6 to 30 carbon atoms, and the arylalkyl group having 7 to 30 carbon atoms represented by R³⁰ and R³¹ include those shown as the examples in the description of R¹¹ and the like in General Formula (2) above.

With regard to the substituents represented by the formula (3-2) above, examples of the cycloalkyl group having 3 to 10 carbon atoms represented by R²¹ include a cyclopropyl, a cyclobutyl, a cyclopentyl, a cycloheptyl, and a cyclooctyl.

Examples of the alkyl group having 1 to 10 carbon atoms represented by R²² include groups having a predetermined numbers of carbon atoms out of the groups shown as the examples of the alkyl group having 1 to 40 carbon atoms represented by R¹¹ and the like.

Examples of the alkoxy group having 1 to 8 carbon atoms represented by R²² include the groups shown as the examples of the alkoxy group having 1 to 8 carbon atoms represented by R¹¹ and the like.

Examples of the alkenyl group having 2 to 10 carbon atoms represented by R²² include groups having a predetermined number of carbon atoms out of the groups shown as the examples of the alkenyl group having 2 to 20 carbon atoms represented by R².

Examples of the substituent with which the aliphatic hydrocarbon group, aromatic ring-containing hydrocarbon group, and heterocycle-containing group mentioned above include ethylenically unsaturated groups such as a vinyl, an allyl, an acryl, and a methacryl; halogen atoms such as fluorine, chlorine, bromine, and iodine; acyl groups such as an acetyl, a 2-chloroacetyl, a propionyl, an octanoyl, an acryloyl, a methacryloyl, a phenylcarbonyl (benzoyl), a phthaloyl, a 4-trifluoromethylbenzoyl, a pivaloyl, a salicyloyl, an oxaloyl, a stearoyl, a methoxycarbonyl, an ethoxycarbonyl, a t-butoxycarbonyl, a n-octadecyloxycarbonyl, and a carbamoyl; acyloxy groups such as an acetyloxy and a benzoyloxy; substituted amino groups such as an amino, an ethylamino, a dimethylamino, a diethylamino, a butylamino, a cyclopentylamino, a 2-ethylhexylamino, a dodecylamino, an anilino, a chlorophenylamino, a toluidino, an anisidino, a N-methyl-anilino, a diphenylamino, a naphthylamino, a 2-pyridylamino, a methoxycarbonylamino, a phenoxycarbonylamino, an acetylamino, a benzoylamino, a formylamino, a pivaloylamino, a lauroylamino, a carbamoylamino, a N,N-dimethylaminocarbonylamino, a N,N-diethylaminocarbonylamino, a morpholinocarbonylamino, a methoxycarbonylamino, an ethoxycarbonylamino, a t-butoxycarbonylamino, a n-octadecyloxycarbonylamino, a N-methyl-methoxycarbonylamino, a phenoxycarbonylamino, a sulfamoylamino, a N,N-dimethylaminosulfonylamino, a methylsulfonylamino, a butylsulfonylamino, and a phenylsulfonylamino; a sulfonamide group, a sulfonyl group, a carboxyl group, a cyano group, a sulfo group, a hydroxyl group, a nitro group, a mercapto group, an imido group, a carbamoyl group, a sulfonamide group, a phosphonate group, a phosphate group, and salts of a carboxyl group, a sulfo group, a phosphonate group, and a phosphate group. These groups may be further substituted. The carboxyl group and sulfo group may form a salt. These substituents are also taken as examples of substituent in General Formula (4) below.

With regard to the groups represented by the formula (3-4) above, examples of the alkyl group having 1 to 10 carbon atoms represented by R²³ and R²⁴ include groups having a predetermined numbers of carbon atoms out of the groups shown as the examples of the alkyl groups having 1 to 40 carbon atoms represented by R¹¹ and the like.

Examples of the halogen atom, the alkoxy group having 1 to 8 carbon atoms, the aryl group having 6 to 30 carbon atoms, the arylalkyl group having 7 to 30 carbon atoms, and the heterocycle-containing group having 3 to 20 carbon atoms represented by R²³ and R²⁴ include those shown as the examples in the description of R¹¹ and the like in General Formula (2) above.

Examples of the aryloxy group having 6 to 30 carbon atoms represented by R²³ and R²⁴ include groups such as a phenyloxy, a naphthyloxy, a 2-methylphenyloxy, a 3-methylphenyloxy, a 4-methylphenyloxy, a 4-vinylphenyldioxy, a 3-iso-propylphenyloxy, a 4-iso-propylphenyloxy, a 4-butylphenyloxy, a 4-tert-butylphenyloxy, a 4-hexylphenyloxy, a 4-cyclohexylphenyloxy, a 4-octylphenyloxy, a 4-(2-ethylhexyl)phenyloxy, a 2,3-dimethylphenyloxy, a 2,4-dimethylphenyloxy, a 2,5-dimethylphenyloxy, a 2.6-dimethylphenyloxy, a 3.4-dimethylphenyloxy, a 3.5-dimethylphenyloxy, a 2,4-di-tert-butylphenyloxy, a 2,5-di-tert-butylphenyloxy, a 2, 6-di-tert-butylphenyloxy, a 2.4-di-tert-pentylphenyloxy, a 2,5-di-tert-amylphenyloxy, a 4-cyclohexylphenyloxy, a 2,4,5-trimethylphenyloxy, and a ferrocenyloxy.

Examples of the arylthio group having 6 to 30 carbon atoms represented by R²³ and R²⁴ include groups obtained by replacing the oxygen atom of the above-mentioned aryloxy groups having 6 to 30 carbon atoms with a sulfur atom.

Examples of the arylalkenyl group having 8 to 30 carbon atoms represented by R²³ and R²⁴ include groups obtained by replacing the oxygen atom of the above-mentioned aryloxy groups having 6 to 30 carbon atoms with an alkenyl group such as a vinyl, an allyl, a 1-propenyl, an isopropenyl, a 2-butenyl, a 1,3-butadienyl, a 2-pentenyl, or a 2-octenyl.

With regard to the groups represented by General Formula (3-5) above, examples of the alkylene group having 1 to 8 carbon atoms represented by Y¹ and Y² include a methylene, an ethylene, a methylethylene, a propylene, a butylene, a 1-methylpropylene, a 2-methylpropylene, a 1,1-dimethylpropylene, a 1,2-dimethylpropylene, a 1,3-dimethylpropylene, a 1-methylbutylene, a 2-methylbutylene, a 3-methylbutylene, a 4-methylbutylene, a 2,4-dimethylbutylene, a 1,3-dimethylbutylene, a 1,1-dimethylbutylene, a pentylene, a 1-methylpentylene, a 2-methylpentylene, a 3-methylpentylene, a 1,1-dimethylpentylene, a 1,3-dimethylpentylene, a hexylene, a heptylene, and an octylene.

Examples of the halogen atom, the alkyl group having 1 to 40 carbon atoms, the alkoxy group having 1 to 8 carbon atoms, the aryl group having 6 to 30 carbon atoms, the arylalkyl group having 7 to 30 carbon atoms, and the heterocycle-containing group having 3 to 20 carbon atoms represented by R²⁵, R²⁶, R²⁷, and R²⁸ in General Formula (4) above include those shown as the examples in the description of R¹¹ and the like in General Formula (2) above.

Examples of the trivalent aliphatic hydrocarbon group having 1 to 35 carbon atoms represented by Y¹¹ in General Formula (4-1) above include trivalent groups obtained by substituting, with Z¹, Z², and Z³, the aliphatic hydrocarbon groups represented by R³² and R³³ in General Formula (3-1) above, the groups shown as the examples of the divalent aliphatic hydrocarbon groups represented by X¹ in General Formula (2) above, alkylidynes such as a propylidyne and a 1,1,3-butylidyne, and trivalent aliphatic hydrocarbon groups obtained by substituting these groups with the above-described substituents.

Examples of the trivalent aromatic ring-containing hydrocarbon group having 6 to 35 carbon atoms represented by Y¹¹ include trivalent groups obtained by substituting, with Z¹, Z², and Z³, the aromatic ring-containing hydrocarbon groups represented by R³² and R³³ in General Formula (3-1) above, the groups shown as the examples of the divalent aromatic ring-containing hydrocarbon groups represented by X¹ in General Formula (2) above, a phenyl-1,3,5-trimethylene, and the like, and groups obtained by substituting these groups with the above-described substituents.

Examples of the trivalent heterocycle-containing group having 3 to 35 carbon atoms represented by Y¹¹ include trivalent groups obtained by substituting, with Z¹, Z², and Z³, the heterocycle-containing groups represented by R³² and R³³ in General Formula (3-1) above, groups having an isocyanuric ring, groups having a triazine ring, and the like, and groups obtained by substituting these groups with the above-described substituents.

Examples of the aliphatic hydrocarbon group having 1 to 35 carbon atoms, aromatic hydrocarbon group having 6 to 35 carbon atoms, and heterocyclic group having 3 to 35 carbon atoms represented by R³⁴ respectively include the aliphatic hydrocarbon groups, aromatic ring-containing hydrocarbon groups, and heterocycle-containing groups shown as the examples in the description of R³² and R³³ in General Formula (3-1) above.

Examples of the tetravalent aliphatic hydrocarbon group having 1 to 35 carbon atoms represented by Y¹² in General Formula (4-2) above include tetravalent groups obtained by substituting, with Z¹, Z², Z³, and Z⁴, the groups shown as the examples of the monovalent to trivalent aliphatic hydrocarbon groups represented by Y¹¹ in General Formula (4-1) above, and the like.

Examples of the aromatic ring-containing hydrocarbon group having 6 to 35 carbon atoms represented by Y¹² include tetravalent groups obtained by substituting, with Z¹, Z², Z³, and Z⁴, the monovalent to trivalent aromatic ring-containing hydrocarbon groups represented by Y¹¹ in General Formula (4-1) above, and the like.

Examples of the tetravalent heterocycle-containing group having 3 to 35 carbon atoms represented by Y¹² include tetravalent groups obtained by substituting, with Z¹, Z², Z³, and Z⁴, the groups shown as the examples of the monovalent or trivalent heterocycle-containing groups represented by Y¹¹ in General Formula (4-1) above, and the like.

Examples of the pentavalent aliphatic hydrocarbon group having 2 to 35 carbon atoms represented by Y¹³ in General Formula (4-3) above include pentavalent groups obtained by substituting, with Z¹, Z², Z³, Z⁴, and Z⁵, the groups shown as the examples of the monovalent to trivalent aliphatic hydrocarbon groups represented by Y¹¹ in General Formula (4-1) above, and the like.

Examples of the pentavalent aromatic ring-containing hydrocarbon group having 6 to 35 carbon atoms represented by Y¹⁴ include pentavalent groups obtained by substituting, with Z¹, Z², Z³, Z⁴, and Z⁵, the groups shown as the examples of the monovalent to trivalent aromatic ring-containing hydrocarbon groups represented by Y¹¹ in General Formula (4-1) above, and the like.

Examples of the pentavalent heterocycle-containing group having 3 to 35 carbon atoms represented by Y¹⁴ include pentavalent groups obtained by substituting, with Z¹, Z², Z³, Z⁴, and Z⁵, the groups shown as the examples of the monovalent or trivalent heterocycle-containing groups represented by Y¹¹ in General Formula (4-1) above, and the like.

Examples of the hexavalent aliphatic hydrocarbon group having 2 to 35 carbon atoms represented by Y¹⁴ in General Formula (4-4) above include hexavalent groups obtained by substituting, with Z¹, Z², Z³, Z⁴, Z⁵, and Z⁶, the groups shown as the examples of the monovalent to trivalent aliphatic hydrocarbon groups represented by Y¹¹ in General Formula (4-1) above, and the like.

Examples of the hexavalent aromatic ring-containing hydrocarbon group having 6 to 35 carbon atoms represented by Y¹⁴ include hexavalent groups obtained by substituting, with Z¹, Z², Z³, Z⁴, Z⁵, and Z⁶, the groups shown as the examples of the monovalent to trivalent aromatic ring-containing hydrocarbon groups represented by Y¹¹ in General Formula (4-1) above, and the like.

Examples of the hexavalent heterocycle-containing group having 3 to 35 carbon atoms represented by Y¹⁴ include hexavalent groups obtained by substituting, with Z¹, Z², Z³, Z⁴, Z⁵, and Z⁶, the monovalent or trivalent heterocycle-containing groups represented by Y¹¹ in General Formula (4-1) above, and the like.

Specific examples of the antioxidants for a thermoplastic resin represented by any of General Formulae (2) to (4) above include compounds represented by Chemical Formulae 13 to 36 below, but the present invention is not limited to these compounds.

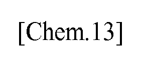

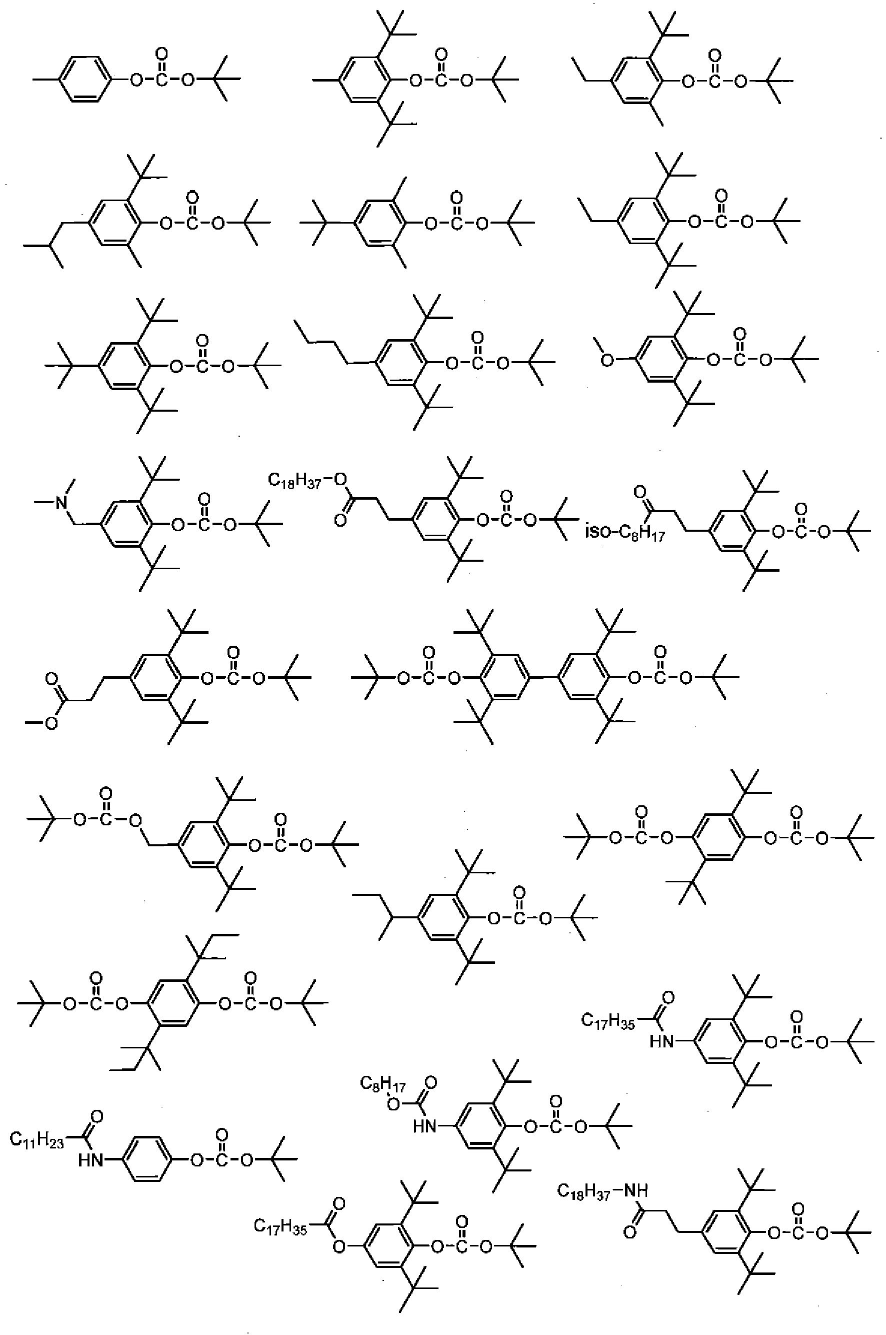

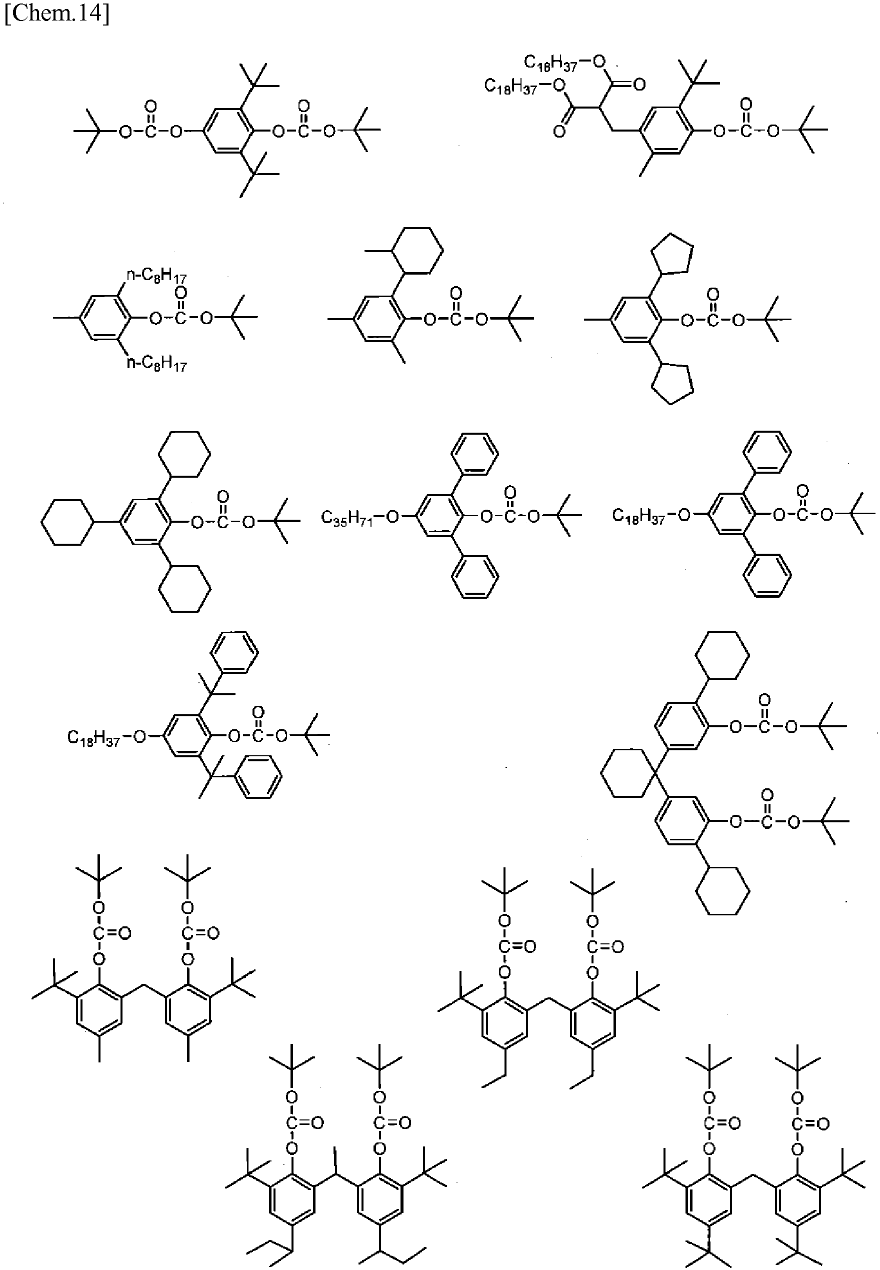

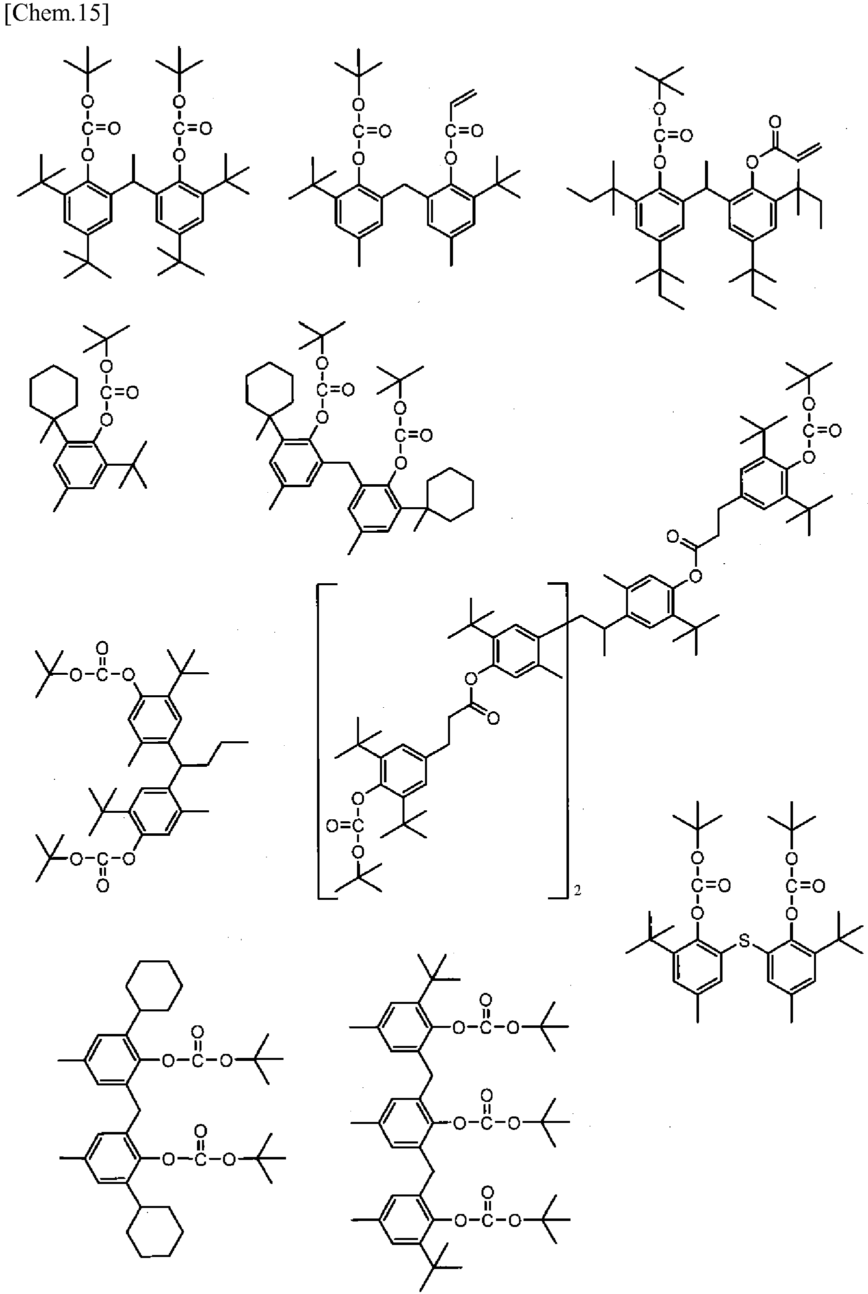

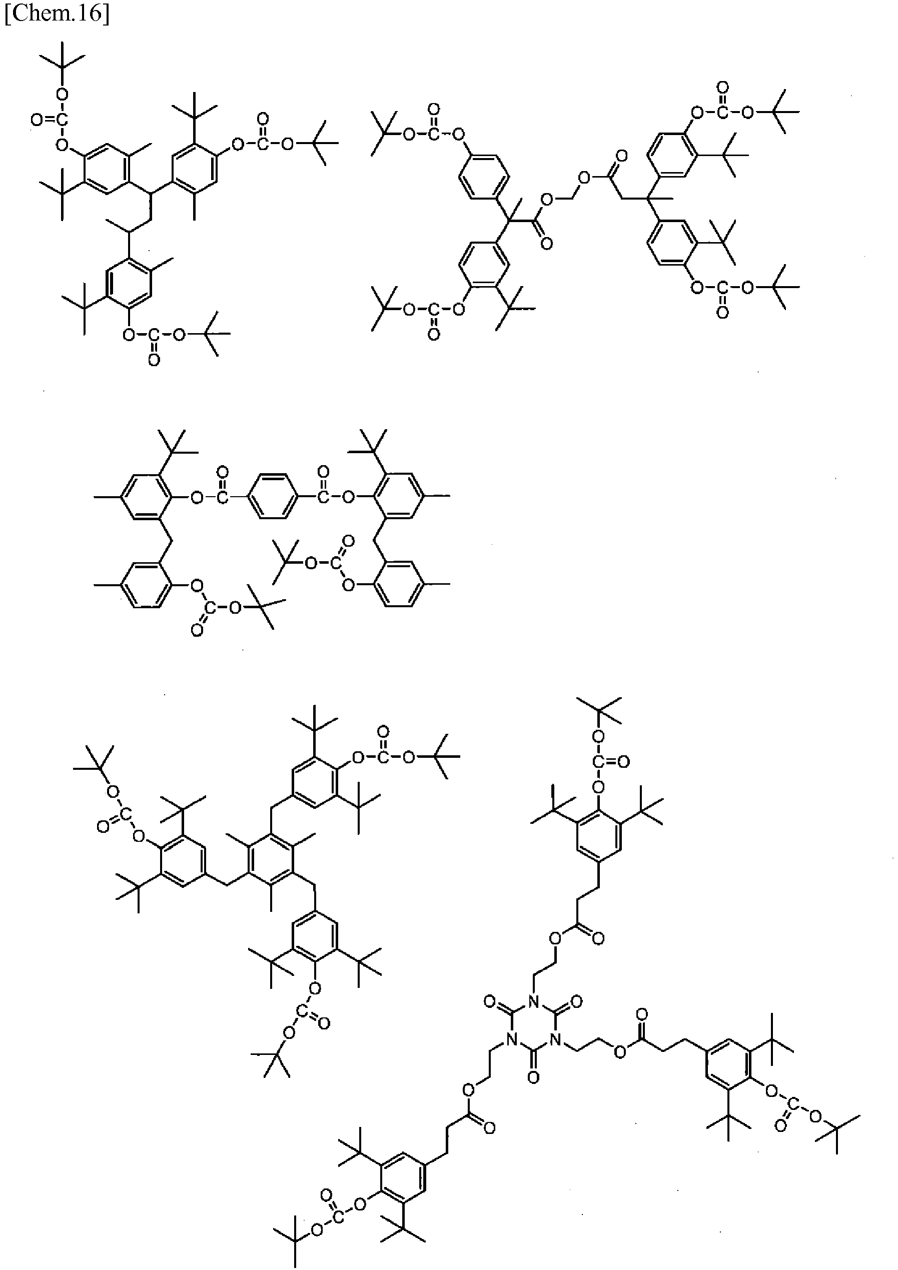

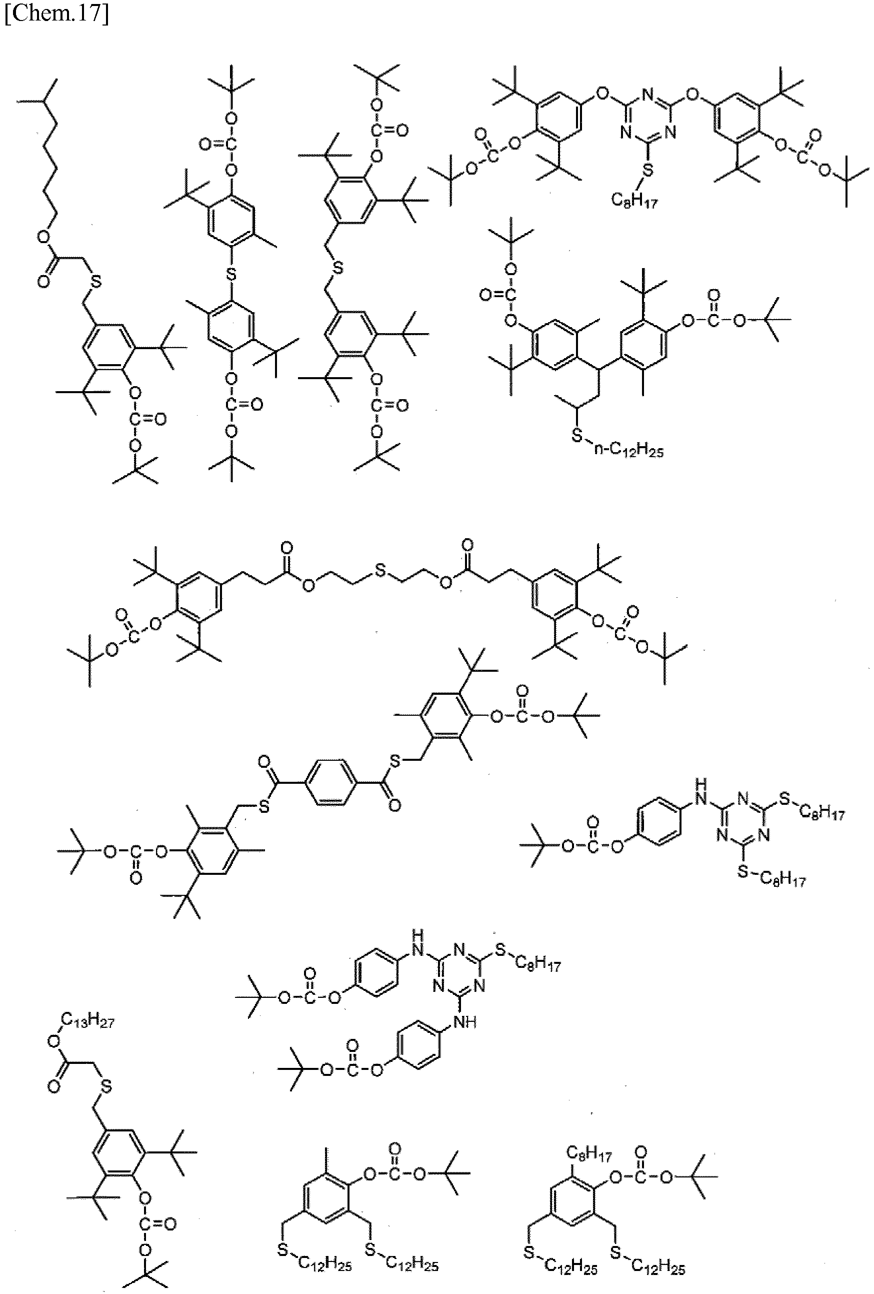

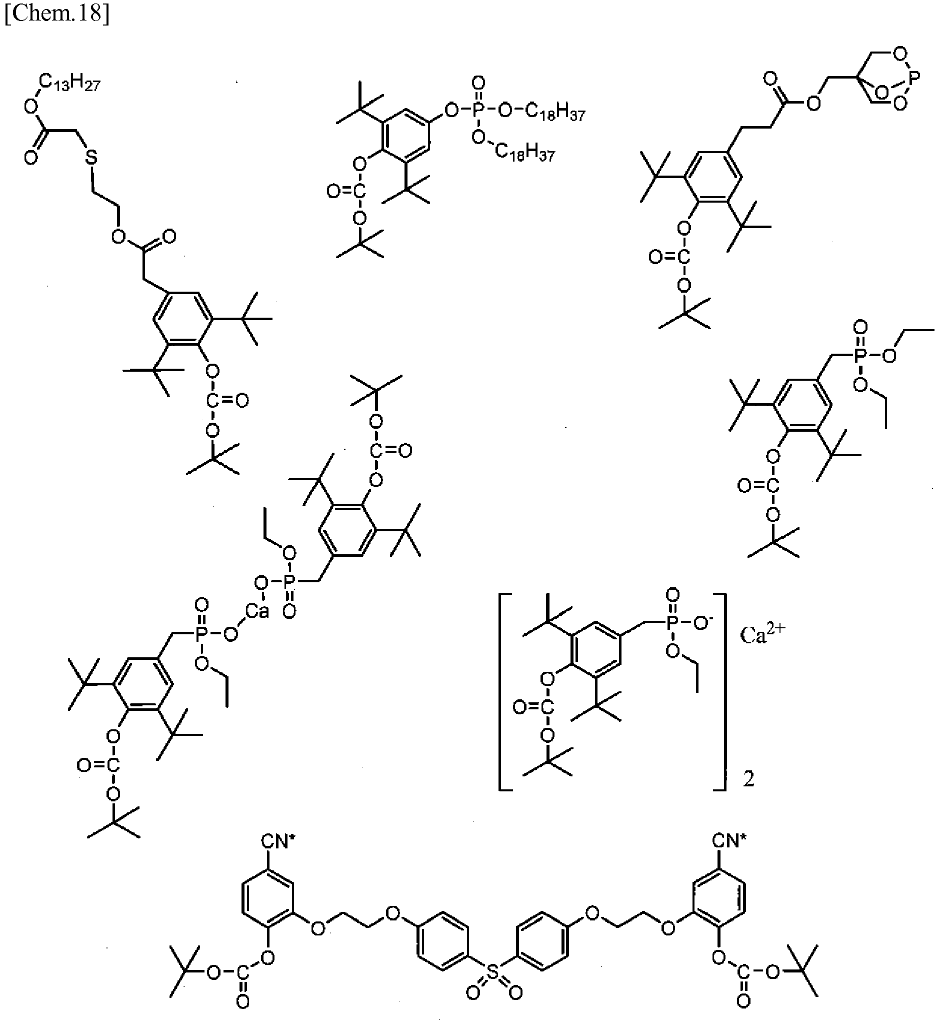

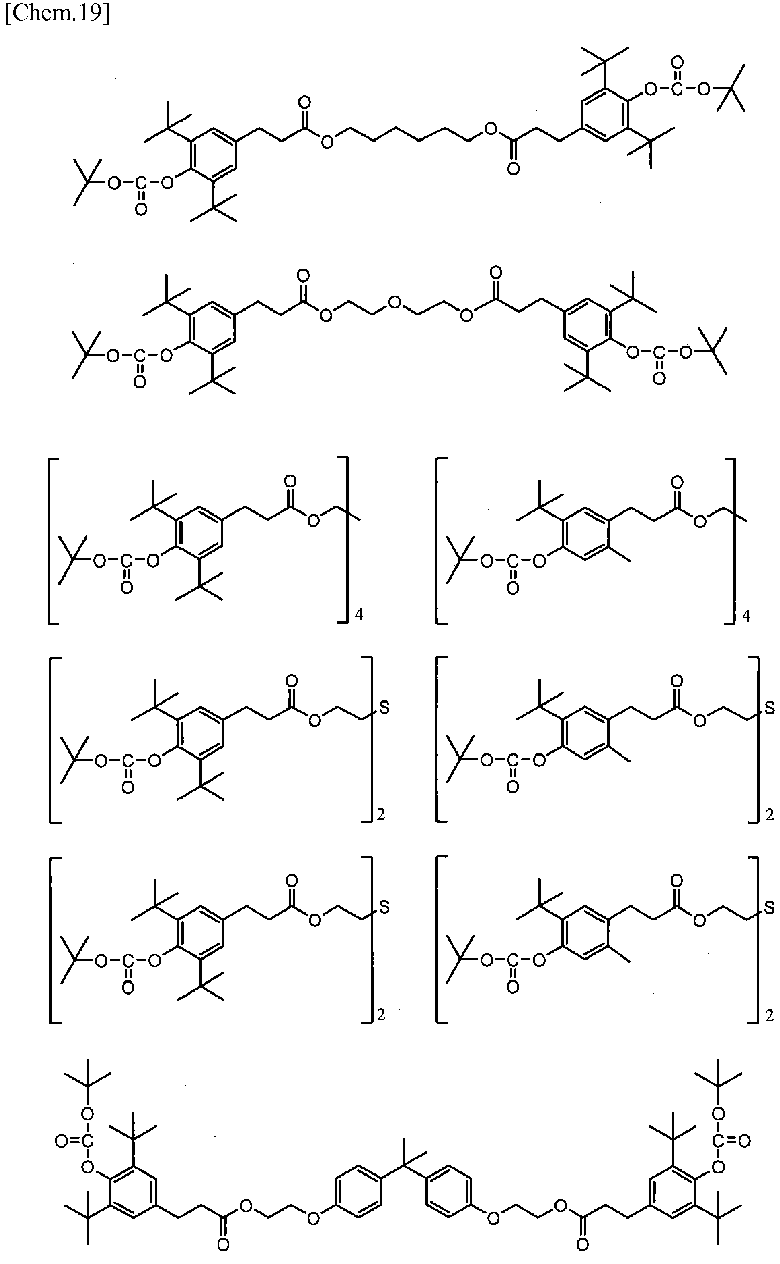

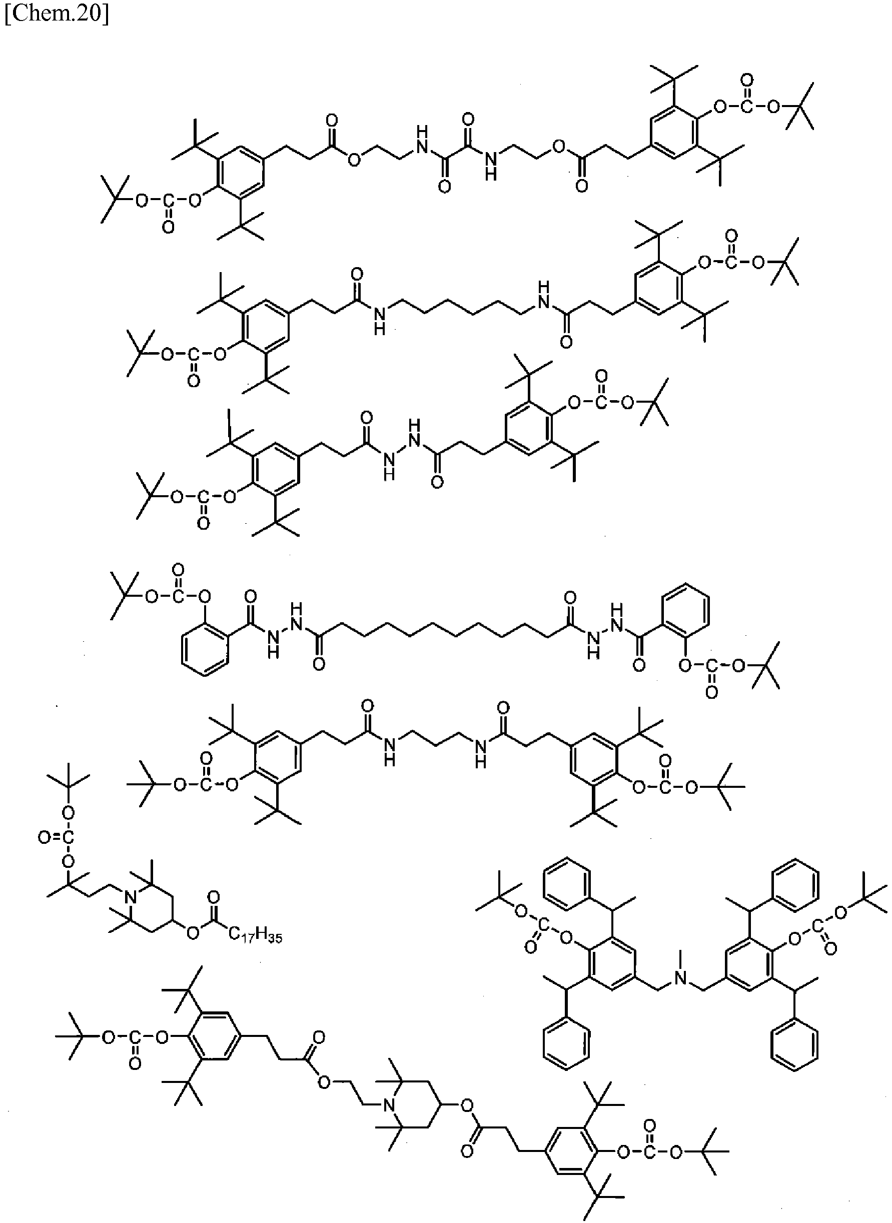

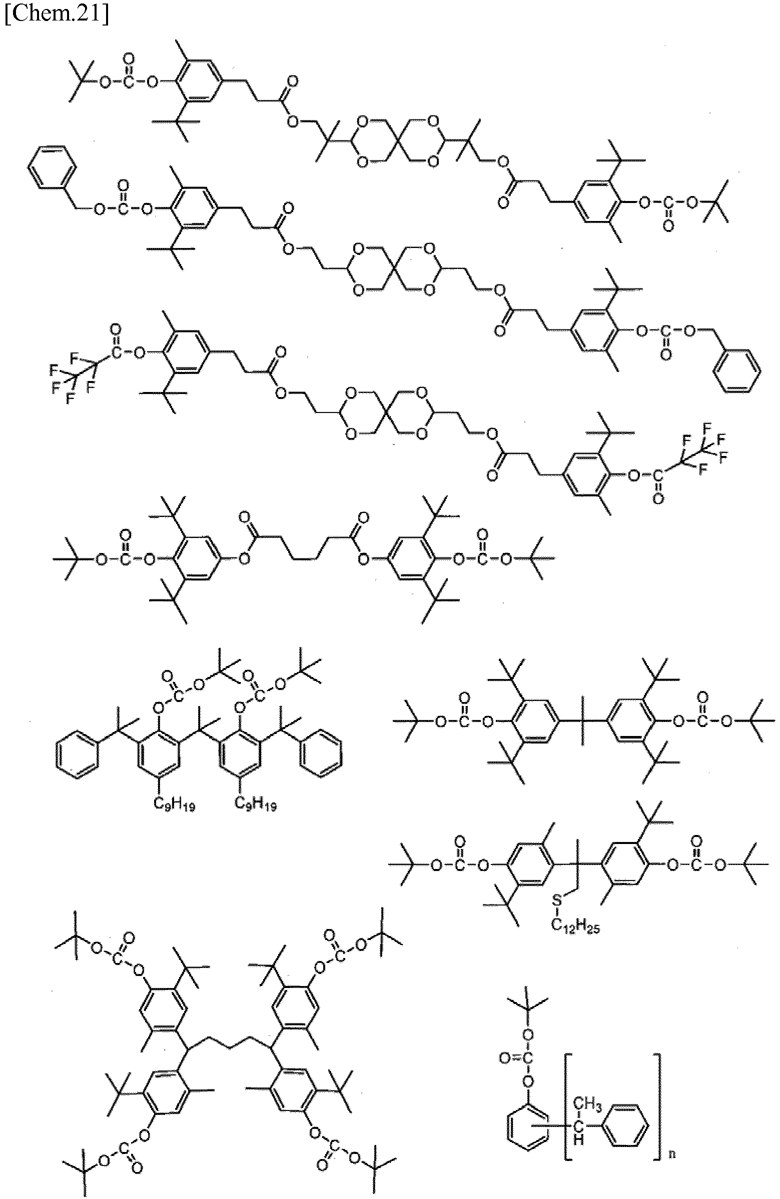

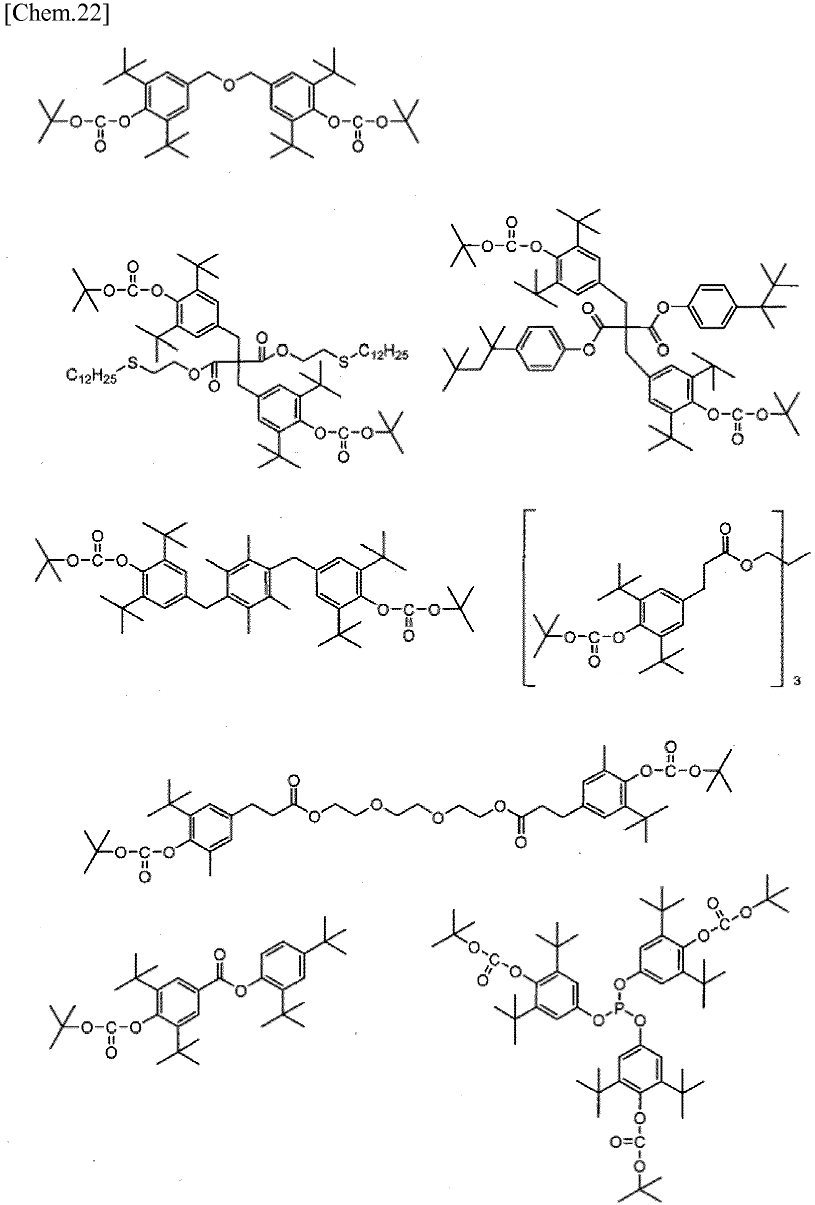

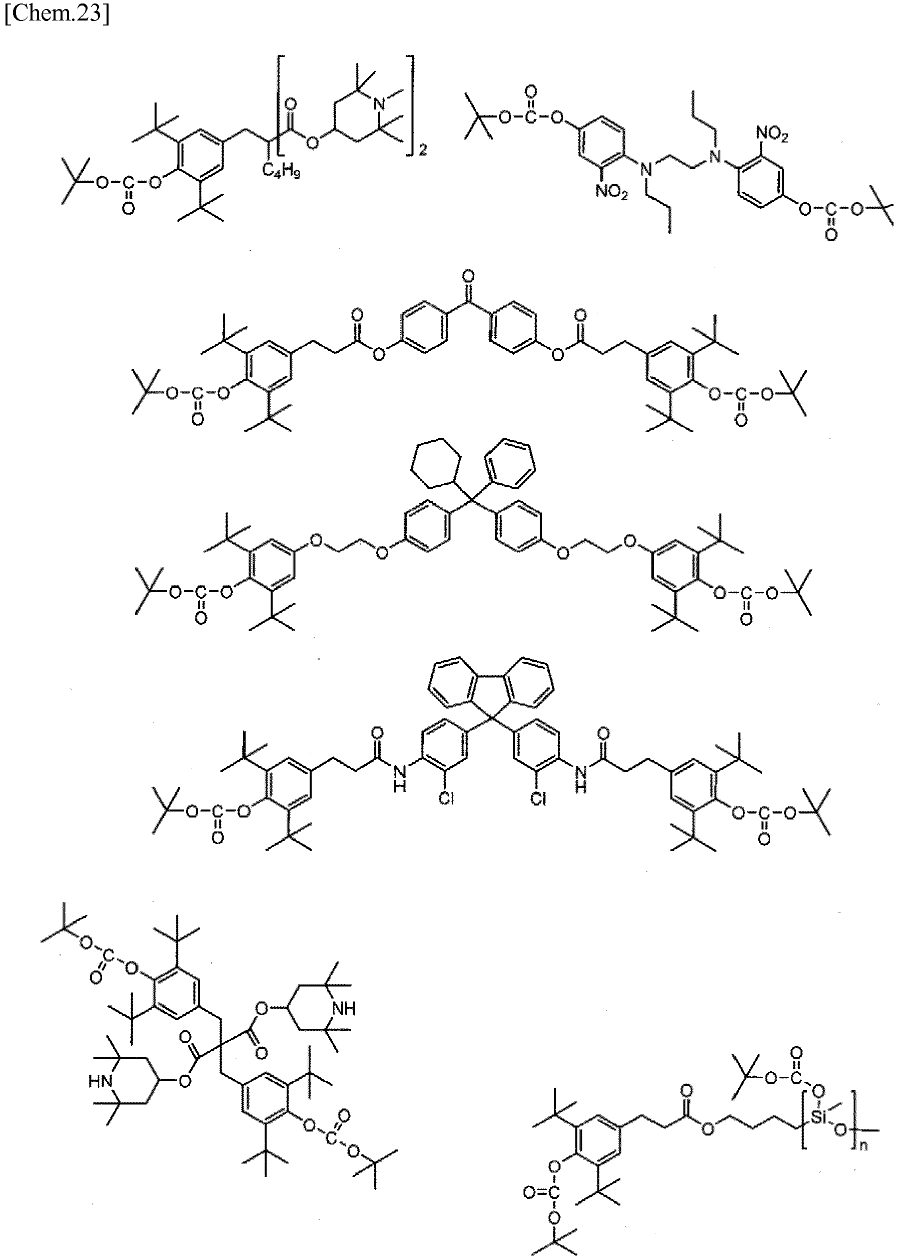

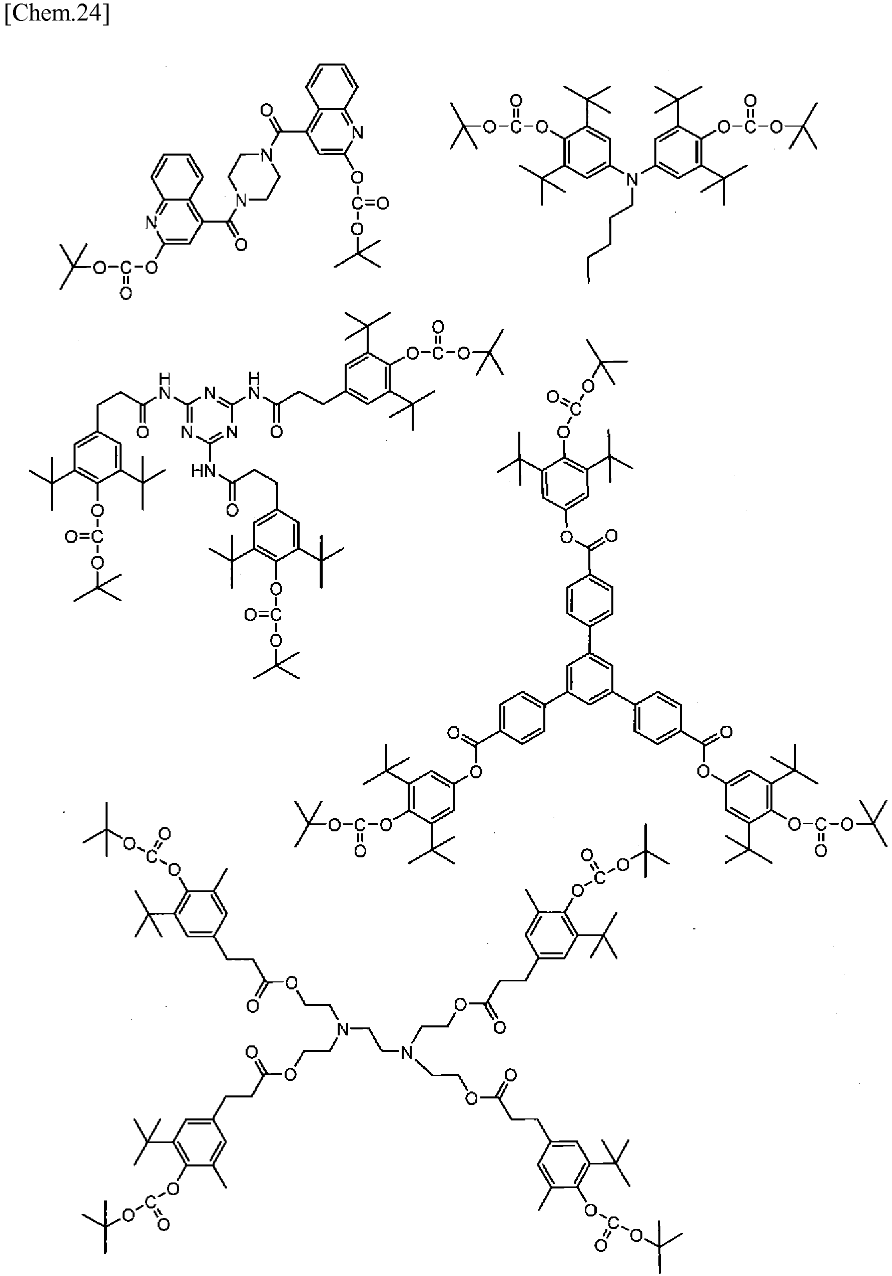

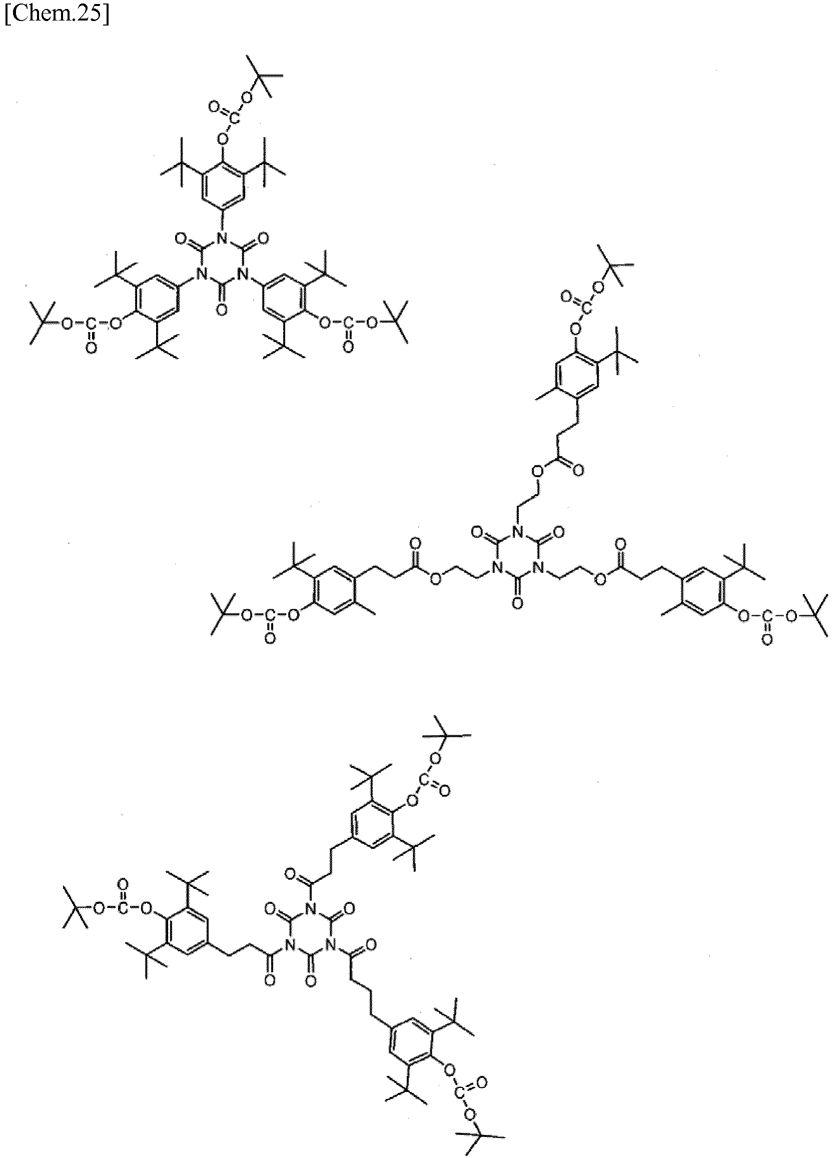

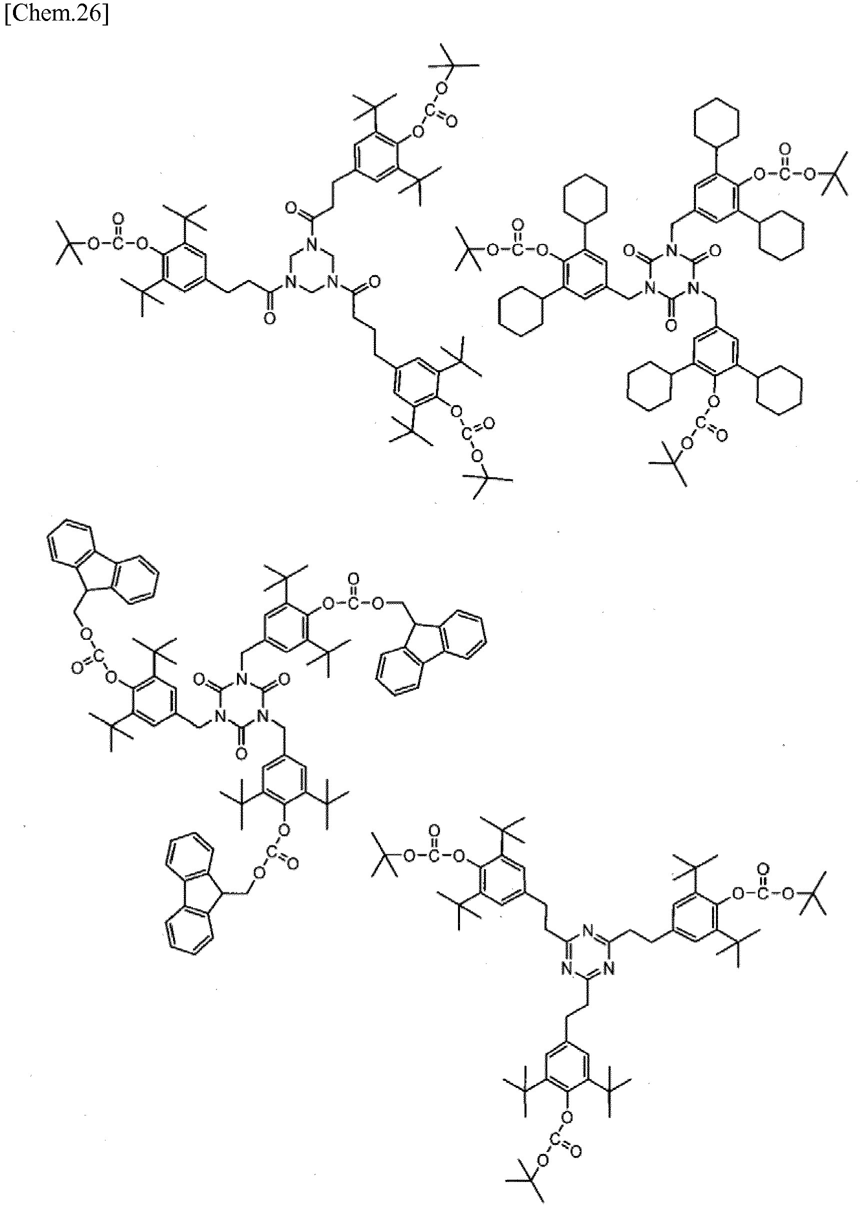

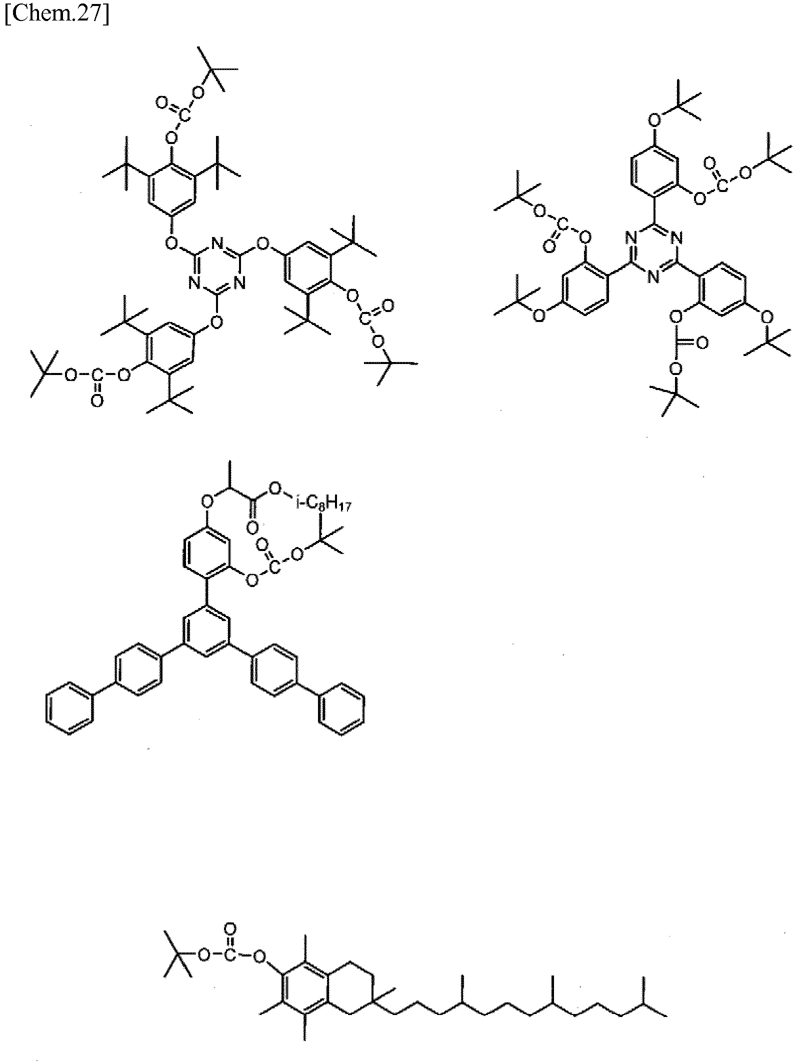

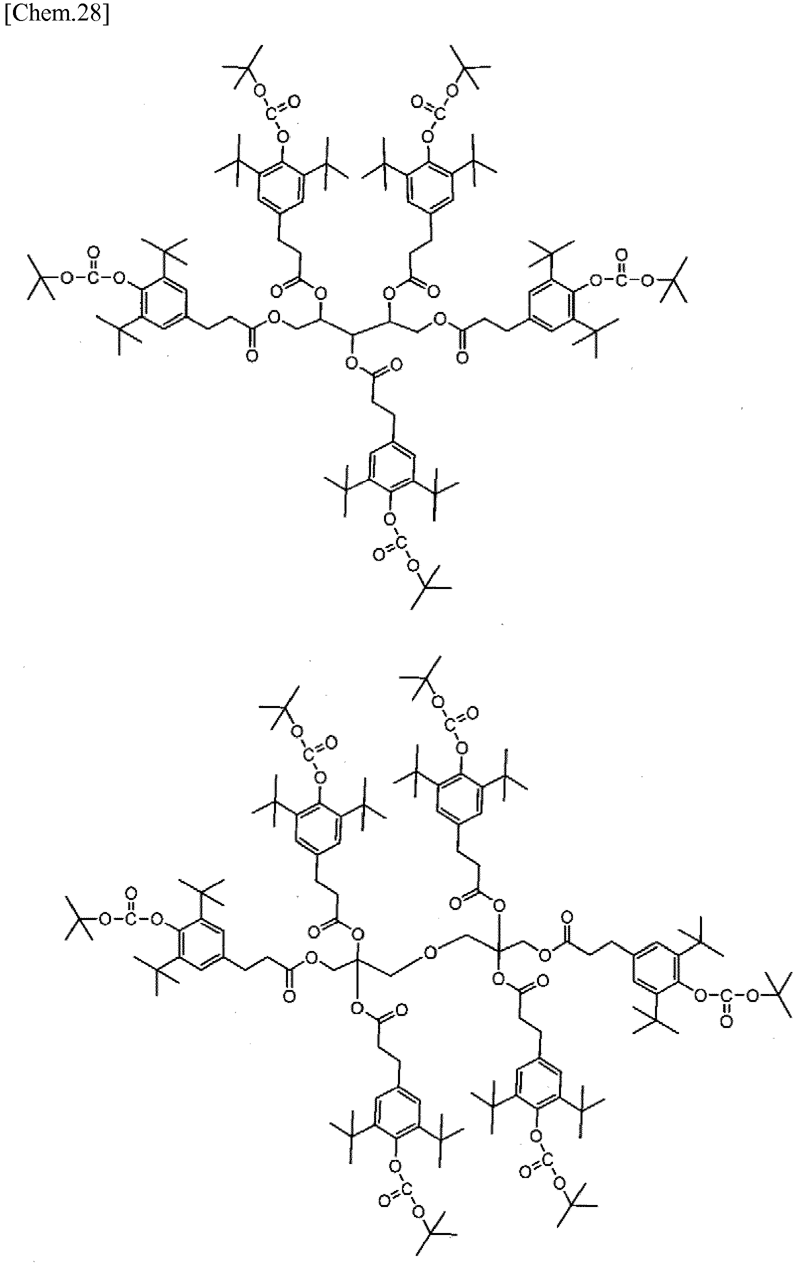

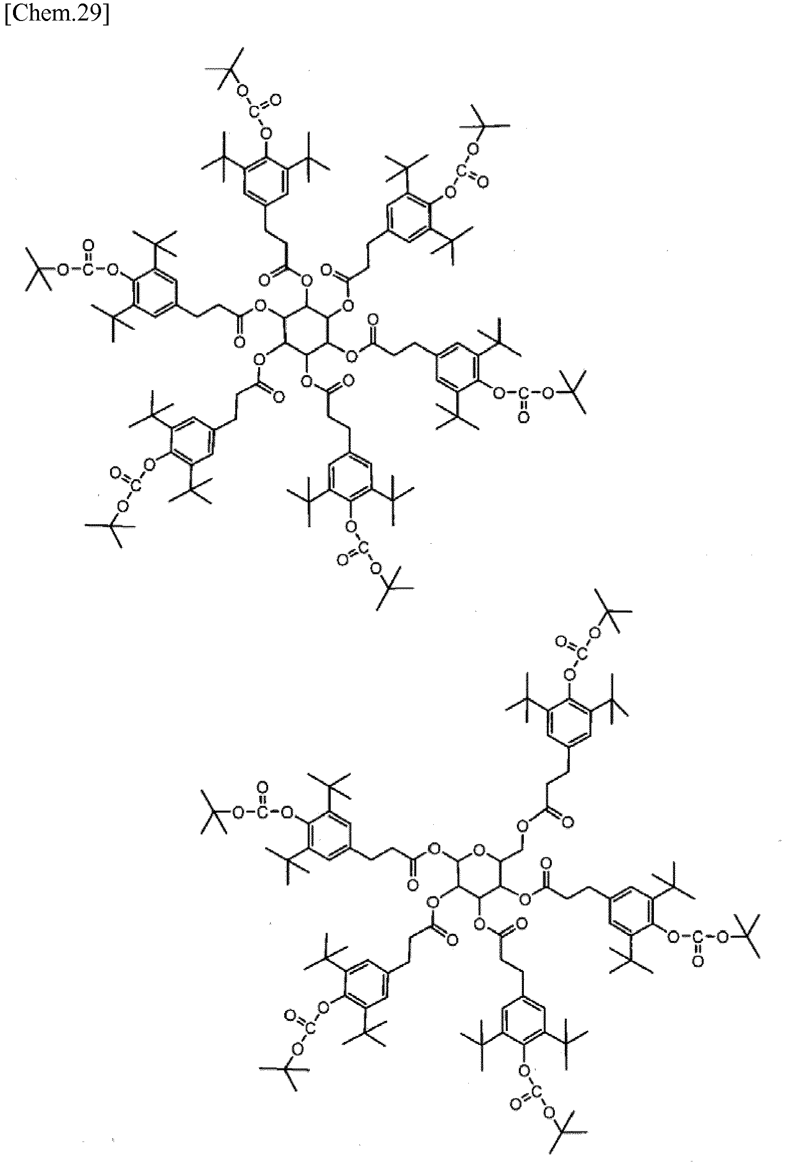

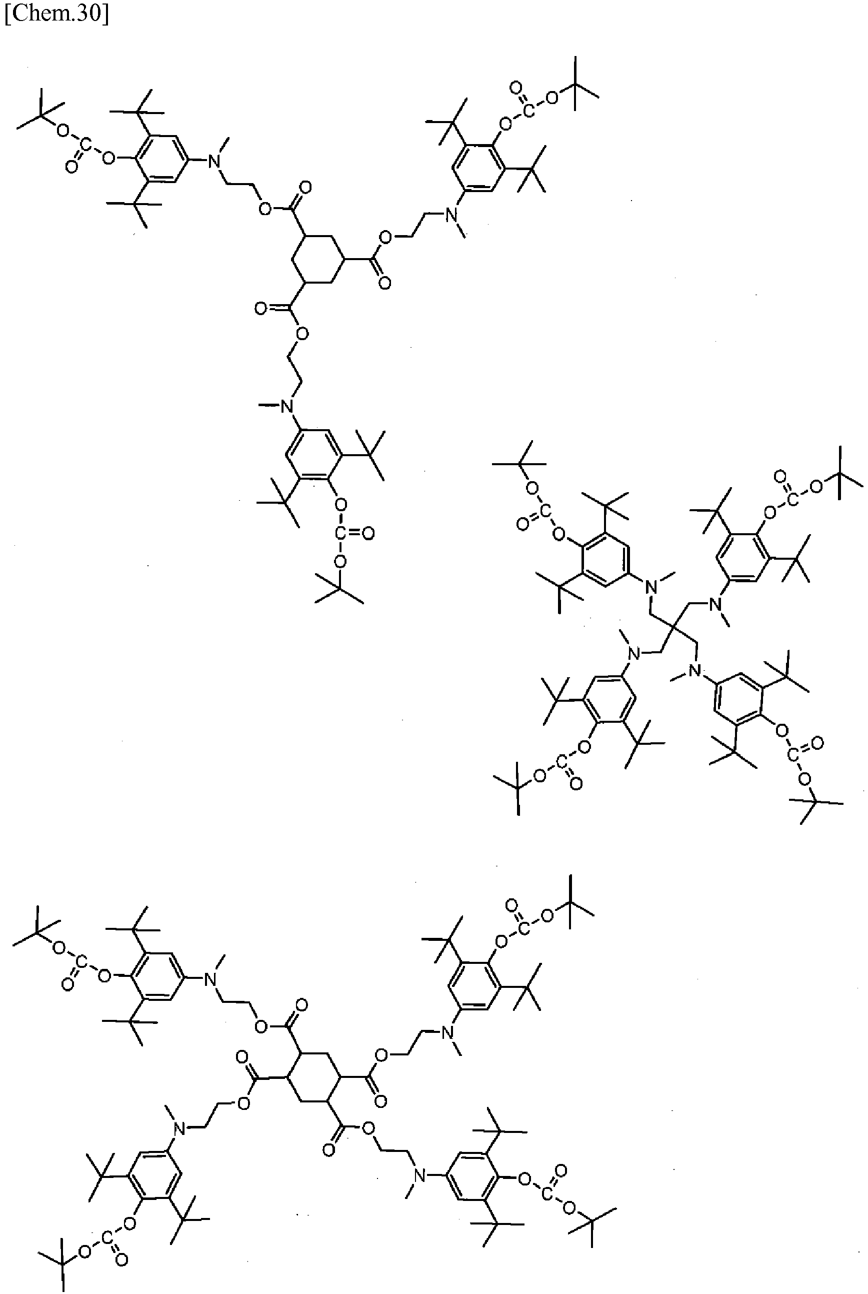

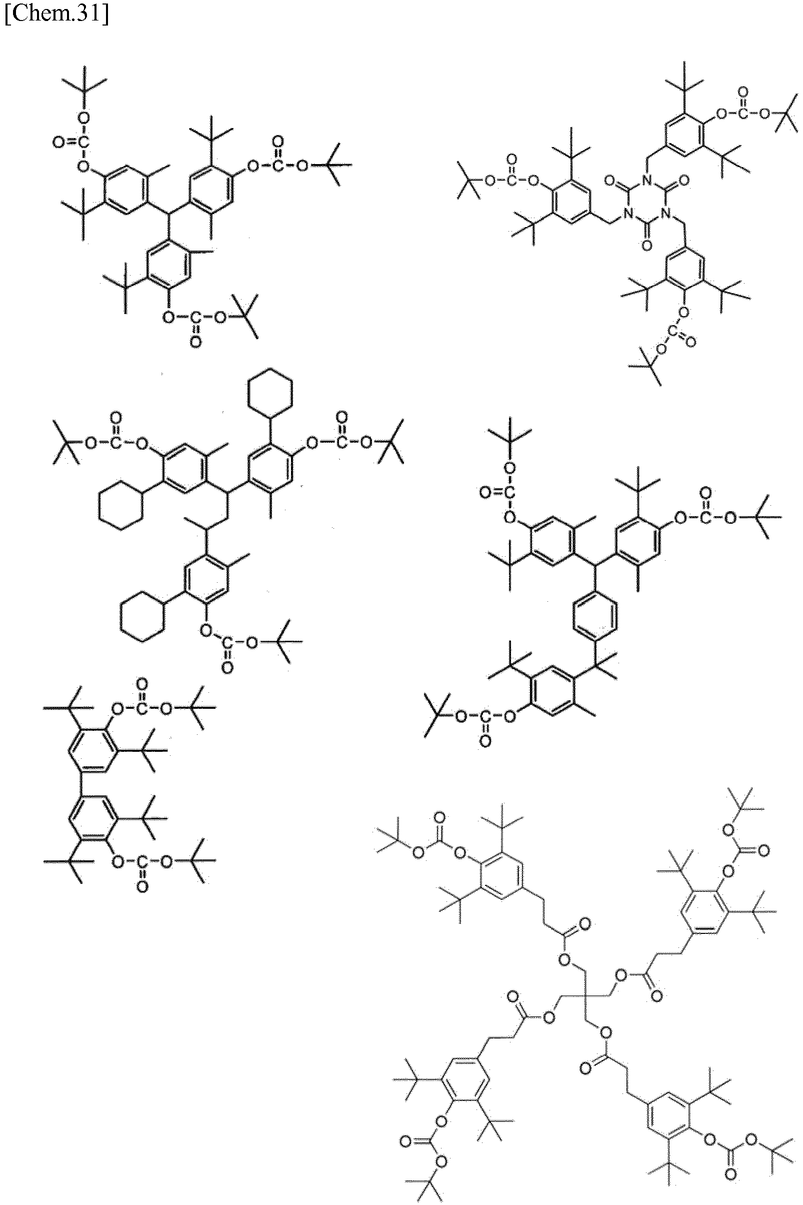

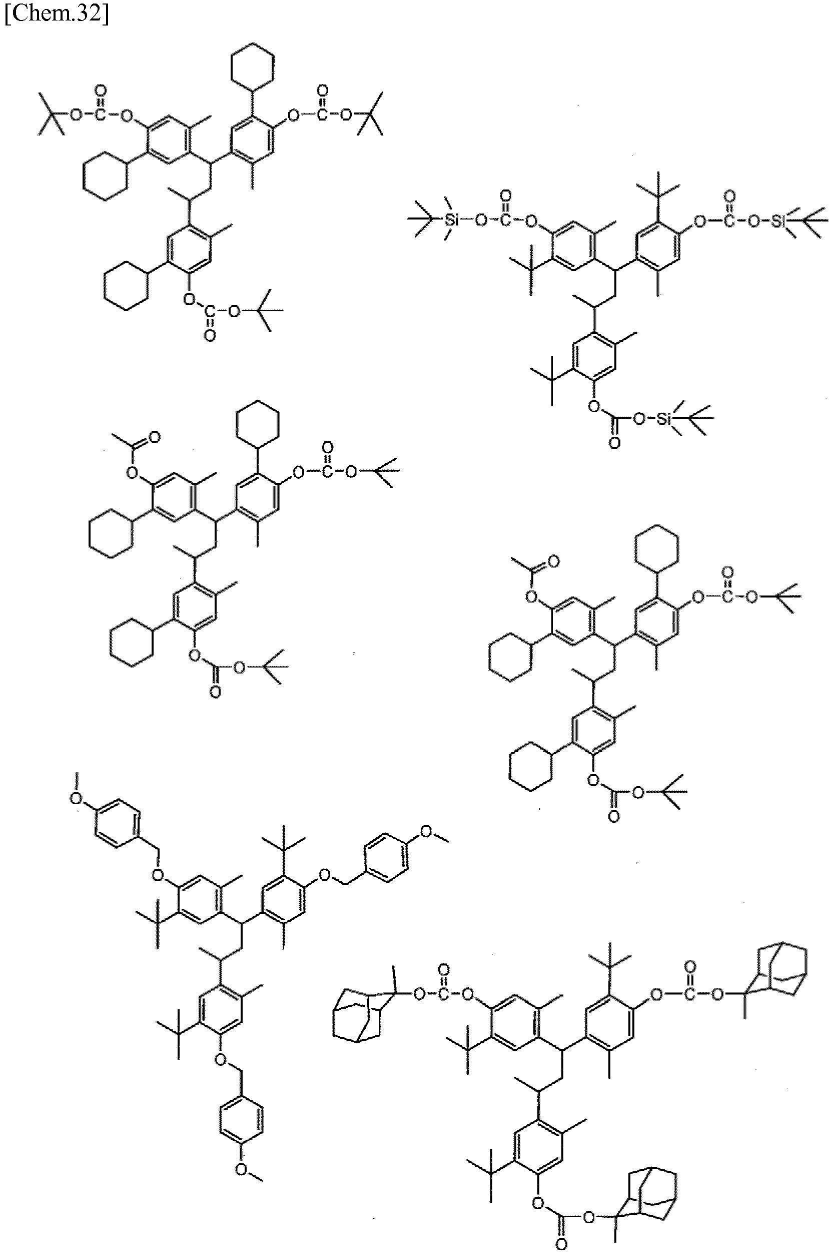

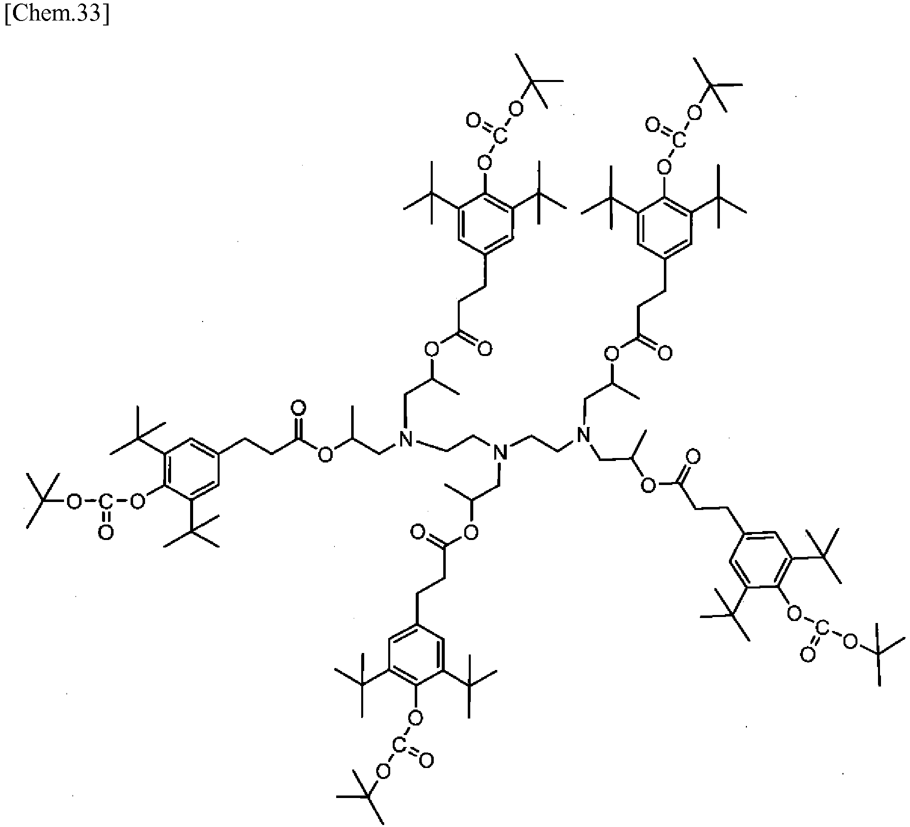

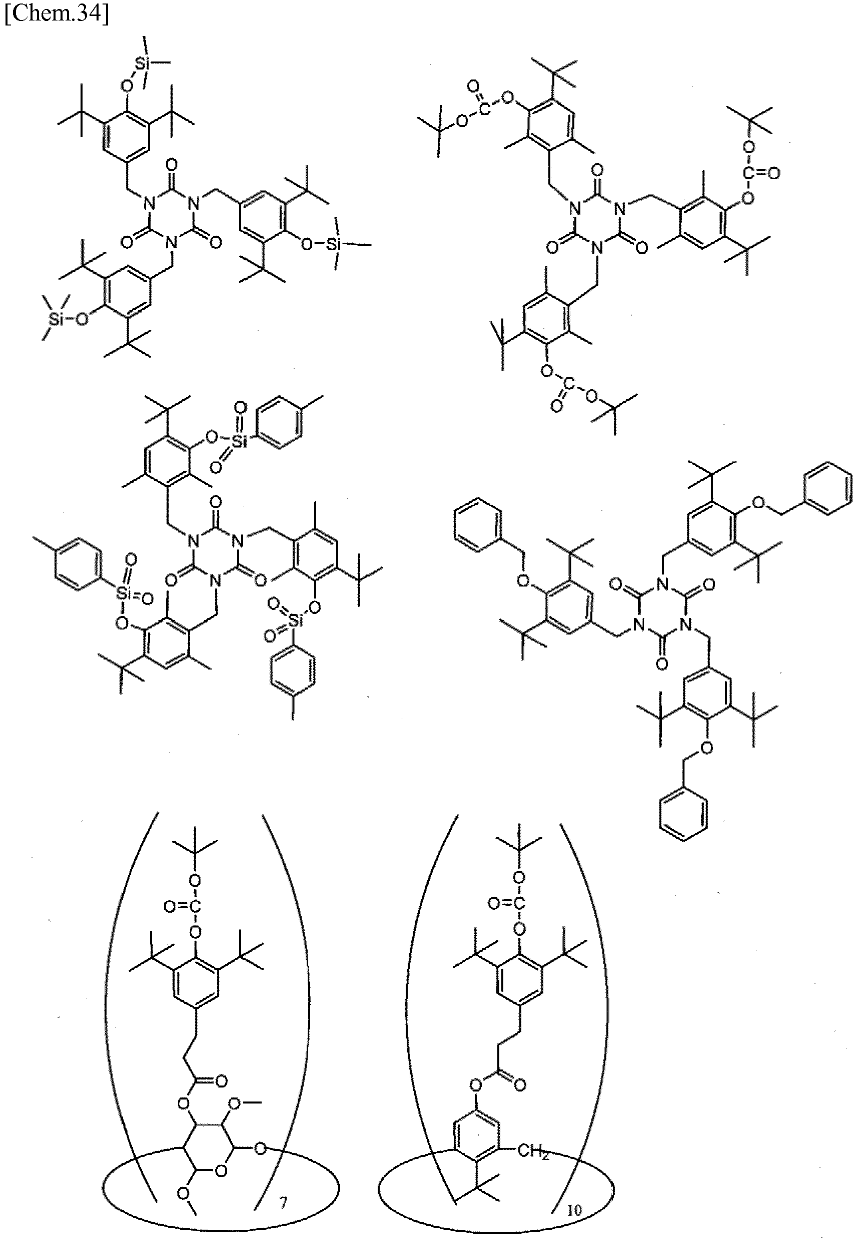

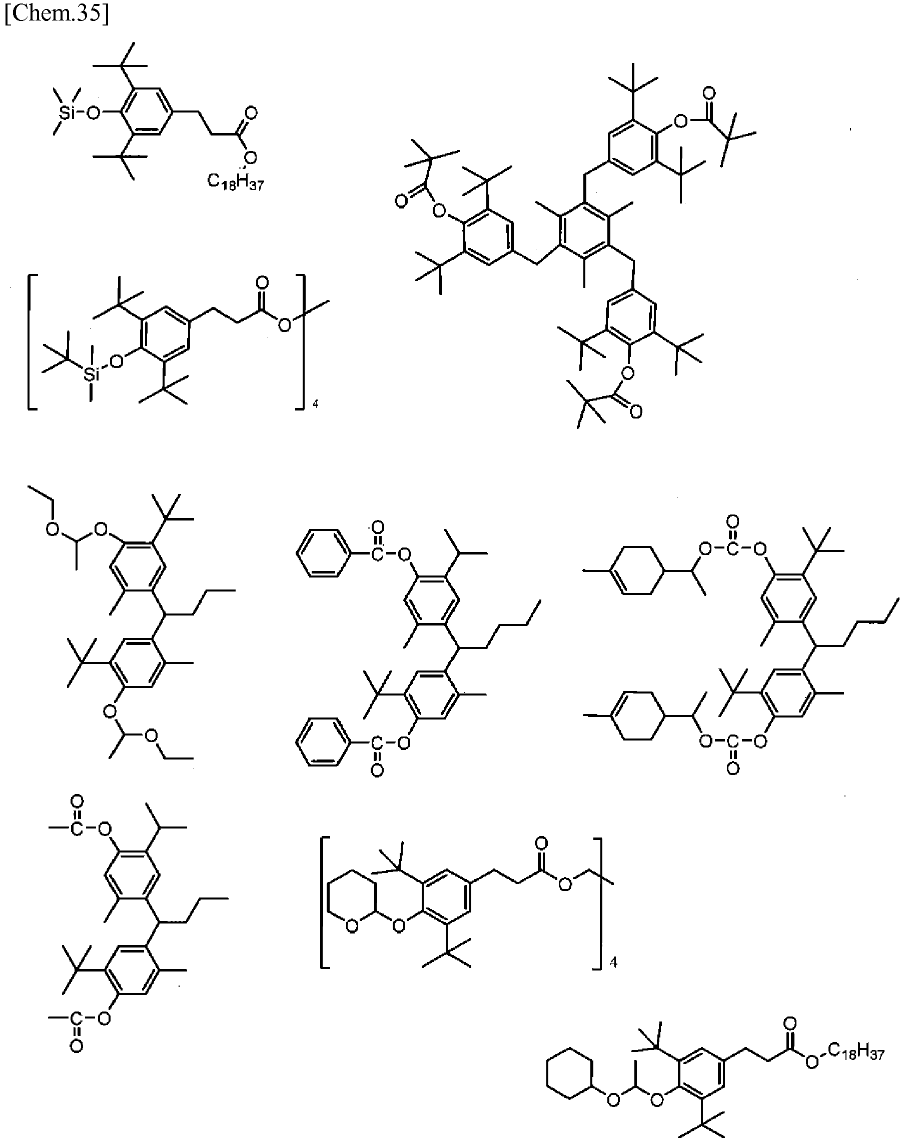

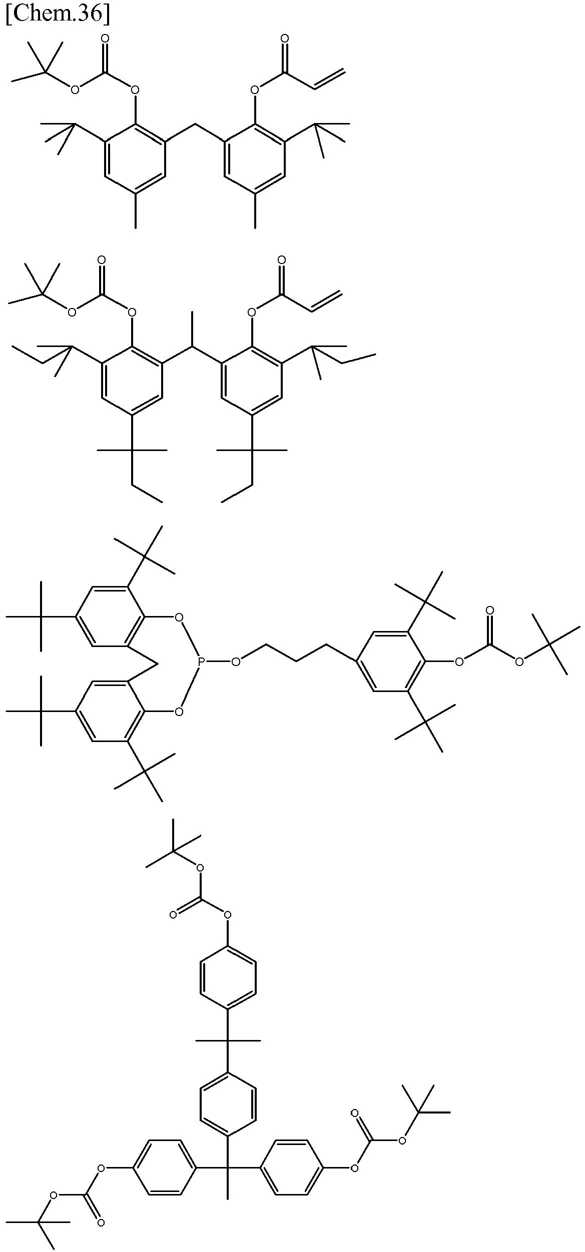

In the compounds represented by General Formula (2) above,
the ring A² is preferably benzene or naphthalene, and
R¹¹ and/or R¹⁵ is preferably an alkyl group having 3 to 20 carbon atoms, an aryl group having 6 to 30 carbon atoms, an arylalkyl group having 7 to 30 carbon atoms, or a heterocycle-containing group having 2 to 20 carbon atoms, and particularly preferably a branched alkyl group having 3 to 8 carbon atoms (e.g., iso-propyl, sec-butyl, tert-butyl, iso-butyl, 1-methylbutyl, 2-methylbutyl, 3-methylbutyl, 1-ethylpropyl, 1,1-dimethylpropyl, 1,2-dimethylpropyl, or 2,2-dimethylpropyl), from the viewpoint of thermal resistance.

Also, a structure in which R¹³ is an alkyl group having 6 to 20 carbon atoms is preferable, and a structure in which the methylene chain in the alkyl group is replaced with -CO-O- is particularly preferable.

R² constituted by the following substituent linked via -CO-O- provides a great oxidation-preventing effect to a thermoplastic resin and thus is preferable.
- Alkyl group having 1 to 8 carbon atoms, particularly branched alkyl group having 3 to 8 carbon atoms (e.g., iso-propyl, sec-butyl, tert-butyl, iso-butyl, 1-methylbutyl, 2-methylbutyl, 3-methylbutyl, 1-ethylpropyl, 1,1-dimethylpropyl, 1,2-dimethylpropyl, or 2,2-dimethylpropyl)
- Arylalkyl group having 1 to 10 carbon atoms (particularly benzyl group)
- Alkenyl group having 2 to 6 carbon atoms (particularly propen-1-yl group)

In the compounds represented by General Formula (3) above,
the ring A² is preferably benzene or naphthalene, and
R¹⁹ is preferably an alkyl group having 3 to 20 carbon atoms, an aryl group having 6 to 30 carbon atoms, an arylalkyl group having 7 to 30 carbon atoms, or a heterocycle-containing group having 2 to 20 carbon atoms from the viewpoint of thermal resistance.

R² constituted by the following substituent linked via -CO-O- provides a great oxidation-preventing effect to a thermoplastic resin and thus is preferable.
- Alkyl group having 1 to 8 carbon atoms, particularly branched alkyl group having 3 to 8 carbon atoms (e.g., iso-propyl, sec-butyl, tert-butyl, iso-butyl, 1-methylbutyl, 2-methylbutyl, 3-methylbutyl, 1-ethylpropyl, 1,1-dimethylpropyl, 1,2-dimethylpropyl, or 2,2-dimethylpropyl)
- Arylalkyl group having 1 to 10 carbon atoms (particularly benzyl group)
- Alkenyl group having 2 to 6 carbon atoms (particularly propen-1-yl group)

In General Formula (3-1) above, which represents X¹¹, X¹ is preferably a sulfur atom, an aromatic ring-containing hydrocarbon group having 6 to 25 carbon atoms, or a heterocycle-containing group having 2 to 21 carbon atoms, and

Z¹ and Z² are preferably a direct bond, -CO-O-, -O-CO-, an optionally substituted aliphatic hydrocarbon group having 1 to 20 carbon atoms, or an aromatic hydrocarbon group having 6 to 10 carbon atoms, and particularly preferably an optionally substituted aliphatic hydrocarbon group having 1 to 8 carbon atoms.

In the compounds represented by General Formula (4) above,
the ring A² is preferably benzene or naphthalene, and
R²⁵, R²⁶, R²⁷, and R²⁸ are preferably an alkyl group having 1 to 20 carbon atoms, an aryl group having 6 to 30 carbon atoms, an arylalkyl group having 7 to 30 carbon atoms, or a heterocycle-containing group having 2 to 20 carbon atoms, and particularly preferably an alkyl group having 1 to 20 carbon atoms, an aryl group having 6 to 10 carbon atoms, an arylalkyl group having 7 to 12 carbon atoms, or a heterocyclic group having 1 to 10 carbon atoms. It is particularly preferable that any one of R²⁵ to R²⁸ is an alkyl group having 1 to 6 carbon atoms (particularly methyl, ethyl, propyl, iso-propyl, butyl, sec-butyl, tert-butyl, iso-butyl, amyl, 1-methylbutyl, 2-methylbutyl, 3-methylbutyl, 1-ethylpropyl, 1,1-dimethylpropyl, 1,2-dimethylpropyl, 2,2-dimethylpropyl, cyclopentyl, or cyclohexyl) or a heterocyclic group having 1 to 10 carbon atoms.

R² in General Formula (4) above constituted by the following substituent linked via -CO-O- provides a great oxidation-preventing effect to a thermoplastic resin and thus is preferable.
- Alkyl group having 1 to 8 carbon atoms (particularly methyl, ethyl, propyl, iso-propyl, butyl, sec-butyl, tert-butyl, iso-butyl, amyl, 1-methylbutyl, 2-methylbutyl, 3-methylbutyl, 1-ethylpropyl, 1,1-dimethylpropyl, 1,2-dimethylpropyl, or 2,2-dimethylpropyl)
- Arylalkyl group having 1 to 10 carbon atoms (particularly benzyl group)
- Alkenyl group having 2 to 6 carbon atoms (particularly propen-1-yl group)

When X³ in General Formula (4) above is represented by General Formula (3-1) above, that is, when X³ in General Formula (4) above has a structure represented by the formula (4-a1) below, structures represented by General Formulae (4-a2) and (4-a3) below show preferred examples of sites at which the ring A² is substituted with R²⁵, R²⁶, R²⁷, and R²⁸. 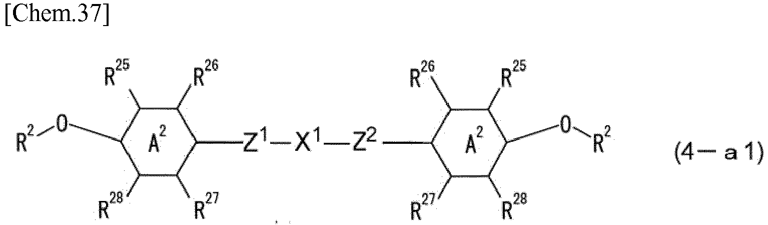
(In the formula, R²⁵, R²⁶, R²⁷, and R²⁸ are the same as those in General Formula (4) above,
R² and the ring A² are the same as those in General Formula (2) above, and
Z¹, Z², and X¹ are the same as those in General Formula (3-1) above.)

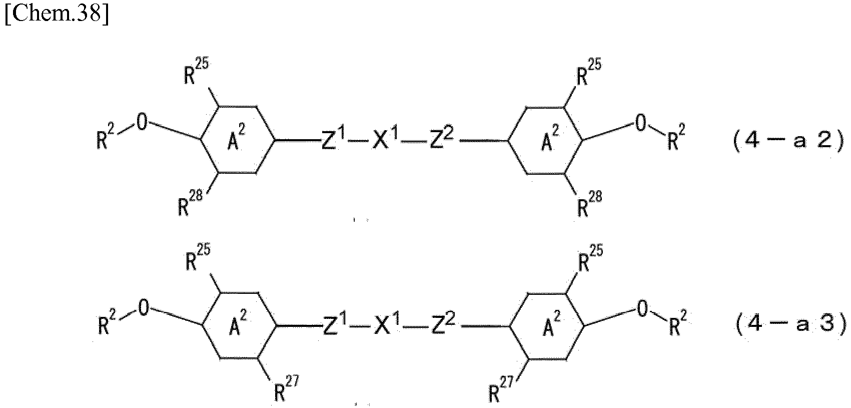

(In the formulae, R²⁵, R²⁷, R²⁸, R², the ring A², Z¹, Z², and X¹ are the same as those in General Formula (IV-1) above.)

X¹ in General Formula (3-1) above is preferably a sulfur atom, an alkyl group having 1 to 20 carbon atoms, an aromatic ring-containing hydrocarbon group having 6 to 25 carbon atoms, a heterocycle-containing group having 2 to 21 carbon atoms, or 2,4,8,10-tetraoxaspiro[5,5]undecane, and particularly preferably an alkyl group having 1 to 15 carbon atoms, an aromatic ring-containing hydrocarbon group having 6 to 15 carbon atoms, or 2,4,8,10-tetraoxaspiro[5,5]undecane.

More specific examples of X¹ shown as a structure containing Z¹ and Z² are as shown in the following #1 to #20 in Group 1.

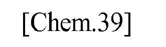

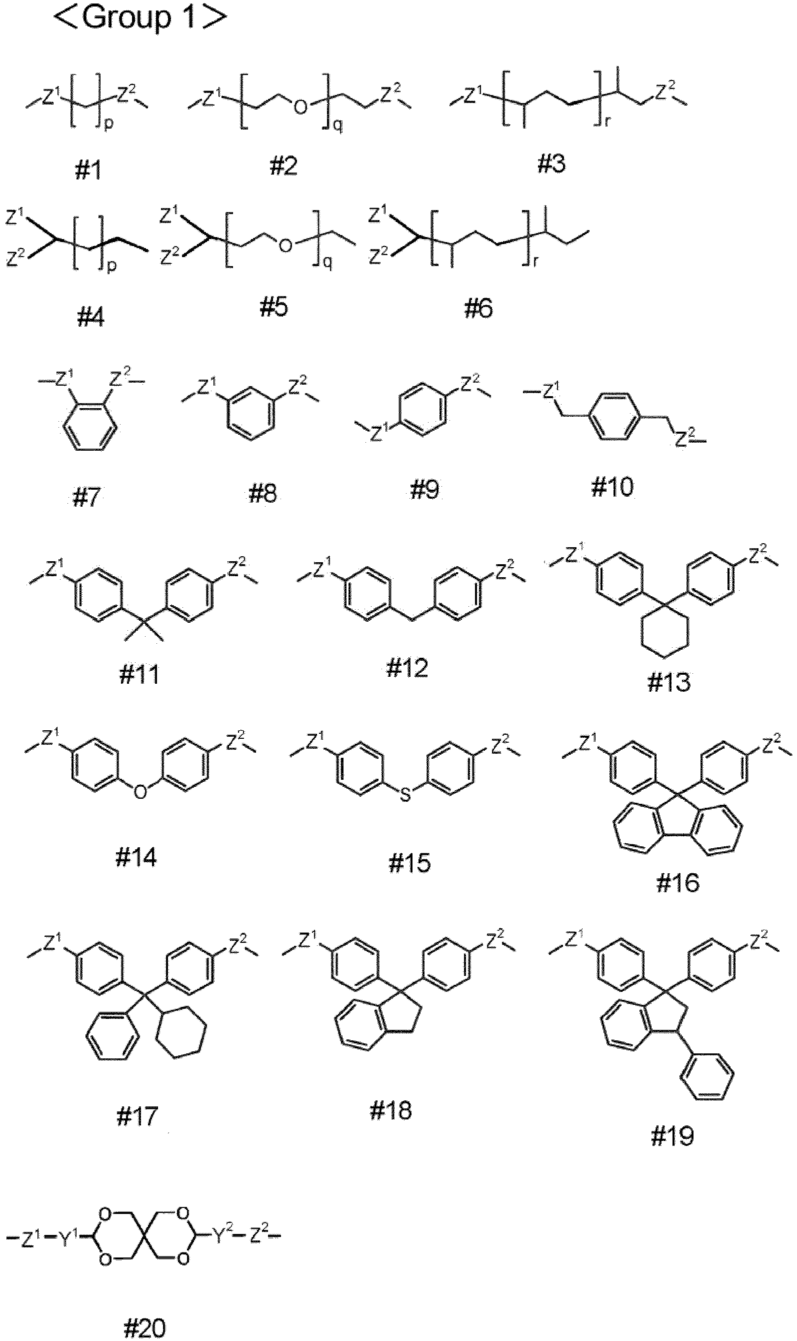

(In the formulae, p represents an integer from 1 to 19, q represents an integer from 1 to 3, r represents an integer from 0 to 3, Y¹ and Y² are the same as those in General Formula (3-5) above, and Z¹ and Z² are the same as those in General Formula (3-1) above.)

Z¹ and Z² in General Formula (3-1) above are preferably a direct bond, -CO-O-, -O-CO-, an optionally substituted aliphatic hydrocarbon group having 1 to 20 carbon atoms, or an aromatic hydrocarbon group having 6 to 10 carbon atoms, and particularly preferably an optionally substituted aliphatic hydrocarbon group having 1 to 8 carbon atoms.

When X³ in General Formula (4) above is represented by General Formula (4-1) above, that is, when X³ in General Formula (4) above has a structure represented by the formula (4-b1) below, structures represented by General Formulae (4-b2) and (4-b3) below show preferred examples of sites at which the ring A² is substituted with R²⁵, R²⁶, R²⁷, and R²⁸. 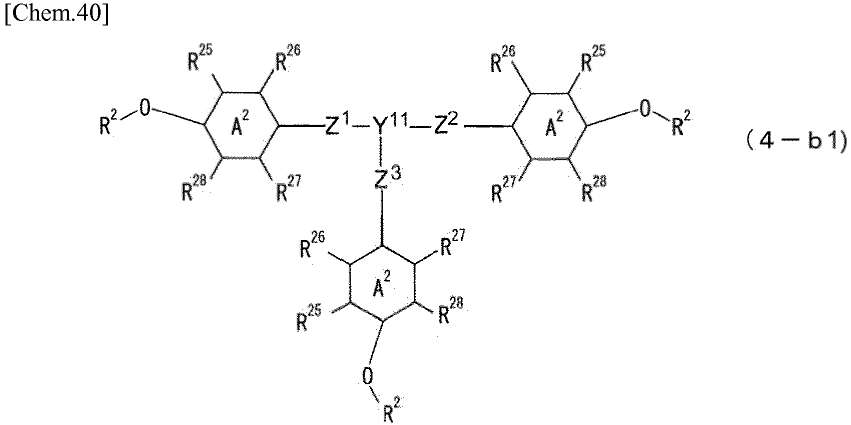
(In the formula, R²⁵, R²⁶, R²⁷, and R²⁸ are the same as those in General Formula (4) above,
R² and the ring A² are the same as those in General Formula (2) above, and
Z¹, Z², Z³, and Y¹¹ are the same as those in General Formula (4-1) above.)

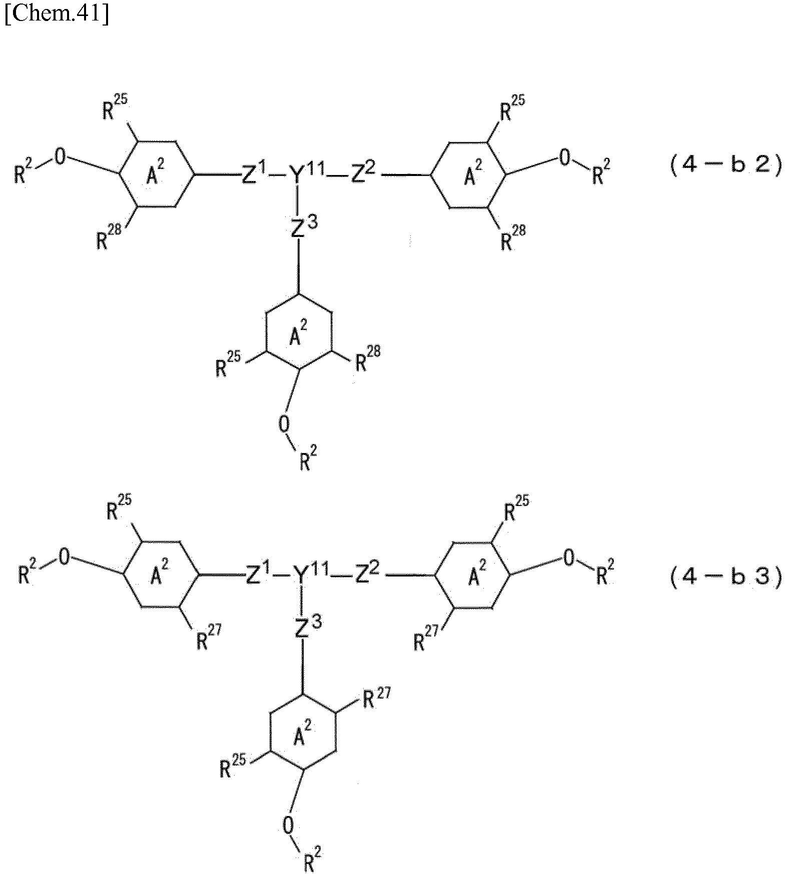

(In the formulae, R²⁵, R²⁷, R²⁸, R², the ring A², Z¹, Z², Z³, and Y¹¹ are the same as those in General Formula (4-a1) above.)

Y¹¹ in General Formula (4-1) above is preferably an alkyl group having 1 to 20 carbon atoms, an aryl group having 6 to 10 carbon atoms, an arylalkyl group having 7 to 12 carbon atoms, or a heterocyclic group having 1 to 10 carbon atoms, and particularly preferably an alkyl group having 1 to 8 carbon atoms, an aryl group having 6 to 9 carbon atoms, or a heterocyclic group having 1 to 6 carbon atoms.

More specific examples of Y¹¹ in General Formula (4-1) above shown as a structure containing Z¹, Z², and Z³ are as shown in the following #21 to #35 in Group 2.

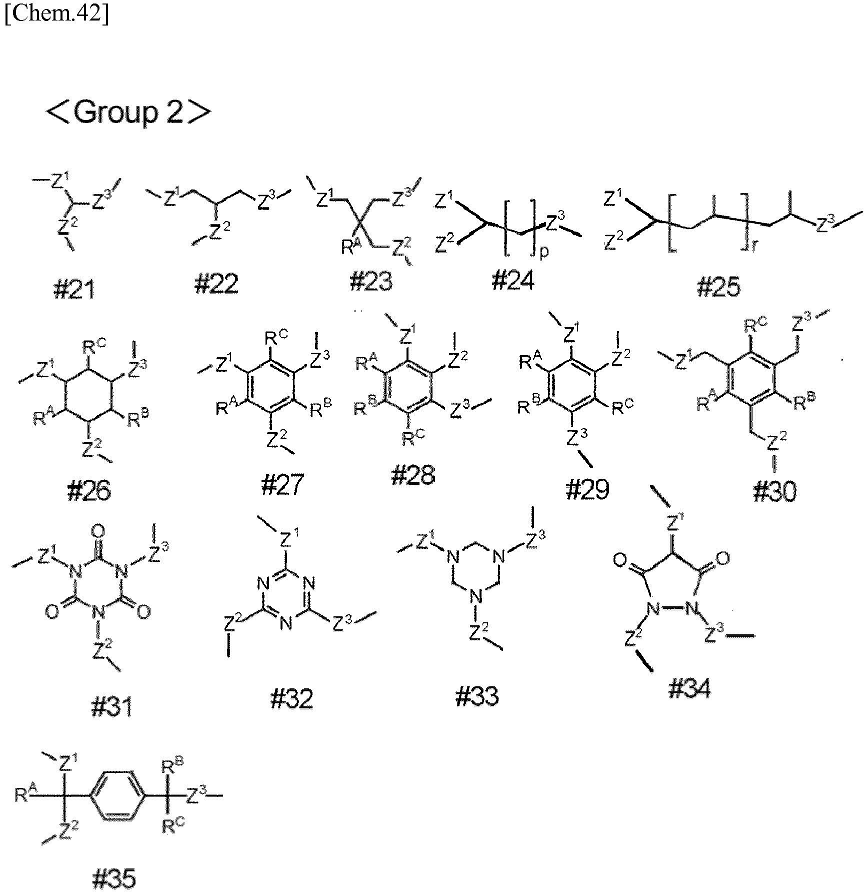

(In the formulae, Z¹, Z², and Z³ are the same as those in General Formula (4-1) above, p represents an integer from 1 to 19, r represents an integer from 0 to 3, R^{A}, R^{B}, and R^{C} represent a hydrogen atom, an aliphatic hydrocarbon group having 1 to 20 carbon atoms, an aromatic ring-containing hydrocarbon group having 6 to 25 carbon atoms, or a heterocycle-containing group having 2 to 21 carbon atoms (preferably an aliphatic hydrocarbon group having 1 to 8 carbon atoms, and particularly preferably a methyl, an ethyl, a propyl, an iso-propyl, a butyl, a sec-butyl, a tert-butyl, an iso-butyl, an amyl, a 1-methylbutyl, a 2-methylbutyl, a 3-methylbutyl, a 1-ethylpropyl, a 1,1-dimethylpropyl, a 1,2-dimethylpropyl, a 2,2-dimethylpropyl, a cyclopentyl, or a cyclohexyl), and R^{A}, R^{B}, and R^{C} may be the same or different.)

Z¹, Z², and Z³ in General Formula (4-1) above are preferably the same as Z¹ and Z².

When X³ in General Formula (4) above is represented by General Formula (4-2) above, that is, when X³ in General Formula (4) above has a structure represented by the formula (4-c1) below, structures represented by General Formulae (4-c2) and (4-c3) below show preferred examples of sites at which the ring A² is substituted with R²⁵, R²⁶, R²⁷ and R²⁸. 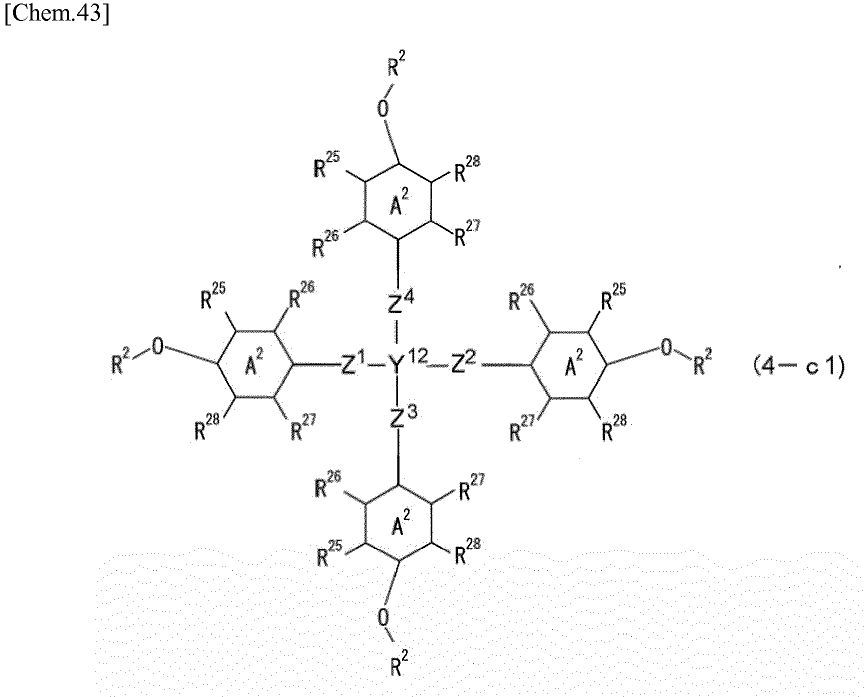
(In the formula, R²⁵, R²⁶, R²⁷, and R²⁸ are the same as those in General Formula (4) above,
R² and the ring A² are the same as those in General Formula (2) above, and
Z¹, Z², Z³, Z⁴, and Y¹² are the same as those in General Formula (4-2) above.) 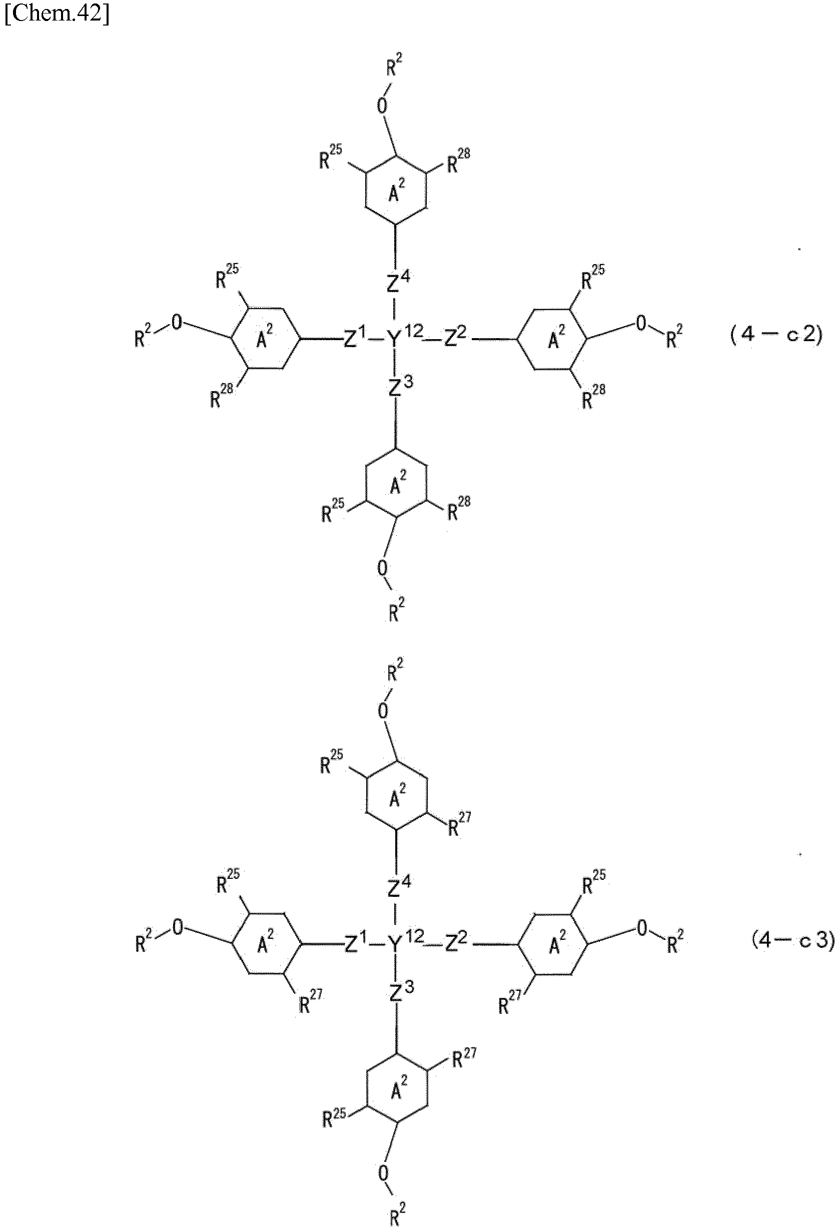
(In the formulae, R²⁵, R²⁷, R²⁸, R², the ring A², Z¹, Z², Z³, Z⁴, and Y¹² are the same as those in General Formula (4-c1) above.)

Y¹² in General Formula (4-2) above is preferably the same as Y¹¹.

More specific examples of Y¹² shown as a structure containing Z¹, Z², Z³, and Z⁴ are as shown in the following #36 to #40 in Group 3.

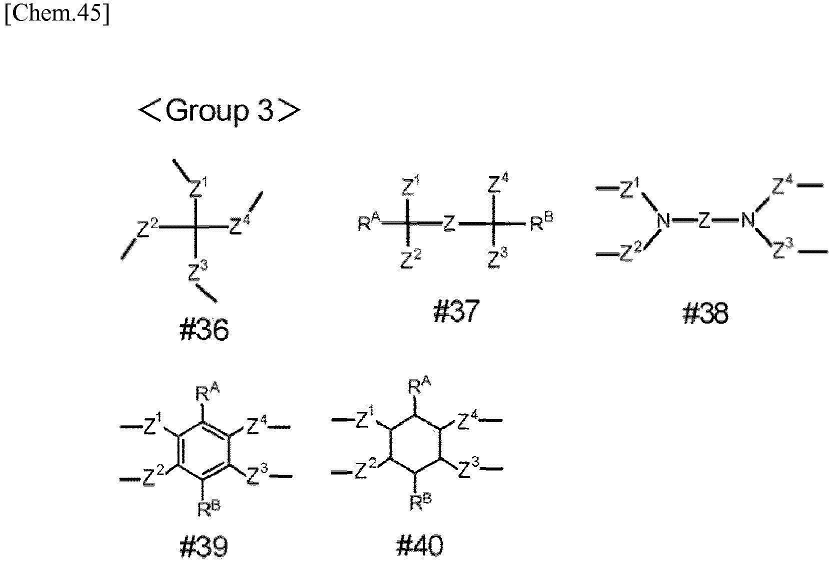

(In the formulae, Z¹, Z², Z³, and Z⁴ are the same as those in General Formula (4-2) above, R^{A} and R^{B} represent a hydrogen atom, an aliphatic hydrocarbon group having 1 to 20 carbon atoms, an aromatic ring-containing hydrocarbon group having 6 to 25 carbon atoms, or a heterocycle-containing group having 2 to 21 carbon atoms (preferably a hydrogen atom or an aliphatic hydrocarbon group having 1 to 8 carbon atoms, and particularly preferably a hydrogen atom, a methyl, an ethyl, a propyl, an iso-propyl, a butyl, a sec-butyl, a tert-butyl, an iso-butyl, an amyl, a 1-methylbutyl, a 2-methylbutyl, a 3-methylbutyl, a 1-ethylpropyl, a 1,1-dimethylpropyl, a 1,2-dimethylpropyl, a 2,2-dimethylpropyl, a cyclopentyl, or a cyclohexyl), and R^{A}, R^{B}, and R^{C} may be the same or different.)

Z¹ to Z⁴ in General Formula (4-2) above are preferably the same as Z¹ and Z².

When X³ in General Formula (4) above has a structure represented by General Formula (4-3) above, Y¹³ is preferably the same as Y¹¹, and
Z¹ to Z⁵ are preferably the same as Z¹ and Z², and
when X³ has a structure represented by General Formula (4-4) above, Y¹⁴ is preferably the same as Y¹¹, and
Z¹ to Z⁶ are preferably the same as Z¹ and Z².

In the present invention, compounds having an alkyl carbonate group greatly enhance the effect of the present invention and thus are particularly preferable.

Although there is no particular limitation on a method for manufacturing an antioxidant for a thermoplastic resin represented by General Formula (1) above, the antioxidant can be obtained by reacting a phenol compound, a silyl chloride compound, an allyl ether compound, and the like manufactured by using the methods disclosed in JP S57-11375A, JP H3-173843A, JP H6-128195A, JP H7-206771A, JP H7-252191A, and JP 2004-501128A, for example.

The antioxidant for a thermoplastic resin according to the present invention is useful as a NOx-resistant antioxidant or a SOx-resistant antioxidant.

Next, a thermoplastic resin composition that is stabilized by the antioxidant for a thermoplastic resin according to the present invention will be described. As the thermoplastic resin that can be stabilized by the antioxidant for a thermoplastic resin according to the present invention, any type of resins may be used, and examples thereof include thermoplastic resins such as homopolymers and copolymers (e.g., polypropylene, low-density polyethylene, linear low-density polyethylene, high-density polyethylene, polybutene-1, poly-3-methylpentene, poly-4-methylpentene, and ethylene-propylene copolymer) of α-olefins, copolymers between the α-olefins and a polyunsaturated compound (e.g., a conjugated diene or a non-conjugated diene), acrylic acid, methacrylic acid, vinyl acetate or the like, biodegradable resins (e.g., polyethylene terephthalate, polyethylene terephthalate-isophthalate, polyethylene terephthalate-paraoxybenzoate, and polybutylene terephthalate) including linear polyester, acid-modified polyester, aliphatic polyester and the like, liquid crystal polyester, polyamides (e.g., polycaprolactam and polyhexamethylene adipamide), liquid crystal polyamide, polyimides, polystyrene, copolymers (e.g., acrylonitrile styrene copolymer (AS) resin, acrylonitrile-butadiene-styrene copolymer (ABS) resin, methyl methacrylate-butadiene-styrene copolymer (MBS) resin, and thermal resistant ABS resin) between styrene and/or α-methylstyrene and another monomer (e.g., maleic anhydride, phenylmaleimide, methyl methacrylate, butadiene, and acrylonitrile), halogen-containing resins (e.g., polyvinyl chloride, polyvinylidene chloride, chlorinated polyethylene, chlorinated polypropylene, polyvinylidene fluoride, chlorinated rubber, a vinyl chloride-vinyl acetate copolymer, a vinyl chloride-ethylene copolymer, a vinyl chloride-vinylidene chloride copolymer, vinyl chloride-vinylidene chloride-vinyl acetate terpolymer, a vinyl chloride-acrylic ester copolymer, a vinyl chloride-maleic ester copolymer, and vinyl chloride-cyclohexylmaleimide copolymer), polymer of (meth)acrylic ester (e.g., methyl (meth)acrylate, ethyl (meth)acrylate, and octyl (meth)acrylate), polyether ketone, polyvinyl acetate, polyvinyl formal, polyvinyl butyral, polyvinyl alcohol, linear or branched polycarbonates, a petroleum resin, a coumarone resin, polyphenylene oxide, polyphenylene sulfide, thermoplastic polyurethane, and a cellulosic resin; naturally derived resins (e.g., natural rubber, aliphatic polyesters (e.g., 3-hydroxybutyrate) produced by microorganisms, aliphatic polyamides produced by microorganisms, starch, cellulose, chitin-chitosan, and gluten-gelatin), a general-purpose resin, an engineering resin, and a polymer alloy. The polymer alloy as used herein refers to a macromolecular multicomponent system, and may be a block polymer obtained by copolymerization or a polymer blend obtained by mixing.

Furthermore, the thermoplastic resin may also be isoprene rubber, butadiene rubber, butadiene-styrene copolymer rubber, butadiene-acrylonitrile copolymer rubber, acrylonitrile-butadiene-styrene copolymer rubber, copolymer rubber between ethylene and α-olefin such as propylene or butene-1, an elastomer such as a terpolymer rubber between ethylene-α-olefin and a non-conjugated diene such as ethylidene norbornene or cyclopentadiene, an α-olefin elastomer, silicone resin, or the like, or a resin obtained by alloying or blending the resins and/or the elastomers and the rubber.

Although the above-mentioned thermoplastic resins may exhibit the stabilizing effect at different levels depending on the stereoregularity, the specific gravity, the type of polymerization catalyst, whether or not the polymerization catalyst is removed, the degree to which the polymerization catalyst is removed, the degree of crystallization, the polymerization conditions such as temperature and pressure, the type of crystals, the size of crystal lamellae measured by using X-ray small angle scattering, the aspect ratio of crystals, the solubility in an aromatic solvent or an aliphatic solvent, the solution viscosity, the melt viscosity, the average molecular weight, the degree of molecular weight distribution, the number of peaks in the molecular weight distribution, whether a block copolymer or a random copolymer is formed, the blending ratio of monomers, and the like, any thermoplastic resin can be selected and used.

In the present invention, polyolefin-based resins can be preferably used as the above-mentioned thermoplastic resin because the effect of the present invention is greatly enhanced. Examples of the polyolefin-based resins include homopolymers and copolymers of α-olefins, such as polypropylene, low-density polyethylene, linear low-density polyethylene, high-density polyethylene, polybutene-1, poly-3-methylpentene, poly-4-methylpentene, and ethylene-propylene copolymer.

There is no particular limitation on the method for blending the antioxidant for a thermoplastic resin according to the present invention into the above-mentioned thermoplastic resin, and a known technique for blending a resin additives can be used. For example, when the above-mentioned thermoplastic resin is polymerized, a method in which the antioxidant is added to a polymerization system in advance, a method in which the antioxidant is added during the polymerization, or a method in which the antioxidant is added after the polymerization may be used. When the method in which the antioxidant for a thermoplastic resin according to the present invention is blended after the polymerization of the above-mentioned thermoplastic resin is used, examples thereof include a method in which a mixture obtained by using a Henschel mixer to mix the antioxidant and powder or pellets of a thermoplastic resin to be stabilized is kneaded using a processing apparatus such as an extruder, and a method in which a masterbatch of the antioxidant is produced and then blended into the thermoplastic resin. There are also no particular limitations on the type of processing apparatus to be used, the processing temperature, and the cooling condition after processing, and the conditions can be selected such that the physical properties of the obtained resin are suitable for the application. Moreover, the antioxidant for a thermoplastic resin according to the present invention can be blended into the thermoplastic resin after being granulated alone or together with other resin additives.

The antioxidant for a thermoplastic resin according to the present invention is used in an amount in a range from 0.001 to 0.5 parts by mass, and preferably in a range from 0.001 to 0.3 parts by mass, with respect to 100 parts by mass of the above-mentioned thermoplastic resin. When the use amount is less than 0.001 parts by mass, there is a case where a desired oxidation-preventing effect cannot be obtained, and when the use amount is more than 0.5 parts by mass, the antioxidant may bleed out of a molded article obtained by molding the above-mentioned thermoplastic resin composition, resulting in the deterioration of the external appearance of the molded article.

It should be noted that the above-mentioned use amount refers to a use amount in a molded article as an end product. For example, when the use amount of the antioxidant for a thermoplastic resin can be diluted by adding the antioxidant to the thermoplastic resin and then molding the resulting mixture, as in the case where a masterbatch is used, the use amount of the antioxidant may exceed the above-mentioned use amount.

In general, commonly used resin additives can be blended into the thermoplastic resin composition according to the present invention as long as the desired effect of the present invention is not impaired. Examples of the above-mentioned resin additives include phenol-based antioxidants, phosphorus-based antioxidants, thioether-based antioxidants, ultraviolet absorbers, hindered amine-based photostabilizers, nucleating agents, flame retardants, flame retardant assistants, lubricants, fillers, metallic soap, hydrotalcites, antistatic agents, pigments, and dyes.

Examples of the above-mentioned phenol-based antioxidants include 2,6-di-tert-butyl-4-ethylphenol, 2-tert-butyl-4,6-dimethylphenol, styrenated phenol, 2,2'-methylenebis(4-ethyl-6-tert-butylphenol), 2,2'-thiobis-(6-tert-butyl-4-methylphenol), 2,2'-thiodiethylenebis[3-(3,5-di-tert-butyl-4-hydroxyphenyl)propionate], 2-methyl-4,6-bis(octylsulfanylmethyl)phenol, 2,2'-isobutylidenebis(4,6-dimethylphenol), isooctyl-3-(3,5-di-tert-butyl-4-hydroxyphenyl)propionate, N,N'-hexane-1,6-diylbis[3-(3,5-di-tert-butyl-4-hydroxyphenyl)propionamide], 2,2'-oxamide-bis[ethyl-3-(3,5-di-tert-butyl-4-hydroxyphenyl)propionate], 2-ethylhexyl-3-(3',5'-di-tert-butyl-4'-hydroxyphenyl)propionate, 2,2'-ethylenebis(4,6-di-tert-butylphenol), esters between 3,5-bis(1,1-dimethylethyl)-4-hydroxy-benzenepropanoic acid and a C13-15 alkyl, 2,5-di-tert-amylhydroquinone, a hindered phenol polymer (AO.OH998 manufactured by ADEKA PALMAROLE), 2,2'-methylenebis[6-(1-methylcyclohexyl)-p-cresol], 2-tert-butyl-6-(3-tert-butyl-2-hydroxy-5-methylbenzyl)-4-methylphenyl acrylate, 2-[1-(2-hydroxy-3,5-di-tert-pentylphenyl)ethyl]-4,6-di-tert-pentylphenyl acrylate, 6-[3-(3-tert-butyl-4-hydroxy-5-methyl)propoxy]-2,4,8,10-tetra-tert-butylbenzo[d,f][1,3,2] -dioxaphosphepin, hexamethylenebis[3-(3,5-di-tert-butyl-4-hydroxyphenyl)propionate], calcium bis[monoethyl(3,5-di-tert-butyl-4-hydroxybenzyl)phosphonate], a reaction product between 5,7-bis(1,1-dimethylethyl)-3-hydroxy-2(3H)-benzofuranone and o-xylen, 2,6-di-tert-butyl-4-(4,6-bis(octylthio)-1,3,5-triazin-2-ylamino)phenol, DL-a-tocopherol (vitamin E), 2,6-bis(a-methylbenzyl)-4-methylphenol, bis[3,3-bis-(4'-hydroxy-3'-tert-butyl-phenyl)butanoic acid]glycol ester, 2,6-di-tert-butyl-p-cresol, 2,6-diphenyl-4-octadecyloxyphenol, stearyl(3,5-di-tert-butyl-4-hydroxyphenyl)propionate, distearyl(3,5-di-tert-butyl-4-hydroxybenzyl)phosphonate, tridecyl-3,5-tert-butyl-4-hydroxybenzylthioacetate, thiodiethylenebis[(3,5-di-tert-butyl-4-hydroxyphenyl)propionate], 4,4'-thiobis(6-tert-butyl-m-cresol), 2-octylthio-4,6-di(3,5-di-tert-butyl-4-hydroxyphenoxy)-s-triazine, 2,2'-methylenebis(4-methyl-6-tert-butylphenol), bis[3,3-bis(4-hydroxy-3-tert-butylphenyl)butylic acid]glycol ester, 4,4'-butylidenebis(2,6-di-tert-butylphenol), 4,4'-butylidenebis(6-tert-butyl-3-methylphenol), 2,2'-ethylidenebis(4,6-di-tert-butylphenol), 1,1,3-tris(2-methyl-4-hydroxy-5-tert-butylphenyl)butane, bis[2-tert-butyl-4-methyl-6-(2-hydroxy-3-tert-butyl-5-methylbenzyl)phenyl]terephthalate, 1,3,5-tris(2,6-dimethyl-3-hydroxy-4-tert-butylbenzyl)isocyanurate, 1,3,5-tris(3,5-di-tert-butyl-4-hydroxybenzyl)isocyanurate, 1,3,5-tris(3,5-di-tert-butyl-4-hydroxybenzyl)-2,4,6-trimethylbenzene, 1,3,5-tris[(3,5-di-tert-butyl-4-hydroxyphenyl)propionyloxyethyl]isocyanurate, tetrakis[methylene-3-(3',5'-tert-tributyl-4'-hydroxyphenyl)propionate]methane, 2-tert-butyl-4-methyl-6-(2-acryloyloxy-3-tert-butyl-5-methylbenzyl)phenol, 3,9-bis[2-(3-tert-butyl-4-hydroxy-5-methylhydrocinnamoyloxy)-1,1-dimethylethyl]-2,4,8, 10-tetraoxaspiro[5.5]undecane, triethylene glycol bis[β-(3-tert-butyl-4-hydroxy-5-methylphenyl)propionate], and 3-(3,5-dialkyl-4-hydroxyphenyl)propionic acid derivatives such as stearyl-3-(3,5-di-tert-butyl-4-hydroxyphenyl)propionic acid amide, palmityl-3-(3,5-di-tert-butyl-4-hydroxyphenyl)propionic acid amide, myristyl-3-(3,5-di-tert-butyl-4-hydroxyphenyl)propionic acid amide, and lauryl-3-(3,5-di-tert-butyl-4-hydroxyphenyl)propionic acid amide.

In the present invention, the phenol-based antioxidant is used preferably in an amount of 0.001 to 10 parts by mass, and more preferably 0.01 to 0.5 parts by mass, with respect to 100 parts by mass of the thermoplastic resin.

Examples of the above-mentioned phosphorus-based antioxidants include triphenyl phosphite, diisooctyl phosphite, heptakis triphosphite, triisodecyl phosphite, diphenylisooctyl phosphite, diisooctylphenyl phosphite, diphenyltridecyl phosphite, triisooctyl phosphite, trilauryl phosphite, diphenyl phosphite, tris(dipropylene glycol) phosphite, diisodecylpentaerythritol diphosphite, dioleyl hydrogen phosphite, trilauryl trithiophosphite, bis(tridecyl) phosphite, tris(isodecyl) phosphite, tris(tridecyl) phosphite, diphenyldecyl phosphite, dinonylphenylbis(nonylphenyl) phosphite, poly(dipropylene glycol)phenyl phosphite, tetraphenyl dipropyl glycol diphosphite, trisnonylphenyl phosphite, tris(2,4-di-tert-butylphenyl) phosphite, tris(2,4-di-tert-butyl-5-methylphenyl) phosphite, tris[2-tert-butyl-4-(3-tert-butyl-4-hydroxy-5-methylphenylthio)-5-methylphenyl] phosphite, tridecyl phosphite, octyldiphenyl phosphite, di(decyl)monophenyl phosphite, distearylpentaerythritol diphosphite, a mixture of distearylpentaerythritol and calcium stearate, alkyl(C10)bisphenol A phosphite, di(tridecyl)pentaerythritol diphosphite, di(nonylphenyl)pentaerythritol diphosphite, bis(2,4-di-tert-butylphenyl)pentaerythritol diphosphite, bis(2,6-di-tert-butyl-4-methylphenyl)pentaerythritol diphosphite, bis(2,4,6-tri-tert-butylphenyl)pentaerythritol diphosphite, bis(2,4-dicumylphenyl)pentaerythritol diphosphite, tetraphenyl-tetra(tridecyl)pentaerythritol tetraphosphite, bis(2,4-di-tert-butyl-6-methylphenyl)ethyl phosphite, tetra(tridecyl)isopropylidenediphenol diphosphite, tetra(tridecyl)-4,4'-n-butylidenebis(2-tert-butyl-5-methylphenol) diphosphite, hexa(tridecyl)-1,1,3-tris(2-methyl-4-hydroxy-5-tert-butylphenyl)butane triphosphite, tetrakis(2,4-di-tert-butylphenyl)biphenylene diphosphonite, 9,10-dihydro-9-oxa-10-phosphaphenanthrene-10-oxide, (1-methyl-1-propanyl-3-ylidene)tris(1,1-dimethylethyl)-5-methyl-4,1-phenylene)hexatrid ecyl phosphite, 2,2'-methylenebis(4,6-di-tert-butylphenyl)-2-ethylhexyl phosphite, 2,2'-methylenebis(4,6-di-tert-butylphenyl)-octadecyl phosphite, 2,2'-ethylidenebis(4,6-di-tert-butylphenyl) fluorophosphite, 4,4'-butylidenebis(3-methyl-6-tert-butylphenylditridecyl) phosphite, tris(2-[(2,4,8,10-tetrakis-tert-butyldibenzo[d,f][1,3,2]dioxaphosphepin-6-yl)oxy]ethyl)ami ne, 3,9-bis(4-nonylphenoxy)-2,4,8,10-tetraoxa-3,9-diphosphaspiro[5,5]undecane, 2,4,6-tri-tert-butylphenyl-2-butyl-2-ethyl-1,3-propanediol phosphite, and poly4,4'-isopropylidenediphenol C12-15 alcohol phosphite.

In the present invention, the phosphorus-based antioxidant is used preferably in an amount of 0.001 to 10 parts by mass, and more preferably 0.01 to 0.5 parts by mass, with respect to 100 parts by mass of the thermoplastic resin.

Examples of the above-mentioned thioether-based antioxidants include tetrakis[methylene-3-(laurylthio)propionate]methane, bis(methyl-4-[3-n-alkyl(C12/C14)thiopropionyloxy]5-tert-butylphenyl)sulfide, ditridecyl-3,3'-thio dipropionate, dilauryl-3,3'-thio dipropionate, dimyristyl-3,3'-thio dipropionate, distearyl-3,3'-thio dipropionate, lauryl/stearylthio dipropionate, 4,4'-thiobis(6-tert-butyl-m-cresol), 2,2'-thiobis(6-tert-butyl-p-cresol), and distearyl-disulfide.

In the present invention, the thioether-based antioxidant is used preferably in an amount of 0.001 to 10 parts by mass, and more preferably 0.01 to 0.5 parts by mass, with respect to 100 parts by mass of the thermoplastic resin.

Examples of the above-mentioned ultraviolet absorbers include 2-hydroxybenzophenones such as 2,4-dihydroxybenzophenone, 2-hydroxy-4-methoxybenzophenone, 2-hydroxy-4-octoxybenzophenone, and 5,5'-methylenebis(2-hydroxy-4-methoxybenzophenone); 2-(2-hydroxyphenyl)benzotriazoles such as 2-(2-hydroxy-5-methylphenyl)benzotriazole, 2-(2-hydroxy-5-tert-octylphenyl)benzotriazole, 2-(2-hydroxy-3,5-di-tert-butylphenyl)-5-chlorobenzotriazole, 2-(2-hydroxy-3-tert-butyl-5-methylphenyl)-5-chlorobenzotriazole, 2-(2-hydroxy-3,5-dicumylphenyl)benzotriazole, 2,2'-methylenebis(4-tert-octyl-6-benzotriazolyl phenol), polyethylene glycol ester of 2-(2-hydroxy-3-tert-butyl-5-carboxyphenyl)benzotriazole, 2-[2-hydroxy-3-(2-acryloyloxyethyl)-5-methylphenyl]benzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-octylphenyl]benzotriazole, 2-[2-hydroxy-3-(2-methacryloyloxyethyl)-5-tert-butylphenyl]-5-chlorobenzotriazole, 2-[2-hydroxy-5-(2-methacryloyloxyethyl)phenyl]benzotriazole, 2-[2-hydroxy-3-tert-butyl-5-(2-methacryloyloxyethyl)phenyl]benzotriazole, 2-[2-hydroxy-3-tert-amyl-5-(2-methacryloyloxyethyl)phenyl]benzotriazole, 2-[2-hydroxy-3-tert-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(3-methacryloyloxy-2-hydroxypropyl)phenyl]benzotriazole, and 2-[2-hydroxy-4-(3-methacryloyloxypropyl)phenyl]benzotriazole; 2-(2-hydroxyphenyl)-4,6-diaryl-1,3,5-triazines such as 2-(2-hydroxy-4-methoxyphenyl)-4,6-diphenyl-1,3,5-triazine, 2-(2-hydroxy-4-hexyloxyphenyl)-4,6-diphenyl-1,3,5-triazine, 2-(2-hydroxy-4-octoxyphenyl)-4,6-bis(2,4-dimethylphenyl)-1,3,5-triazine, 2-[2-hydroxy-4-(3-C12-13 mixed alkoxy-2-hydroxypropoxy)phenyl]-4,6-bis(2,4-dimethylphenyl)-1,3,5-triazine, 2-[2-hydroxy-4-(2-acryloyloxyethoxy)phenyl]-4,6-bis(4-methylphenyl)-1,3,5-triazine, 2-(2,4-dihydroxy-3-allylphenyl)-4,6-bis(2,4-dimethylphenyl)-1,3,5-triazine, and 2,4,6-tris(2-hydroxy-3-methyl-4-hexyloxyphenyl)-1,3,5-triazine; benzoates such as phenyl salicylate, resorcinol monobenzoate, 2,4-di-tert-butylphenyl-3,5-di-tert-butyl-4-hydroxybenzoate, octyl(3,5-di-tert-butyl-4-hydroxy) benzoate, dodecyl(3,5-di-tert-butyl-4-hydroxy) benzoate, tetradecyl(3,5-di-tert-butyl-4-hydroxy) benzoate, hexadecyl(3,5-di-tert-butyl-4-hydroxy) benzoate, octadecyl(3,5-di-tert-butyl-4-hydroxy) benzoate, and behenyl(3,5-di-tert-butyl-4-hydroxy) benzoate; substituted oxanilides such as 2-ethyl-2'-ethoxy oxanilide and 2-ethoxy-4'-dodecyl oxanilide; cyano acrylates such as ethyl-α-cyano-β,β-diphenyl acrylate and methyl-2-cyano-3-methyl-3-(p-methoxyphenyl) acrylate; various metal salts and metal chelates, particularly salts and chelates of nickel and chromium.

In the present invention, the ultraviolet absorber is used preferably in an amount of 0.001 to 5 parts by mass, and more preferably 0.01 to 0.5 parts by mass, with respect to 100 parts by mass of the thermoplastic resin.

Examples of the above-mentioned hindered amine-based photostabilizers include 2,2,6,6-tetramethyl-4-piperidyl stearate, 1,2,2,6,6-pentamethyl-4-piperidyl stearate, 2,2,6,6-tetramethyl-4-piperidyl benzoate, bis(2,2,6,6-tetramethyl-4-piperidyl) sebacate, tetrakis(2,2,6,6-tetramethyl-4-piperidyl)-1,2,3,4-butanetetra carboxylate, tetrakis(1,2,2,6,6-pentamethyl-4-piperidyl)-1,2,3,4-butanetetra carboxylate, bis(2,2,6,6-tetramethyl-4-piperidyl)-di(tridecyl)-1,2,3,4-butanetetra carboxylate, bis(1,2,2,6,6-pentamethyl-4-piperidyl)-di(tridecyl)-1,2,3,4-butanetetra carboxylate, bis(1,2,2,4,4-pentamethyl-4-piperidyl)-2-butyl-2-(3,5-di-tert-butyl-4-hydroxybenzyl) malonate, a polycondensate between 1-(2-hydroxyethyl)-2,2,6,6-tetramethyl-4-piperidinol and diethyl succinate, a polycondensate between 1,6-bis(2,2,6,6-tetramethyl-4-piperidylamino)hexane and 2,4-dichloro-6-morpholino-s-triazine, a polycondensate between 1,6-bis(2,2,6,6-tetramethyl-4-piperidylamino)hexane and 2,4-dichloro-6-tert-octylamino-s-triazine, 1,5,8,12-tetrakis[2,4-bis(N-butyl-N-(2,2,6,6-tetramethyl-4-piperidyl)amino)-s-triazin-6-yl ]-1,5,8,12-tetraazadodecane, 1,5,8,12-tetrakis[2,4-bis(N-butyl-N-(1,2,2,6,6-pentamethyl-4-piperidyl)amino)-s-triazin-6 -yl]-1,5,8-12-tetraazadodecane, 1,6,11 -tris[2,4-bis(N-butyl-N-(2,2,6,6-tetramethyl-4-piperidyl)amino)-s-triazin-6-yl]amin oundecane, 1,6,11-tris[2,4-bis(N-butyl-N-(1,2,2,6,6-pentamethyl-4-piperidyl)amino)-s-triazin-6-yl]a minoundecane, bis {4-(1-octyloxy-2,2,6,6-tetramethyl)piperidyl} decanedionate, bis{4-(2,2,6,6-tetramethyl-1-undecyloxy)piperidyl) carbonate, and TINUVIN NOR 371 manufactured by Ciba Specialty Chemicals.

In the present invention, the above-mentioned hindered amine-based photostabilizer is used preferably in an amount of 0.001 to 5 parts by mass, and more preferably 0.005 to 0.5 parts by mass, with respect to 100 parts by mass of the thermoplastic resin.

Examples of the above-mentioned nucleating agents include metal carboxylates such as sodium benzoate, aluminum 4-tert-butylbenzoate, sodium adipate, and 2-sodium bicyclo[2.2.1]heptane-2,3-dicarboxylate; metal phosphates such as sodium bis(4-tert-butylphenyl)phosphate, sodium-2,2'-methylenebis(4,6-di-tert-butylphenyl)phosphate, and lithium-2,2'-methylenebis(4,6-di-tert-butylphenyl)phosphate; polyhydric alcohol derivatives such as dibenzylidene sorbitol, bis(methylbenzylidene)sorbitol, bis(3,4-dimethylbenzylidene)sorbitol, bis(p-ethylbenzylidene)sorbitol, and bis(dimethylbenzylidene)sorbitol; and amide compounds such as N,N',N"-tris[2-methylcyclohexyl]-1,2,3-propane tricarboxamide, N,N',N"-tricyclohexyl-1,3,5-benzene tricarboxamide, N,N'-dicyclohexylnaphthalene dicarboxamide, and 1,3,5-tri(dimethylpropanamide)benzene.

In the present invention, the nucleating agent is used preferably in an amount of 0.001 to 5 parts by mass, and more preferably 0.005 to 0.5 parts by mass, with respect to 100 parts by mass of the thermoplastic resin.

Examples of the above-mentioned flame retardants include aromatic phosphates such as triphenyl phosphate, tricresyl phosphate, tryxylenyl phosphate, cresyldiphenyl phosphate, cresyl-2,6-dixylenyl phosphate, resorcinolbis(diphenylphosphate), (1-methylethylidene)-4,1-phenylenetetraphenyl diphosphate, 1,3-phenylenetetrakis(2,6-dimethylphenyl) phosphate, ADK STAB FP-500 manufactured by ADEKA Corporation, ADK STAB FP-600 manufactured by ADEKA Corporation, and ADK STAB FP-800 manufactured by ADEKA Corporation; phosphonates such as divinyl phenylphosphonate, diallyl phenylphosphonate, and 1-butenyl phenylphosphonate; phosphinates such as phenyl diphenylphosphinate, methyl diphenylphosphinate, and 9,10-dihydro-9-oxa-10-phosphaphenanthrene-10-oxide derivatives; phosphazene compounds such as bis(2-allylphenoxy)phosphazene and dicresyl phosphazene; phosphorus-based flame retardants such as melamine phosphate, melamine pyrophosphate, melamine polyphosphate, melam polyphosphate, ammonium polyphosphate, piperazine phosphate, piperazine pyrophosphate, piperazine polyphosphate, phosphorus-containing vinylbenzyl compounds, and red phosphorus; metal hydroxides such as magnesium hydroxide and aluminum hydroxide; and bromine-based flame retardants such as a brominated bisphenol A epoxy resin, a brominated phenol novolac epoxy resin, hexabromobenzene, pentabromotoluene, ethylenebis(pentabromophenyl), ethylenebis-tetrabromophthalimide, 1,2-dibromo-4-(1,2-dibromoethyl)cyclohexane, tetrabromocyclooctane, hexabromocyclododecane, bis(tribromophenoxy)ethane, brominated polyphenylene ether, brominated polystyrene, 2,4,6-tris(tribromophenoxy)-1,3,5-triazine, tribromophenyl maleimide, tribromophenyl acrylate, tribromophenyl methacrylate, tetrabromo bisphenol A dimethacrylate, pentabromobenzyl acrylate, and brominated styrene. It is preferable to use these flame retardants together with a drip preventing agent such as a fluororesin and a flame retardant assistant such as a polyhydric alcohol or a hydrotalcite.

In the present invention, the flame retardant is used preferably in an amount of 1 to 50 parts by mass, and more preferably 10 to 30 parts by mass, with respect to 100 parts by mass of the thermoplastic resin.

The above-mentioned lubricant is added for the purpose of providing a lubricating property to the surface of the molded article and enhancing a damage preventing effect. Examples of the lubricants include unsaturated fatty acid amides such as oleic acid amide and erucic acid amide; and saturated fatty acid amides such as behenic acid amide and stearic acid amide. These lubricants may be used alone or in combination of two or more.

In the present invention, the lubricant is used preferably in an amount of 0.03 to 2 parts by mass, and more preferably 0.01 to 0.5 parts by mass, with respect to 100 parts by mass of the thermoplastic resin.

Examples of the above-mentioned fillers include talc, mica, calcium carbonate, calcium oxide, calcium hydroxide, magnesium carbonate, magnesium hydroxide, magnesium oxide, magnesium sulfate, aluminum hydroxide, barium sulfate, glass powder, glass fiber, clay, dolomite, mica, silica, alumina, potassium titanate whisker, wollastonite, and fibrous magnesium oxysulfate. The particle diameter (fiber diameter, fiber length, and aspect ratio in the case of a fibrous filler) of these fillers can be selected as appropriate. Moreover, the fillers that have been subjected to surface processing can be used as needed.

In the present invention, the filler is used preferably in an amount of 0.01 to 80 parts by mass, and more preferably 1 to 50 parts by mass, with respect to 100 parts by mass of the thermoplastic resin.

Hydrotalcites are composite salt compounds known as a natural product and a synthetic product and composed of magnesium, aluminum, a hydroxyl group, a carbonate group, and optional crystallization water, and examples of the above-mentioned hydrotalcites include salts obtained by replacing a portion of magnesium or aluminum with another metal such as alkali metal or zinc and salts obtained by replacing the hydroxyl group or the carbonate group with another anionic group. Specific examples thereof include salts obtained by replacing metal in hydrotalcites represented by General Formula (5) below with alkali metal. Also, as Al-Li-based hydrotalcites, compounds represented by General Formula (6) below can be used.
[Chem.46]

Mgₓ₁Znₓ₂Al₂(OH)_{2(x1+X2)+4}(CO₃)*p*H₂O (5)

(In the formula, x1 and x2 represent numbers that satisfy the conditions shown by the following formulae: 0≤x2/x1<10 and 2≤x1+x2≤20; and p represents 0 or a positive number.)
[Chem.47]

[Li_{1/3}Al_{2/3}(OH)₂]●[A^{q-}_{1/3q}●*p*H₂O] (6)

(In the formula, Aq- represents an anion with a valency of q, and p represents 0 or a positive number.)

Also, a portion of the carbonate anions in the above-mentioned hydrotalcites may be replaced with other anions.

The above-mentioned hydrotalcites may be dehydrated and coated with a higher fatty acid such as stearic acid, a metal salt of a higher fatty acid such as an alkali metal oleate, a metal salt of an organic sulfonic acid such as an alkali metal dodecylbenzenesulfonate, a higher fatty acid amide, a higher fatty acid ester, wax, or the like.

The above-mentioned hydrotalcites may be a natural product or a synthetic product. Examples of the method for synthesizing the compound include known methods disclosed in JP S46-2280B, JP S50-30039B, JP S51-29129B, JP H3-36839B, JP S61-174270A, JP H5-179052A, and the like. The above-mentioned hydrotalcites can be used without being limited by their crystal structures and crystal grains.

In the present invention, the hydrotalcite is used preferably in an amount of 0.001 to 5 parts by mass, and more preferably 0.05 to 3 parts by mass, with respect to 100 parts by mass of the thermoplastic resin.

Examples of the above-mentioned antistatic agents include cationic antistatic agents such as fatty acid quaternary ammonium ion salts and polyamine quaternary salts; anionic antistatic agents such as higher alcohol phosphates, higher alcohol EO adducts, polyethylene glycol fatty acid esters, anionic alkylsulfonates, higher alcohol sulfates, higher alcohol ethylene oxide adduct sulfates, and higher alcohol ethylene oxide adduct phosphates; nonionic antistatic agents such as polyhydric alcohol fatty acid esters, polyglycol phosphates, and polyoxyethylene alkyl allyl ethers; and amphoteric antistatic agents such as amphoteric alkyl betaines (e.g., alkyldimethylamino acetic acid betaine) and imidazoline-type amphoteric activators. These antistatic agents may be used alone or in combination of two or more.

In the present invention, the antistatic agent is used preferably in an amount of 0.03 to 2 parts by mass, and more preferably 0.1 to 0.8 parts by mass, with respect to 100 parts by mass of the thermoplastic resin.

The thermoplastic resin composition according to the present invention can be preferably used in vehicle members. Examples of the vehicle members include interior and exterior members, outer plates, and resin windows for a vehicle such as an automobile, an industrial vehicle, a personal vehicle, a self-traveling vehicle body, or a train. The resin composition according to present invention may be used as resin base materials or protective coatings for these members.

Examples of the above-mentioned exterior members for a vehicle include door moldings, frameworks for a door mirror, doorknobs, wheel covers, spoilers, bumpers, blinker lenses, pillar garnishes, rear finishers, and headlamp covers.

Examples of the above-mentioned interior members for a vehicle include instrument panels, console boxes, meter covers, door lock bezels, steering wheels, power window switch bases, window handles, center clusters, dashboards, and hoods.

Examples of the above-mentioned outer plates for a vehicle include front fenders, door panels, roof panels, hood panels, trunk lids, and back door panels.

Examples of the above-mentioned resin windows for a vehicle include sunroofs, windshields, side glass, rear glass, rear quarter glass, and rear door quarter glass.

Also, the thermoplastic resin composition according to the present invention can be used in electric apparatuses, electronic apparatuses, and housings, and is preferable as a material of home electrical appliances, particularly a plastic material to be used in exteriors of the home electrical appliances. Examples of the home electrical appliances include televisions, videocassette recorders, DVD recorders, Blu-ray Disk player, audios, component stereo systems, refrigerators, microwave ovens, rice cookers, washing machines, dish washing machines, vacuum cleaners, and air conditioners.

Hereinafter, the present invention will be described by way of examples and comparative examples. The present invention is not limited to the following examples and the like.

### Examples 1-1 to 1-6 and Comparative Examples 1-1 to 1-5

### Evaluation by NOx testing

The antioxidants for a thermoplastic resin according to the present invention (Compounds 1 to 6) and conventional antioxidants shown in Table 1 were each placed in a petri dish and allowed to stand for 24 hours in the environment where the concentration of NOx was 3% and the temperature was 40°C. The YI. of the antioxidants that had undergone the testing was measured using a spectral colorimeter (SC-T manufactured by Suga Test Instruments Co., Ltd.). Table 1 below shows the results.

**Table 1**

| | Antioxidant | Y.I. before testing | Y.I. after testing |
|---|---|---|---|
| Ex. 1-1 | Compound 1¹⁾ | 2.1 | 20 |
| Ex. 1-2 | Compound 2²⁾ | 3.0 | 16 |
| Ex. 1-3 | Compound 3³⁾ | 3.5 | 23 |
| Ex. 1-4 | Compound 4⁴⁾ | 1.9 | 2.2 |
| Ex. 1-5 | Compound 5⁵⁾ | 2.4 | 5.1 |
| Ex. 1-6 | Compound 6⁶⁾ | 4.8 | 18 |
| Comp. Ex. 1-1 | AO-20⁷⁾ | 2.6 | 58 |
| Comp. Ex. 1-2 | AO-30⁸⁾ | 10.1 | 84 |
| Comp. Ex. 1-3 | AO-40⁹⁾ | 2.3 | 92 |
| Comp. Ex. 1-4 | AO-60¹⁰⁾ | 5.0 | 79 |
| Comp. Ex. 1-5 | AO-80¹¹⁾ | 5.3 | 33 |

1) Compound 1: Tetrakis[methylene-3-(3,5-di-tert-butyl-4-tert-butylcarbonatephenyl) propionate]methane 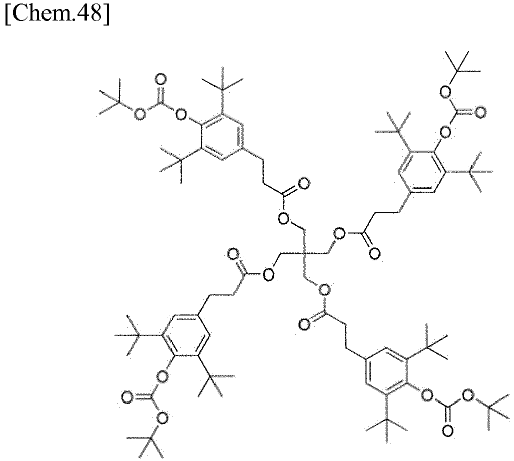
2) Compound 2: 1,3,5-Tris(3,5-di-tert-butyl-4-tert-butylcarbonatebenzyl)isocyanurate 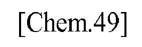 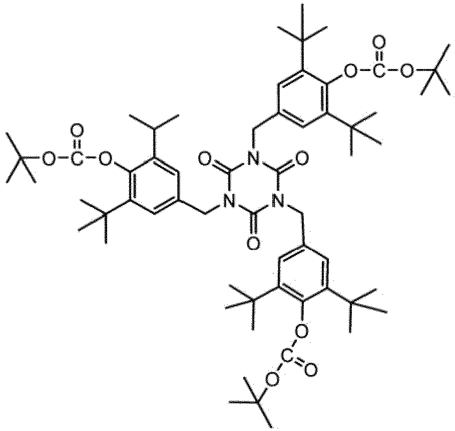
3) Compound 3: 1,1,3-Tris(2-methyl-4-tert-butylcarbonate-5-tert-butylphenyl)butane 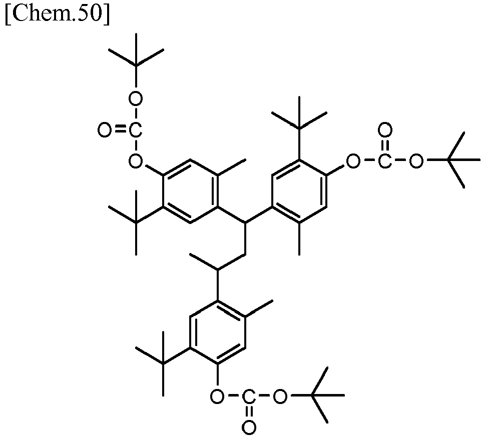
4) Compound 4: 1,1-Bis(4-tert-butylcarbonate-2-methyl-5-tert-butylphenyl)butane 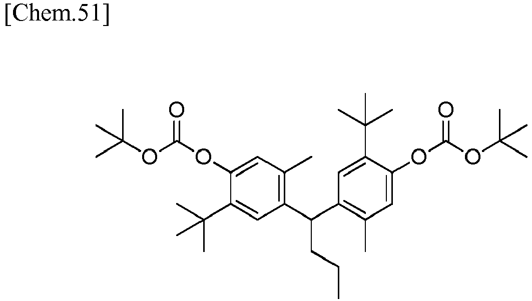
5) Compound 5:3,9-Bis(2-(3-(3-t-butyl-4-tert-butylcarbonate-5-methylphenyl) propionyloxy-1,1-dimethylethyl)-2,4,8,10-tetraoxaspiro[5.5]undecane 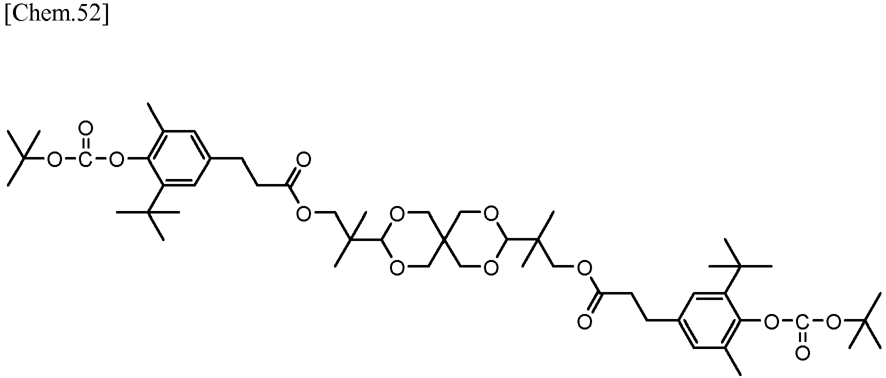
6) Compound 6: 4,4'-[1-[4-[1-(4tert-butylcarbonatephenyl)-1-methylethyl]phenyl] ethylidene]bisphenol 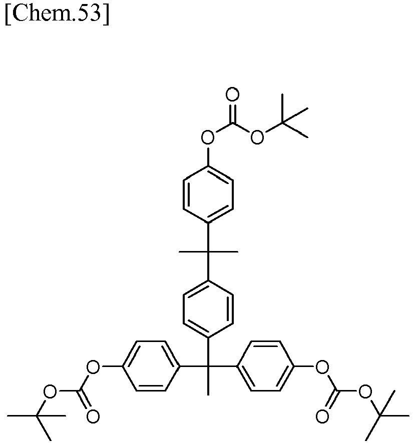
7)AO-20: 1,3,5-Tris(3,5-di-tert-butyl-4-hydroxybenzyl)isocyanurate 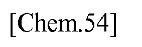 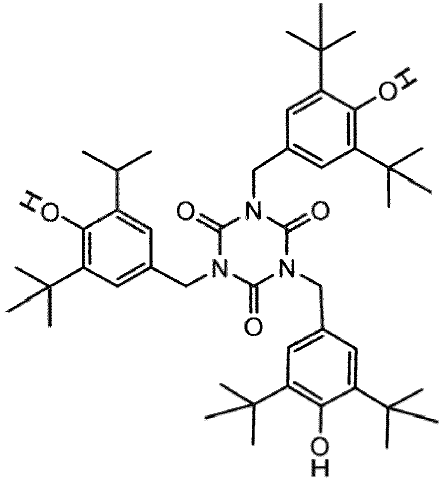
8) AO-30: 1,1,3-Tris(2-methyl-4-hydroxy-5-tert-butylphenyl)butane 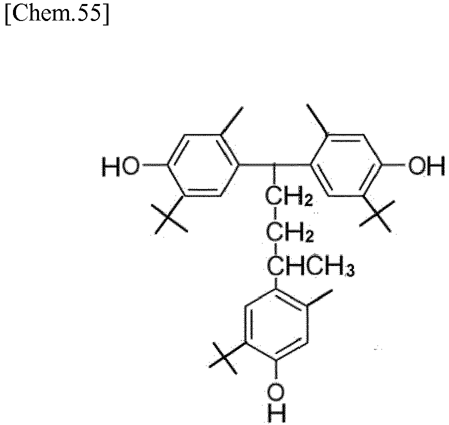
9) AO-40: 4,4'-Butylidenebis(6-tert-butyl-m-cresol) 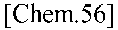 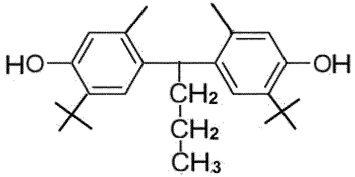
10) AO-60: Tetrakis[methylene-3-(3,5-di-tert-butyl-4-hydroxyphenyl)propionate]methane 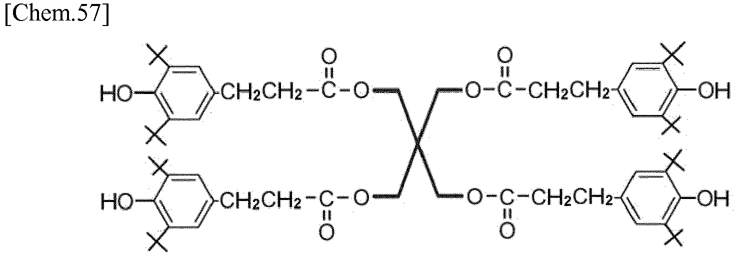
11) AO-80: 3,9-Bis(2-(3-(3-t-butyl-4-hydroxy-5-methylphenyl)propionyloxy -1,1-dimethylethyl)-2,4,8,10-tetraoxaspiro[5,5]undecane

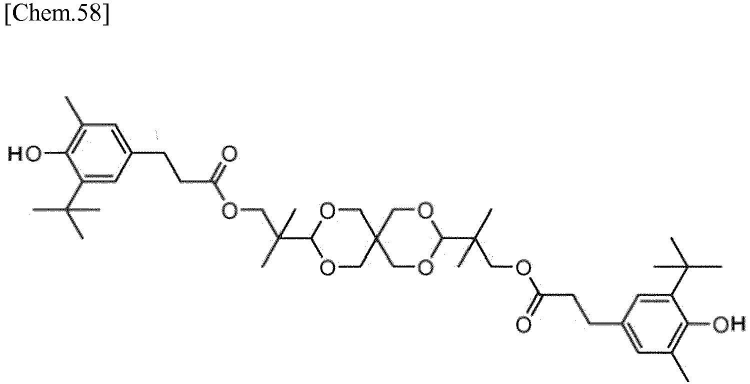

### Examples 2-1 to 2-8 and Comparative Examples 2-1 to 2-8

To 100 parts by mass of homopolypropylene (Prime Polypro H700 manufactured by Prime Polymer Co., Ltd.; MFR=9.9 g/10 min), 0.05 parts by mass of each of the antioxidants for a thermoplastic resin according to the present invention (Compounds 1 to 8) and conventional antioxidants shown in Tables 2, 3, and 4, and 0.05 parts by mass of calcium stearate were added, and mixed for 30 minutes using a rocking mixer. After the mixing, the mixture was granulated at a melting temperature of 230°C and a screw speed of 30 rpm using a single screw extruder (OEX3024 manufactured by DDM Co., Ltd.), and thus pellets were manufactured. The obtained pellets were evaluated as follows.

### Influence on melt flow rate

The melt flow rates (MFRs: g/10 min) of the pellets obtained in the above-mentioned examples and comparative examples were measured in accordance with JIS K 7210 (at a temperature of 230°C and a weight of 1.85 kg). Tables 2 to 4 below show the results.

### 150°C oven testing

An injection molding apparatus (EC 100-2A manufactured by Toshiba Machine Co., Ltd.) was used to subject the pellets obtained in the above-mentioned examples and comparative examples to injection molding under the conditions that the injection temperature was 230°C and the temperature of a metal mold was 40°C, and thus test pieces having a flat plate shape and a size of 20 mm×60 mm×2 mm were obtained. Just after the injection molding, the test pieces were allowed to stand for 48 hours or more in a constant temperature chamber in which the chamber temperature was set at 23°C and then placed in an oven in which the temperature was set at 150°C. After a week, the states of the test pieces were checked. The test pieces in which no abnormalities of the external appearance were observed were evaluated as "Good", the test pieces that had been colored were evaluated as "Fair", and the test pieces that had been thermally embrittled were evaluated as "Poor". Tables 2 to 4 show the results.

**Table 2**

| | | Examples | | | | | | |
|---|---|---|---|---|---|---|---|---|
| | | 2-1 | 2-2 | 2-3 | 2-4 | 2-5 | 2-6 | 2-7 |
| | 2112 11) | 0.05 | 0.05 | 0.05 | 0.05 | 0.05 | 0.05 | 0.05 |
| | Compound 1 | 0.05 | | | | | | |
| | Compound 2 | | 0.05 | | | | | |
| | Compound 3 | | | 0.05 | | | | |
| | Compound 4 | | | | 0.05 | | | |
| | Compound 5 | | | | | 0.05 | | |
| Blend amount of antioxidant | Compound 7 12) | | | | | | 0.05 | |
| | Compound 8 13) | | | | | | | 0.05 |
| | AO-60 | | | | | | | |
| | AO-20 | | | | | | | |
| | AO-30 | | | | | | | |
| | AO-40 | | | | | | | |
| | AO-80 | | | | | | | |
| | AO-50 14) | | | | | | | |
| | A-330 15) | | | | | | | |
| Thermal resistance | MFR (g / 10 min.) | 15.2 | 15.9 | 13.1 | 13.2 | 13.0 | 16.5 | 13.3 |
| | 150°C oven | Good | Fair | Fair | Fair | Good | Fair | Good |

**Table 3**

| | | Example | Comparative Example | | | |
|---|---|---|---|---|---|---|
| | | 2-8 | 2-1 | 2-2 | 2-3 | 2-4 |
| | 2112 11) | 0.05 | 0.05 | 0.05 | 0.05 | 0.05 |
| | Compound 1 | | | | | |
| | Compound 2 | | | | | |
| | Compound 3 | | | | | |
| | Compound 4 | | | | | |
| | Compound 5 | | | | | |
| Blend amount of antioxidant | Compound 7 12) | | | | | |
| | Compound 8 13) | | | | | |
| | Compound 6 | 0.05 | | | | |
| | AO-60 | | | 0.05 | | |
| | AO-20 | | | | 0.05 | |
| | AO-30 | | | | | 0.05 |
| | AO-40 | | | | | |
| | AO-80 | | | | | |
| | AO-50 14) | | | | | |
| | A-330 15) | | | | | |
| Thermal resistance | MFR (g / 10 min.) | 17.6 | 17.9 | 12.7 | 13.4 | 11.0 |
| | 150°C oven | Fair | Poor | Good | Fair | Fair |

11) 2112: Tris(2,4-di-tert-butylphenyl)phosphite 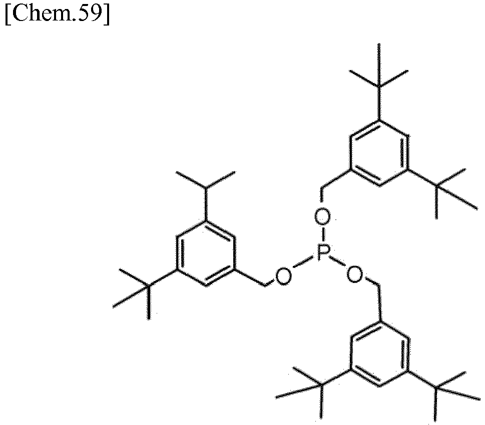
12) Compound 7: Stearyl 3-(3,5-di-tert-butyl-4-tert-butylcarbonatephenyl)propionate 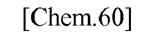 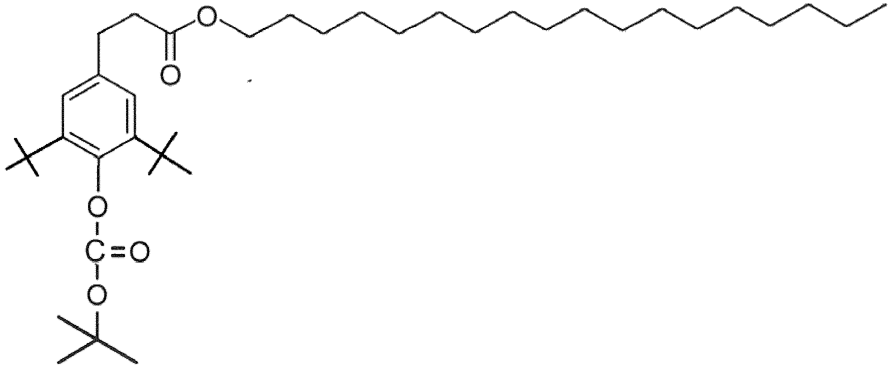
13) Compound 8: 2,4,6-Tris(3',5'-di-tert-butyl-4'-butylcarbonatebenzyl)mesitylene 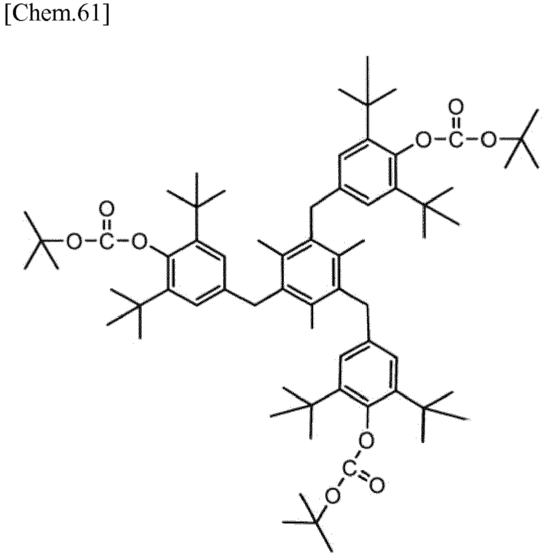
14) AO-50: Stearyl 3-(3,5-di-tert-butyl-4-hydroxyphenyl)propionate 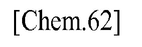 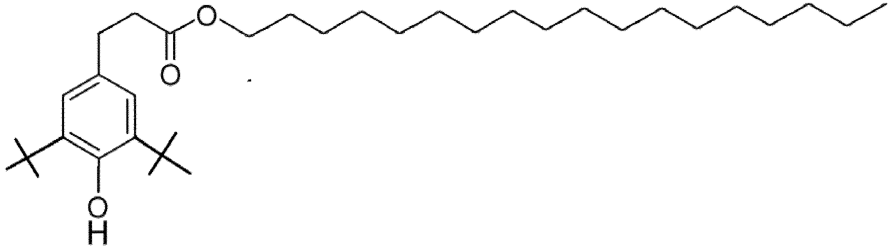
15) AO-330: 2,4,6-Tris(3',5'-di-tert-butyl-4'-hydroxybenzyl)mesitylene

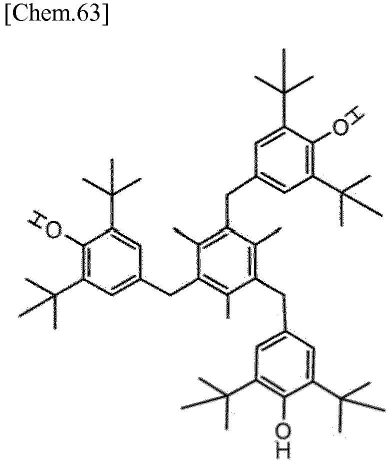

**Table 4**

| | | Comparative Example | | | |
|---|---|---|---|---|---|
| | | 2-5 | 2-6 | 2-7 | 2-8 |
| | 2112 11) | 0.05 | 0.05 | 0.05 | 0.05 |
| | Compound 1 | | | | |
| | Compound 2 | | | | |
| | Compound 3 | | | | |
| | Compound 4 | | | | |
| | Compound 5 | | | | |
| | Compound 7 12) | | | | |
| Blend amount of antioxidant | Compound 8 13) | | | | |
| | Compound 9 14) | | | | |
| | AO-60 | | | | |
| | AO-20 | | | | |
| | AO-30 | | | | |
| | AO-40 | 0.05 | | | |
| | AO-80 | | 0.05 | | |
| | AO-50 15) | | | 0.05 | |
| | A-330 16) | | | | 0.05 |
| Thermal resistance | MFR (g /10 min.) | 11.2 | 12.2 | 14.5 | 12.2 |
| | 150°C oven | Fair | Good | Fair | Good |

It is clear from the results of Comparative Examples 1-1 to 1-5 shown in Table 1 that the conventionally used phenol-based antioxidants significantly turned yellow due to exposure to nitrogen oxide in the NOx testing, whereas it is clear from the results of Examples 1-1 to 1-6 that the antioxidants for a thermoplastic resin according to the present invention slightly turned yellow.

In addition, it can be confirmed from the results of Examples 2-1 to 2-8 and Comparative Examples 2-1 to 2-8 shown in Table 2 that the antioxidants for a synthetic resin according to the present invention have an effect of improving thermal resistance of polyolefin-based resins, the effect being similar to that of the conventional antioxidants.

Accordingly, the antioxidants for a thermoplastic resin according to the present invention are antioxidants that provide excellent thermal resistance to a thermoplastic resin and that can also suppress a change in color in the NOx testing, and thus the antioxidants can be preferably used in vehicle interior and exterior materials.

## Claims

1. An antioxidant for a thermoplastic resin represented by any of General Formulae (2) to (4) below: 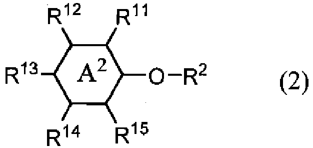
wherein a ring A² is a six-membered hydrocarbon ring, aromatic ring, or heterocycle,
R² represents an unsubstituted or substituted alkyl group having 1 to 20 carbon atoms, an unsubstituted or substituted alkenyl group having 2 to 20 carbon atoms, an unsubstituted or substituted aryl group having 6 to 30 carbon atoms, an unsubstituted or substituted arylalkyl group having 7 to 30 carbon atoms, an unsubstituted or substituted heterocycle-containing group having 3 to 35 carbon atoms, or a trialkylsilyl group,
a methylene group in the alkyl group, alkoxy group or arylalkyl group represented by R² is optionally replaced with one or more groups selected from a carbon-carbon double bond, -O-, -S-, -CO-, -O-CO-, -CO-O-, -O-CO-O-, -S-CO-, -CO-S-, -S-CO-O-, -O-CO-S-, -CO-NH-, -NH-CO-, -NH-CO-O-, -NR'-, -S-S-, and -SO₂-(provided that oxygen atoms are not adjacent to each other when the methylene group is replaced with two or more groups),
R' represents a hydrogen atom or an alkyl group having 1 to 8 carbon atoms,
R¹¹, R¹², R¹³, R¹⁴, and R¹⁵ represent a hydrogen atom, a halogen atom, a cyano group, a hydroxyl group, a nitro group, carboxyl group, an unsubstituted or substituted alkyl group having 1 to 40 carbon atoms, an unsubstituted or substituted alkoxy group having 1 to 8 carbon atoms, an unsubstituted or substituted aryl group having 6 to 30 carbon atoms, an unsubstituted or substituted arylalkyl group having 7 to 30 carbon atoms, an unsubstituted or substituted heterocycle-containing group having 3 to 20 carbon atoms, or -O-R²,
a methylene group in the alkyl group or arylalkyl group represented by R¹¹, R¹², R¹³, R¹⁴, and R¹⁵ is optionally replaced with one or more groups selected from -O-, -CO-, -OC-, -O-CO-, and -CO-O- (provided that oxygen atoms are not adjacent to each other when the methylene group is replaced with two or more groups), and
at least one of R¹¹, R¹², R¹³, R¹⁴, and R¹⁵ is not a hydrogen atom; 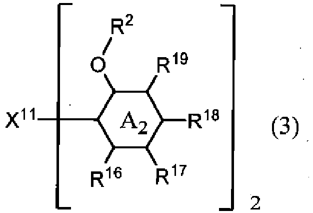
wherein X¹¹ represents a group represented by General Formula (3-1) below,
R¹⁶, R¹⁷, R¹⁸, and R¹⁹ represent a hydrogen atom, a halogen atom, a cyano group, a hydroxyl group, a nitro group, a carboxyl group, an unsubstituted or substituted alkyl group having 1 to 40 carbon atoms, an unsubstituted or substituted alkoxy group having 1 to 8 carbon atoms, an unsubstituted or substituted aryl group having 6 to 30 carbon atoms, an unsubstituted or substituted arylalkyl group having 7 to 30 carbon atoms, or an unsubstituted or substituted heterocycle-containing group having 3 to 20 carbon atoms,
at least one of R¹⁶, R¹⁷, R¹⁸, and R¹⁹ is not a hydrogen atom, and
R² and a ring A² are the same as R² and the ring A² in General Formula (2),
*-Z¹-X¹-Z²-* (3-1)
wherein X¹ represents -CR³⁰R³¹-, -NR³²-, an unsubstituted or substituted divalent aliphatic hydrocarbon group having 1 to 120 carbon atoms, an unsubstituted or substituted divalent aromatic ring-containing hydrocarbon group having 6 to 35 carbon atoms, an unsubstituted or substituted divalent heterocycle-containing group having 3 to 35 carbon atoms, or a substituent represented by one of formulae (3-2) to (3-5) below,
the aliphatic hydrocarbon group is optionally interrupted by -O-, -S-, -CO-, -COO-, -OCO-, or -NH-, or a linking group in which these groups are used in combination in a state in which oxygen atoms are not adjacent to each other,
R³⁰ and R³¹ represent a hydrogen atom, an unsubstituted or substituted alkyl group having 1 to 10 carbon atoms, an unsubstituted or substituted alkoxy group having 1 to 8 carbon atoms, an unsubstituted or substituted aryl group having 6 to 30 carbon atoms, or an unsubstituted or substituted arylalkyl group having 7 to 30 carbon atoms,
Z¹ and Z² independently represent a direct bond, -O-, -S-, >CO, -CO-O-, -O-CO-, -SO₂-, -SS-, -SO-, >NR³³, an unsubstituted or substituted aliphatic hydrocarbon group having 1 to 35 carbon atoms, an unsubstituted or substituted aromatic ring-containing hydrocarbon group having 6 to 35 carbon atoms, or an unsubstituted or substituted heterocycle-containing group having 3 to 35 carbon atoms, and
R³² and R³³ represent a hydrogen atom, an unsubstituted or substituted aliphatic hydrocarbon group having 1 to 35 carbon atoms, an unsubstituted or substituted aromatic ring-containing hydrocarbon group having 6 to 35 carbon atoms, or an unsubstituted or substituted heterocycle-containing group having 3 to 35 carbon atoms, 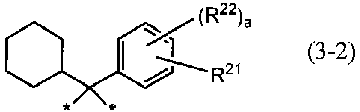
wherein R²¹ represents a hydrogen atom, an unsubstituted or substituted phenyl group, or an unsubstituted or substituted cycloalkyl group having 3 to 10 carbon atoms,
R²² represents an unsubstituted or substituted alkyl group having 1 to 10 carbon atoms, an unsubstituted or substituted alkoxy group having 1 to 8 carbon atoms, an unsubstituted or substituted alkenyl group having 2 to 10 carbon atoms, or a halogen atom, and
a is an integer from 0 to 5, 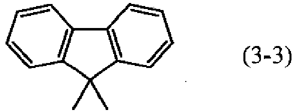 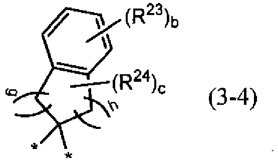
wherein R²³ and R²⁴ independently represent an unsubstituted or substituted alkyl group having 1 to 10 carbon atoms, an unsubstituted or substituted alkoxy group having 1 to 8 carbon atoms, an unsubstituted or substituted aryl group having 6 to 30 carbon atoms, an unsubstituted or substituted aryloxy group having 6 to 30 carbon atoms, an unsubstituted or substituted arylthio group having 6 to 30 carbon atoms, an unsubstituted or substituted arylalkenyl group having 6 to 30 carbon atoms, an unsubstituted or substituted arylalkyl group having 7 to 30 carbon atoms, an unsubstituted or substituted heterocyclic group having 3 to 20 carbon atoms, or a halogen atom,
a methylene group in the alkyl group, the alkoxy group, or the arylalkyl group is optionally replaced with an unsaturated bond, -O-, or -S-,
R²³ optionally forms a ring together with adjacent R²³,
b represents a number from 0 to 4,
c represents a number from 0 to 8,
g represents a number from 0 to 4,
h represents a number from 0 to 4, and
a total number of g and h is 2 to 4, and 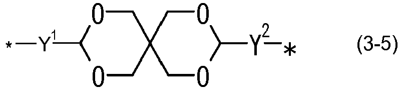
wherein Y¹ and Y² independently represent an alkylene group having 1 to 8 carbon atoms, and a methylene group in the alkylene group is optionally replaced with one or more groups selected from -O-, -CO-, -OC-, -O-CO-, and -CO-O-; and 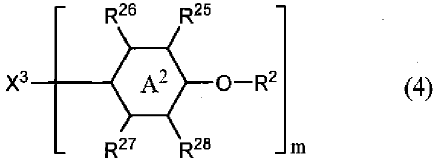
wherein m=2 to 6,
X³ is a group represented by General Formula (3-1) when m=2, X³ is a group represented by General Formula (4-1) when m=3, X³ is a group represented by General Formula (4-2) when m=4, X³ is a group represented by General Formula (4-3) when m=5, and X³ is a group represented by General Formula (4-4) when m=6,
R²⁵, R²⁶, R²⁷, and R²⁸ represent a hydrogen atom, a halogen atom, a cyano group, a hydroxyl group, a nitro group, a carboxyl group, an unsubstituted or substituted alkyl group having 1 to 40 carbon atoms, an unsubstituted or substituted alkoxy group having 1 to 8 carbon atoms, an unsubstituted or substituted aryl group having 6 to 30 carbon atoms, an unsubstituted or substituted arylalkyl group having 7 to 30 carbon atoms, or an unsubstituted or substituted heterocycle-containing group having 3 to 20 carbon atoms,
at least one of R²⁵, R²⁶, R²⁷, and R²⁸ is not a hydrogen atom, and
R² and a ring A² are the same as R² and the ring A² in General Formula (2), 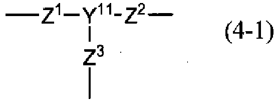
wherein Y¹¹ represents an unsubstituted or substituted trivalent aliphatic hydrocarbon group having 1 to 35 carbon atoms, an unsubstituted or substituted trivalent aromatic ring-containing hydrocarbon group having 6 to 35 carbon atoms, or an unsubstituted or substituted trivalent heterocycle-containing group having 3 to 35 carbon atoms,
Z¹, Z², and Z³ independently represent a direct bond, -O-, -S-, >CO, -CO-O-, -O-CO-, -SO₂-, -SS-, -SO-, >NR³⁴, >PR³⁴, an unsubstituted or substituted aliphatic hydrocarbon group having 1 to 35 carbon atoms, an unsubstituted or substituted aromatic ring-containing hydrocarbon group having 6 to 35 carbon atoms, or an unsubstituted or substituted heterocycle-containing group having 3 to 35 carbon atoms,
R³⁴ represents a hydrogen atom, an unsubstituted or substituted aliphatic hydrocarbon group having 1 to 35 carbon atoms, an unsubstituted or substituted aromatic ring-containing hydrocarbon group having 6 to 35 carbon atoms, or an unsubstituted or substituted heterocycle-containing group having 3 to 35 carbon atoms, and
the aliphatic hydrocarbon group is optionally interrupted by a carbon-carbon double bond, -O-, -CO-, -O-CO-, -CO-O-, or -SO₂-, 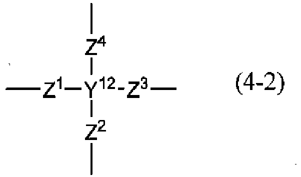
wherein Y¹² represents a carbon atom, an unsubstituted or substituted tetravalent aliphatic hydrocarbon group having 1 to 35 carbon atoms, an unsubstituted or substituted tetravalent aromatic ring-containing hydrocarbon group having 6 to 35 carbon atoms, or an unsubstituted or substituted tetravalent heterocycle-containing group having 3 to 35 carbon atoms,
the aliphatic hydrocarbon group is optionally interrupted by -COO-, -O-, -OCO-, -NHCO-, -NH-, or -CONH-, and
Z¹ to Z⁴ independently represent the same group as the group represented by Z¹ to Z³ in General Formula (4-1), 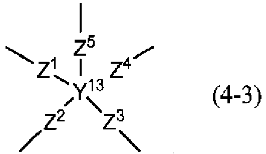
wherein Y¹³ represents an unsubstituted or substituted pentavalent aliphatic hydrocarbon group having 2 to 35 carbon atoms, an unsubstituted or substituted pentavalent aromatic ring-containing hydrocarbon group having 6 to 35 carbon atoms, or an unsubstituted or substituted pentavalent heterocycle-containing group having 3 to 35 carbon atoms,
the aliphatic hydrocarbon group is optionally interrupted by -COO-, -O-, -OCO-, -NHCO-, -NH-, or -CONH-, and
Z¹ to Z⁵ independently represent the same group as the group represented by Z¹ to Z³ in General Formula (4-1), 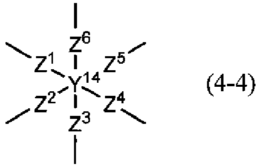
wherein Y¹⁴ represents an unsubstituted or substituted hexavalent aliphatic hydrocarbon group having 2 to 35 carbon atoms, an unsubstituted or substituted hexavalent aromatic ring-containing hydrocarbon group having 6 to 35 carbon atoms, or an unsubstituted or substituted hexavalent heterocycle-containing group having 3 to 35 carbon atoms,
the aliphatic hydrocarbon group is optionally interrupted by -COO-, -O-, -OCO-, -NHCO-, -NH-, or -CONH-, and
Z¹ to Z⁶ independently represent the same group as the group represented by Z¹ to Z³ in General Formula (4-1).

2. The antioxidant for a thermoplastic resin according to claim 1, the antioxidant being a compound in which R² in General Formulae (2) to (4) is an alkyl group having 1 to 8 carbon atoms whose terminal on an oxygen atom side is linked with -CO-O-.

3. A thermoplastic resin composition obtained by adding 0.01 to 0.5 parts by mass of the antioxidant for a thermoplastic resin according to claim 1 or 2 to 100 parts by mass of a thermoplastic resin.

4. The thermoplastic resin composition according to claim 3, wherein the thermoplastic resin is a polyolefin-based resin.

5. A vehicle member formed from the thermoplastic resin composition according to claim 3 or 4.
